# EUROPEAN PATENT APPLICATION

(11) **EP 2 005 975 A2**
(43) Date of publication of application: **24.12.2008**
(21) Application number: 07738054.1
(22) Date of filing: 08.03.2007
(51) Int. Cl.: A61L 27/00

(54) **COMPOSITE IMPLANT MATERIAL**

(30) Priority: 10.03.2006 JP 2006066291; 10.03.2006 JP 2006066292; 31.07.2006 JP 2006207816; 31.07.2006 JP 2006209012; 31.07.2006 JP 2006209013
(71) Applicant: TAKIRON CO., LTD., Osaka-shi, Osaka 541-0052 (JP)
(72) Inventor: The designation of the inventor has not yet been filed
(74) Representative: HOFFMANN EITLE
(86) International application number: PCT/JP2007/054564
(87) International publication number: WO 2007/105600

(57) **Abstract**

An implant composite material is provided which is for use in the treatment of articular cartilage disorders such as hip joint femur head necrosis and knee joint bone head necrosis, the reconstruction/fixing of a bio-derived or artificial ligament or tendon, the uniting/fixing of a bone, etc. Part of the implant composite material is replaced by bone tissues in an early stage to enable the material to stably bond with a living bone, while the other part retains a necessary strength over a necessary time period. Finally, the implant composite material is wholly replaced by the living bone and disappears.

It is an implant composite material having a constitution which comprises a compact composite of a biodegradable and bioabsorbable polymer containing bioabsorbable and bioactive bioceramic particles and a porous composite of a biodegradable and bioabsorbable polymer containing bioabsorbable and bioactive bioceramic particles, the porous composite being united with the compact composite. The porous composite is replaced by bone tissues in an early stage to enable the material to stably bond with a living bone, while the compact composite retains a necessary strength over a necessary time period. Finally, the material is wholly replaced by the living bone and disappears. Consequently, this implant composite material can sufficiently meet desires in this medical field.

## Description

### TECHNICAL FIELD

The present invention relates to an implant composite material which is for use in the treatment of articular cartilage disorders such as hip joint femur head necrosis and knee joint bone head necrosis, the reconstruction/fixing of a bio-derived or artificial ligament or tendon, the uniting/fixing of a bone, etc.

### BACKGROUND ART

Various regenerative medical techniques have hitherto been investigated in order to reconstruct, regenerate, or reinforce hard-bone or cartilage parts which have been destroyed or damaged considerably. It is widely understood that the reconstruction of a damaged part having a given shape essentially necessitates a scaffold which serves to help completion of the reconstruction by avoiding an external mechanical load or a cytological or physiological attack and forming/maintaining the desired shape until the regeneration of tissues is completed.

At present, various ideas have been proposed on scaffold materials for use in the case where a cartilage of a joint such as a hip joint or knee joint is in an abnormal state and this cartilage is required to be repaired, regenerated, or reconstructed. However, no material usable as a scaffold for the treatment or reconstruction of a necrotized part of a joint bone head or for the reinforcement of a ligament part adherent to a joint has been developed because of difficulties in material science. The reason for this is that this scaffold is to be applied to a boundary which is a discontinuous bone joint part which involves different functions and materials and in which a cartilage and a hard bone come into contact with each other while moving, i.e., the scaffold is to be applied to a part in a joint.

One measure for the development of such a scaffold may be a technique in which a prosthetic material comprising a cartilage substitute and a hard-bone substitute combined and united therewith is produced and the hard-bone substitute and the cartilage substitute are implanted in and fixed to an articular bone head part and an articular cartilage part, respectively. However, in the case where the two substitutes are not in a united form but a combination of separate members, continuous and connecting shifting is not obtained between cartilage tissues and hard-bone tissues. In addition, a problem that the two substitutes separate from each other upon joint movements arises. Consequently, a scaffold material usable in an articular part should be one in which the part to be disposed in a hard bone has a satisfactory affinity for the hard bone in terms of affinity concerning vital histology and mechanics and the part to be disposed in a cartilage has a satisfactory affinity for the cartilage in terms of affinity concerning vital histology and mechanics and which is thereby stably held in the joint, which is a movable interface, without detaching therefrom.

In this case, when the target prosthetic material is one not assimilable in the living body, such as a metal, ceramic, or polymer, it is not replaced by living tissues with the lapse of time and the long-term holding of the implanted material continuously has a fear concerning problems such as infection and mechanical troubles. It is therefore necessary that the prosthetic material should combine bioactivity and biodegradability which enable the material to be gradually replaced by living tissues to reconstruct a shape and be finally degraded and assimilated by the living body and disappear. It is mechanically and physiologically desirable that the prosthetic material should be one which simultaneously has both of a compact part and a porous part and in which the porous part, as a substitute for a cartilage, becomes higher in opening rate toward the cartilage surface and the compact part, as a substitute for a hard bone, becomes lower in opening rate toward inner parts of the compact part in which the prosthetic material is implanted.

Namely, in the development of a scaffold for the treatment or reconstruction of, e.g., articular cartilage disorders, there is a desire for a material comprising: a porous part in which cells rapidly penetrate and cartilage tissues inductively grow in a surface-layer part with scaffold degradation to enable the porous part to be replaced by living tissues; and a compact layer which conducts and tightly adheres to a hard bone and retains a sufficient strength over a certain time period until degradation and which finally is wholly degraded and completely replaced by hard-bone tissues.

Incidentally, the present inventor previously proposed an artificial bone for use as an implant material for the repair/reconstruction of a deficient part of a living bone comprising a cancellous bone and a cortical bone formed on the surface layer (outside) of the cancellous bone (patent document 1). This artificial bone comprises: a three-dimensional porous object comprising a biodegradable and bioabsorbable polymer having interconnected pores inside and containing bioactive bioceramic particles; and a compact surface layer superposed on and united with part of the surfaces of the porous object and comprising a biodegradable and bioabsorbable polymer containing bioactive bioceramic particles. This implant material is intended to be implanted in such a manner that the three-dimensional porous object is applied to the deficient part of the cancellous bone in an inner part of the living bone and the compact surface layer is applied to the deficient part of the cortical bone in a surface part. It is an artificial bone suitable for use as a substitute for an autograft bone flap or allograft bone flap.

On the other hand, background art concerning the reconstruction/fixing or reinforcement of a ligament or tendon are as follows. As is well known, there are four (two groups of) ligaments in a knee joint. One is tibial collateral ligament and fibular collateral ligament, and the other is anterior cruciate ligament and posterior cruciate ligament. In relation to knee twisting movements in sports activities, the most common case is damage to an anterior cruciate ligament (ACL). Techniques presently in use for treating the damage are: the BTB (bone tendon bone) method in which a normal bone-attached ACL or patella tendon (PT) of the patient is utilized; the semitendon method in which a hamstring tendon not attached to a bone is utilized; and the method in which an artificial ligament is utilized. Various measures have been taken to highly reliably fix not only autografts, allografts, and cadaveric bone-attached tendons and ligaments but also artificial ligaments in such a manner as to enable natural movements. Typical examples of the BTB (bone tendon bone) method, in which a damaged ACL is fixed between bones with those normal ligaments, and the method in which only a ligament or tendon having no bone is fixed between bones made up of soft tissues include the following three.
(1) Fixing with an interference screw.
(2) Fixing with a cross pin.
(3) Fixing with an end button of a hamstring tendon.

However, these fixing techniques generally have a drawback that the part where the ligament or tendon has been fixed becomes loose with the lapse of time. In the fixing (1), although metallic screws have conventionally been mainly employed, this fixing arouses troubles in extreme knee bends, e.g., sitting on the heels. Because of this, various assimilable screws have recently come to be used in a considerably high proportion. However, such screw fixing has a drawback that the screw does not directly bond with the bone in the implantation part. The screw receives a load caused by bends over a prolonged time period and this is a cause of getting loose. The same problem is pointed out in the case of (3) also. In the case of (2), there is a relatively small fear of that. However, this fixing technique unavoidably has a possibility that metallic cross pins, when present over long in a joint part involved in heavy movements, might shift their positions to cause stimulation and this might sometimes produce a serious harmful effect. Furthermore, assimilable ones have poor reliability with respect to flexural strength and deformation by flex relaxation.

The reconstruction of a damaged ACL with a ligament is explained below as an example. A well known method is to fix both ends of the ligament with metallic interference screws. In this case, the bone-attached ligament is implanted in the following manner. The bone parts on both ends of the ligament are inserted into holes respectively formed in the upper and lower living bones (thighbone side and shinbone side) of a knee joint. A metallic interference screw is screwed into the space between each bone part and the inner surface of the hole to fix the bone part on each end of the ligament. On the other hand, as the artificial ligament for use in this reconstruction, an artificial ligament is known which comprises many filaments stretched and arranged substantially in a row and in which both ends of the filaments have been looped for fixing with screws or the like (patent document 2).

As described above, metallic or ceramic interference screws are used in the reconstruction/fixing of a tendon or ligament. However, these screws have a high modulus of elasticity and, in particular, the metallic interference screws may adversely influence the living body due to metal ion dissolution. There is hence a problem that a reoperative surgery should be performed for taking the screws out of the body in an early stage after the treatment.

Under such circumstances, the present applicant previously proposed an interference screw for tendon or ligament fixing which is an interference screw comprising a biodegradable and bioabsorbable polymer and has a through-hole for Kirschner wire insertion formed along the center line therefor, an upper part of the through-hole (part on the screw head side) being a large elongated-circle hole part for rotating-tool fitting (patent document 3).

This interference screw for tendon or ligament fixing is intended to be used in the following manner. A Kirschner wire (guide wire for leading and screwing the screw in a desired direction with satisfactory accuracy) is inserted into the through-hole. The tip of a rotating tool is fitted into the elongated-circle hole part of the through-hole, and the tip is rotated to screw the screw in the proper direction into each of those holes formed in the bones of a joint (holes respectively formed in the upper and lower bones of a joint) into which the ends of a tendon or ligament to be transplanted/reconstructed have been inserted. Thus, the transplant bone flaps on both ends of the tendon or ligament are pressed against and fixed to the inner surfaces of the holes. The biodegradable and bioabsorbable polymer hydrolyzes due to contact with a body fluid and is assimilated by the living body. Consequently, this interference screw need not be taken out of the body through a reoperative surgery.

Next, background art concerning the uniting/fixing of bones is explained. Techniques for bone uniting/fixing include the following.

### 1. Uniting of fractured parts by osteosynthesis

a) Open-reduction fixation for fractures within and around joints such as an ankle joint, knee joint, hip joint, elbow joint, and shoulder joint
b) Open-reduction fixation for ossicular fractures in a hand or foot, such as one in a metacarpal bone or metatarsal bone

### 2. Fixing of transplant bone in bone transplantation

a) Fixing of a transplant bone flap in replacement with an artificial hip joint
b) Fixing of a transplant bone flap in replacement with an artificial knee joint
c) Fixing of a transplant bone flap in tumor curettage

### 3. Fixing of bone flap in osteotomy

a) Fixing of a bone flap in acetabular osteotomy
b) Fixing of a bone flap in osteotomy for hallux valgus correction
c) Fixing of a bone flap in wrist-joint reconstructive operation (Kapanji method)

### 4. Others

a) Temporary fixing of a joint (e.g., temporary fixing of a tibiofibular joint)
b) Proper uniting/fixing of fractured parts other than 1. a) above

For uniting/fixing those bones, bone-uniting materials such as metallic or ceramic screws orpins have been used hitherto. However, since these bone-uniting materials have a far higher modulus of elasticity than living bones, there are problems, for example, that dependence on their strength reduces rather than increases the strength of the bones surrounding the uniting materials. In particular, in the case of metallic screws, there is a fear that metal ions gradually released therefrom may adversely influence the living body in a prolonged time period exceeding 10 years after implantation. There is hence a fear that a reoperative surgery for taking the screws out of the body must be performed in an early stage.

Under these circumstances, investigations have come to be made on screws which comprise a biodegradable and bioabsorbable polymer and do not necessitate the reoperative surgery. The present applicant further developed various bone-uniting materials, e.g., a screw and a pin, which comprise a biodegradable and bioabsorbable polymer containing bioactive and bioabsorbable bioceramic particles and combine bioactivity and biodegradability and bioabsorbability, in order to satisfy a high degree of demands of doctors and patients (patent documents 4 and 5). Furthermore, a screw comprising that composite material was also developed which had a through-hole formed therein for inserting thereinto a Kirschner wire for leading and screwing the screw in a right direction into a given part with satisfactory accuracy (hollow screw called a cannulated screw).
Patent Document 1: JP-A-2004-121301
Patent Document 2: JP-T-7-505326 (The term "JP-T" as used herein means a published Japanese translation of a PCT patent application.)
Patent Document 3: JP-A-2000-166937
Patent Document 4: JP-A-11-70126
Patent Document 5: JP-A-10-85231

### DISCLOSURE OF THE INVENTION

### PROBLEMS THAT THE INVENTION IS TO SOLVE

However, the implant material proposed in patent document 1, which is an artificial bone suitable for use as a substitute for an autograft bone flap or allograft bone flap, is not suitable for use as a scaffold to be applied to a boundary which is a discontinuous bone joint part which involves different functions and materials and in which a cartilage and a hard bone come into contact with each other while moving, i.e., as a scaffold to be applied to a joint.

The method in which a bone-attached ligament is fixed with metallic interference screws has had the following drawback. The interference screws do not chemically bond directly with the upper and lower living bones of a joint but are physically fixed due to the rugged shape of the interference screws themselves. Because of this, it is difficult to consider that the strength of fixing the bone parts at both ends of the ligament is sufficiently secured over long. In particular, when an artificial ligament such as that disclosed in patent document 2 is used and the end loop parts are fixed with screws, then there is a high possibility that this artificial ligament might detach from the living bones because the loop parts do not directly bond with the upper and lower living bones of the joint. In addition, there has been a high possibility that when a tensile force is repeatedly applied, the artificial ligament might be elongated due to stress relaxation or cut by the screw thread.

The technique of fixing a tendon or ligament with the interference screw proposed in patent document 3 has a problem that the adhesion of the transplant bone flap on an end of a tendon or ligament to the inner surface of a hole formed in a bone (bone adhesion) necessitates much time as in the case of other materials such as metals and bioceramics. There also has been a problem that after bone adhesion is obtained, much time is required for the screw to be completely replaced by a living bone and disappear. On the other hand, it is well known that a biological bone growth factor such as a BMP (bone morphogenic protein) is effective in accelerating replacement by a living bone and regeneration. However, such biological bone growth factors cannot be directly incorporated into the screw comprising a biodegradable and bioabsorbable polymer. This is because the screw comprising a biodegradable and bioabsorbable polymer has a heat history including heating to at least 100°C or higher in the steps of strengthening, molding, and producing the screw and, hence, the biological bone growth factors are thermally altered and are deprived of their activity.

In addition, the through-hole of that interference screw is less apt to undergo bone tissue invasion/growth (bone ingrowth). Because of this, there has been a problem that part of the through-hole remains vacant until the screw is mostly degraded/assimilated and replaced by a bone. Although a technique in which autobone particles taken out of another part are packed into the hole may be employed, the donor part remains as a defective part and, hence, should be filled with artificial bone particles. Complete repair with an autobone is not attained.

The screw disclosed in patent document 4 and the pin disclosed in patent document 5 are ones in which the polymer gradually hydrolyzes in the living body and bone tissues conductively grow due to the bioactivity of the bioceramic particles exposed as a result of the hydrolysis. The screw and pin are replaced by a living bone and disappear after all. However, like the interference screw proposed in patent document 3, the screw and pin have problems that bone adhesion necessitates much time as in the case of other materials such as metals and bioceramics and that after bone adhesion is obtained, much time is required for the screw to be completely replaced by a living bone and disappear. In addition, there also are problems, as in the case of the interference screw proposed in patent document 3, that a biological bone growth factor such as a BMP (bone morphogenic protein) cannot be directly incorporated into the screw or pin comprising a biodegradable and bioabsorbable polymer and that until the cannulated screw is mostly degraded/assimilated and replaced by a bone, part of the through-hole remains vacant.

The present invention has been achieved under these circumstances. A subject for the invention is to provide an implant composite material which is for use as a temporary prosthetic/scaffold material in the case where a cartilage of a joint such as a hip joint or knee joint is in an abnormal state and this cartilage is required to be repaired, regenerated, or reconstructed, i.e., a necrotized part of an articular bone head is required to be treated or reconstructed, or the case where a ligament part adherent to a joint is to be reinforced, and which has the aforementioned properties or functions desired in this medical field and can be stably implanted in and fixed to a joint.

Another subject for the invention is to provide an implant composite material for use as an end anchor (anchor member) of a ligamental member or tendinous member. It is an implant composite material for anchoring to be attached to an end part of a ligamental member or tendinous member (the implant composite material corresponds to a bone of a bone-attached ligament or tendon). It bonds with the upper or lower living bones (thighbone or shinbone) of a knee joint in an early stage and, hence, enables the end part of a ligamental member or tendinous member to be fixed in a shorter time period and to come to have a greatly heightened fixing strength as compared with the case of fixing with metallic or assimilable interference screws heretofore in use.

Still another subject for the invention is to provide an implant composite material for tendon or ligament fixing and an implant composite material for osteosynthesis which are capable of eliminating problems described above, i.e., the problem that bone adhesion necessitates much time as in the case of metals and bioceramics, the problem that much time is required for a screw to be completely replaced by a living bone and disappear after bone adhesion is obtained, the problem that a biological bone growth factor such as a BMP cannot be directly incorporated, and the problem that part of a through-hole remains vacant until the screw is mostly degraded/assimilated and replaced by a bone.

### MEANS FOR SOLVING THE PROBLEMS

In order to accomplish those subjects, the invention provides a bioabsorbable and bioactive implant composite material characterized by comprising a compact composite of a biodegradable and bioabsorbable polymer containing bioabsorbable and bioactive bioceramic particles and a porous composite of a biodegradable and bioabsorbable polymer containing bioabsorbable and bioactive bioceramic particles, the porous composite being united with the compact composite. This implant composite material of the invention includes four types. A first type is an implant composite material which is for use in the treatment or reconstruction of articular cartilage disorders or the reconstruction or reinforcement of a ligament part adherent to a joint and which is applied to part of a joint where an articular bone head is in contact with an articular cartilage, such as knee, hip, ankle, shoulder, elbow, and vertebral (cervical vertebra and lumbar vertebra) joints as a temporary prosthetic material or scaffold and as a support for the gradual release of a biological bone growth factor. A second type is an implant composite material to be attached as an anchor member to an end part of a ligamental member or tendinous member. A third type is an implant composite material for tendon or ligament fixing, such as an interference screw. A fourth type is an implant composite material for osteosynthesis.

The implant composite material of the first type of the invention is characterized in that the porous composite, which comprises a biodegradable and bioabsorbable polymer containing bioabsorbable and bioactive bioceramic particles, has been superposed on and united with one side or all surfaces of the compact composite, which comprises a biodegradable and bioabsorbable polymer containing bioabsorbable and bioactive bioceramic particles.

In this implant composite material of the first type, it is preferred that the porosity of the porous composite having interconnected pores should gradually change to have an inclination so that the porosity increases from an inner-layer part to a surface-layer part of the porous composite having interconnectedpores in the range of 50-90%. It is alsopreferred that the content of the bioceramic particles in the porous composite should gradually change to have an inclination so that it increases from an inner-layer part to a surface-layer part of the porous composite in the range of 30-80% by mass. Furthermore, it is preferred that the porous composite should have been impregnated with at least one biological bone growth factor selected from a BMP (Bone Morphogenic Protein), TGF-β (Transforming Growth Factor β), EP4 (Prostanoid Receptor), b-FGF (basic Fibroblast Growth Factor), and PRP (platelet-rich plasma) and/or an osteoblast derived from a living organism.

The implant composite material of the second type of the invention is an implant composite material for use as an end anchor of a ligamental member or tendinous member. It is an implant composite material to be attached as an anchor member to an end part of a ligamental member or tendinous member so as not to detach therefrom, and is characterized in that the porous composite, which comprises a biodegradable and bioabsorbable polymer containing bioabsorbable and bioactive bioceramic particles, has been superposed on and united with part or all of the surfaces of the compact composite, which comprises a biodegradable and bioabsorbable polymer containing bioabsorbable and bioactive bioceramic particles.

In this implant composite material of the second type (anchor member), it is preferred from the standpoint of strength that the porous composite should have a porosity of 50-90%, at least 50% of all pores be accounted for by interconnected pores, and the porosity of the porous composite gradually change to have an inclination so that the porosity increases from an inner-layer part to a surface-layer part of the porous composite. It is also preferred, from the standpoint of bone conductivity which enables direct bonding with a surrounding bone, that the content of the bioceramic particles in the porous composite layer should gradually change to have an inclination so that it increases from an inner-layer part to a surface-layer part of the porous composite in the range of 30-80% by mass. Furthermore, it is preferred that the porous composite layer should have been impregnated with at least one biological bone growth factor selected from a BMP, TGF-β, EP4, b-FGF, and PRP and/or an osteoblast derived from a living organism. It is further preferred that many small holes or small projections for the attachment of a ligamental member or tendinous member should be formed in or on an end part of this implant composite material (anchor member).

The implant composite material of the third type of the invention is an implant composite material for tendon or ligament fixing which is characterized by comprising: an interference screw which comprises the compact composite and has a through-hole for inserting a Kirschner wire thereinto; and a packing which comprises the porous composite and with which the through-hole is filled, the packing containing a biological bone growth factor.

In this implant composite material of the third type, it is preferred that the content of the bioceramic particles in the compact composite constituting the interference screw should be 30-60% by mass and the content of the bioceramic particles in the porous composite constituting the packing be 60-80% by mass. It is also preferred that the porous composite constituting the packing should be one which has a porosity of 60-90% and in which at least 50% of all pores are accounted for by interconnected pores and the interconnected pores have a pore diameter of 50-600 µm. Furthermore, the packing is preferably impregnated with at least one biological bone growth factor selected from a BMP, TGF-β, EP4, b-FGF, and PRP.

The implant composite material of the fourth type of the invention is an implant compositematerial for osteosynthesis which is characterized by comprising a bone-uniting material main body comprising the compact composite and having a hole bored to have at least one open; and a filler packed in the hole, the filler comprising the porous composite. Examples of this implant composite material (bone-uniting material) include: one in which the bone-uniting material main body is a screw having a bored hole to be filled with the filler, the hole extending along the center line for the screw from the upper end surface of the screw head toward the screw tip (bone-uniting screw) ; and one in which the bone-uniting material main body is a pin having a bored hole to be filled with the filler, the hole extending along the center line for the pin from one end toward the other end of the pin (bone-uniting pin).

In this implant composite material of the fourth type (bone-uniting material), it is preferred that the filler comprising the porous composite should be impregnated with at least one biological bone growth factor selected from a BMP, TGF-β, EP4, b-FGF, and PRP. It is also preferred that the content of the bioceramic particles in the compact composite constituting the bone-uniting material main body should be 30-60% by mass and the content of the bioceramic particles in the porous composite constituting the filler be 60-80%bymass. It is further pre f erred that the porous composite constituting the filler should be one which has a porosity of 60-90% and in which at least 50% of all pores are accounted for by interconnected pores and the interconnected pores have a pore diameter of 50-600 µm.

### ADVANTAGES OF THE INVENTION

In the implant composite material of the invention, the porous composite is rapidly hydrolyzed from the surface and inner parts thereof by the action of a body fluid in contact with the surface and of a body fluid which has penetrated into interconnected pores thereof. With this hydrolysis, the inductive growth of bone tissues is triggered by the bioactive bioceramic particles and bone tissues grow up to inner parts of the porous composite. The implant composite material is thus replaced by (cartilage) bone tissues in a relatively short time period. On the other hand, the compact composite is hard and strong and hydrolyzes far more slowly than the porous composite. It retains a sufficient strength until the hydrolysis proceeds to a certain degree and is wholly degraded finally. A living bone conductively grows by the action of the bioactive bioceramic particles and the compact composite is thus replaced by bone tissues. Since the bioceramic particles contained in the porous composite and in the compact composite are bioabsorbable, they neither remain/accumulate in the (cartilage) bone tissues which have replaced and regenerated nor come into soft tissues or blood vessels.

The implant composite material in which the porous composite has been superposed on and united until one side or all surfaces of the compact composite, like that of the first type of the invention, has the properties or functions required of scaffold materials and the like for use in, e.g., the treatment of articular cartilage disorders as stated above. Because of this, when the implant composite material of the first type in which the porous composite has been superposed on and united with one side of the compact composite is implanted in and fixed to, for example, a part where a necrotized part of an articular bone head has been excised, so that the porous composite is located on the cartilage side of the articular bone head surface, then it functions by the following mechanism. The porous composite is wholly replaced by cartilage tissues inductively grown in an early stage and disappearance, and the compact composite, which has strength, also is wholly replaced finally by conductively grown hard-bone tissues and disappears. The bioceramic particles also are completely assimilated. Thus, the hard-bone part and cartilage part of the necrotized articular bone head part are regenerated. On the other hand, when the implant composite material of the first type in which the porous composite has been superposed on and united with the all surfaces of the compact composite is implanted in an excised part of an articular bone head, the following effect/advantage is brought about besides those described above. Hard-bone tissues rapidly grow inductively in the porous composite in contact with the hard bone in the excised part, whereby this implant composite material is bonded with and fixed to the excised part of the articular bone head in a short time period.

The implant composite material of the first type in which the porosity of the porous composite gradually changes to have an inclination so that the porosity increases from an inner-layer part to a surface-layer part of the porous composite in the range of 50-90% has the following advantage. A body fluid and an osteoblast more easily penetrate into the surface side of the high-porosity porous composite having interconnected pores, and hydrolysis and the inductive growth of (cartilage) bone tissues proceed rapidly. Consequently, this implant composite material bonds with a living (cartilage) bone in an earlier stage to complete regeneration. The content of the bioceramic particles in the porous composite may be even throughout the porous composite. However, the porous composite in which the content thereof gradually changes to have an inclination so that it increases from an inner-layer part to a surface-layer part of the porous composite in the range of 30-80% by mass has the following advantage. Since the surface side of the porous composite has a high bioceramic-particle proportion and hence has higher bioactivity, the inductive growth of an osteoblast and bone tissues on the surface side is especially enhanced. As a result, replacement by (cartilage) bone tissues is further accelerated. The porous composite containing at least one biological bone growth factor selected from a BMP, TGF-β, EP4, b-FGF, and PRP and/or an osteoblast derived from a living organism has the following advantage. Osteoblast multiplication/growth is greatly accelerated and, hence, (cartilage) bone tissues grow vigorously. Thus, regeneration proceeds more rapidly.

The implant composite material of the second type of the invention (anchor member) may be used for the reconstruction/fixing of a ligament, for example, in the following manner. This anchor member is attached to each of both ends of a ligamental member so as not to detach therefrom. The anchor members attached to the end parts of the ligamental member are inserted into holes respectively formed in the upper and lower living bones of a knee joint (thighbone and shinbone) . An interference screw is then screwed into the space between each anchor member and the inner surface of the hole. As a result, the porous composite layer superposed on and united with part or all of the surfaces of the compact composite of each anchor member is rapidly hydrolyzed from the surface and inner parts thereof by a body fluid in contact with the surface thereof and by a body fluid which has penetrated into interconnected pores. With this hydrolysis, bone tissues are inductively grown to inner parts of the porous composite layer by the bone inductivity of the bioactive bioceramic particles. The porous composite layer is thus replaced by a living bone in an early stage and the anchor members bond with the inner surfaces of the holes formed in the upper and lower living bones of the knee joint.

As described above, when the implant composite material of the second type for anchoring (anchor member) is attached to an end part of a ligamental member, this anchor member bonds with a living bone (inner surface of a hole) in an early stage. Because of this, both ends of the ligamental member come to have a greatly improved fixing strength as compared with the conventional physical fixing with interference screws only. Furthermore, in this anchor member, the compact composite is hard and strong, hydrolyzes far more slowly than the porous composite layer, and retains a sufficient strength until the hydrolysis proceeds to a certain degree. Finally, however, the compact composite is wholly hydrolyzed and disappears while being replaced by a living bone conductively formed by the action of the bioactive bioceramic particles. As a result, the holes formed in the upper and lower living bones of the knee joint are filled with the living bones. In addition, since the bioceramic particles contained in the porous composite layer and in the compact composite are bioabsorbable, they neither remain/accumulate in the living bones which have replaced and regenerated nor come into soft tissues or blood vessels.

The implant composite material of the second type for anchoring (anchor member) in which the porous composite has a porosity of 50-90%, at least 50% of all pores are accounted for by interconnected pores, and the porosity of the porous composite layer gradually changes to have an inclination so that the porosity increases from an inner-layer part to a surface-layer part of the porous composite layer has the following advantage. A body fluid and an osteoblast more easily penetrate into surface parts of the porous composite layer having a high porosity, and hydrolysis and the inductive growth of bone tissues proceed rapidly, whereby the anchor member bonds with a living bone (inner surface of a hole) in an earlier stage. The porous composite layer in which the content of the bioceramic particles gradually changes to have an inclination so that it increases from an inner-layer part to a surface-layer part of the porous composite layer in the range of 30-80% by mass has the following advantage. Since the surface-layer part has a high bioceramic-particle proportion and hence has higher bioactivity, the inductive growth of an osteoblast and bone tissues in the surface-layer part is especially enhanced, and replacement by and bonding with a living bone (inner surface of a hole) are further accelerated. Furthermore, the porous composite layer containing at least one biological bone growth factor selected from a BMP, TGF-β, EP4, b-FGF, and PRP and/or an osteoblast derived from a living organism has the following advantage. Osteoblast multiplication/growth is greatly accelerated and, hence, bone tissues grow vigorously to enable bonding with and replacement by a living bone to proceed more rapidly. Moreover, the anchor member in which many small holes or small projections for the attachment of a ligamental member or tendinous member have been formed in or on an end part thereof has the following advantage. A bio-derived or artificial ligamental member or tendinous member can be attached thereto so as not to detach therefrom without fail by passing the organic fibers of the ligamental or tendinous member through the small holes and then hitching them on the anchor member or by hitching the organic fibers on the small projections.

Next, the implant composite material of the third type of the invention, which is for tendon or ligament fixing (interference screw), maybe used, for example, in the following manner. The transplant bone flaps on end parts of a transplant tendon are inserted into holes respectively formed in the upper and lower bones of a joint, and this interference screw is screwed into the space between each transplant bone flap and the inner surface of the hole to thereby press the transplant bone flap against the inner surface of the hole and fix it. In this application, the interference screw itself, which comprises the compact composite comprising a biodegradable and bioabsorbable polymer containing bioceramic particles; has a sufficient mechanical strength, although it is a hollow object having a through-hole formed therein, and slowly undergoes hydrolysis by a body fluid. Because of this, the interference screw retains its strength over a period of at least 3 months, which is necessary for ordinary bone adhesion, and the transplant bone flap on each end of the transplant tendon can be pressed against and fixed to the inner surface of the hole without fail. On the other hand, the packing which comprises the porous composite of a biodegradable and bioabsorbable polymer containing bioceramic particles and which has been inserted in the through-hole of the interference screw is a cancellous-bone-like porous object. This packing enables a body fluid and an osteoblast to penetrate into inner parts of the porous composite through interconnected pores, and is degraded and assimilated earlier than the interference screw comprising the compact composite while exhibiting its bone conductivity and bone inductivity based on the bioactivity of the bioceramic particles. Prior to or simultaneously with this degradation/assimilation, the biological bone growth factor supported, such as a BMP, is gradually released. Because of this, the conductive formation of a living bone (autobone) is efficiently accelerated and bone adhesion is completed in about several weeks, which period is considerably shorter than three months necessary for ordinary bone adhesion. Thus, the transplant bone flaps on end parts of the transplant tendon are fixed to the inner surfaces of the holes (i.e., to the living bones) in such an early stage. Thereafter, each interference screw and the packing further undergo degradation and assimilation and are finally replaced completely by a living bone formed by bone conduction or bone induction, whereby the joint is restored to the original state in which the through-hole of the screw does not remain vacant. Furthermore, since the biological bone growth factor contained in the packing comprising the porous composite has not undergone the heat history attributable to screw production, it has no fear of having undergone thermal alteration. In addition, since the bioceramic particles contained in the packing and in the screw are bioabsorbable, they neither remain/accumulate in the living bones which have replaced nor come into/remain in soft tissues or blood vessels. Moreover, since the surface-layer part of each interference screw bonds in an early stage with the transplant bone flap on an end part of the transplant tendon and with the inner surface of the hole in an early stage due to bone tissues conductively grown with hydrolysis, the screw can be prevented from becoming loose.

This implant composite material of the third type in which the content of the bioceramic particles in the compact composite constituting the interference screw is 30-60% by mass, the content of the bioceramic particles in the porous composite constituting the packing is 60-80% by mass, the porous composite has a porosity of 60-90%, at least 50% of all pores are accounted for by interconnected pores, and the interconnected pores have a pore diameter of 50-600 µm has the following advantages. This implant composite material exhibits satisfactory bone conductivity and bone inductivity while retaining the intact strength required of the interference screw and packing, and can be replaced by and regenerate a livingbone. Furthermore, this packing canbe easily and rapidly impregnated with a biological bone growth factor.

The implant composite material of the fourth type of the invention for osteosynthesis (bone-uniting material) is one to be used in a state in which a biological bone growth factor has been injected/infiltrated into the filler comprising the porous composite. Based on the functions of the following basic constituent materials, this bone-uniting material provides excellent measures against problems of the related-art bone-uniting materials described above. This applies in the case of the implant composite material for tendon or ligament fixing as the third embodiment.

### 1. (Constituent Materials)

This implant composite material is a composite comprising three components. Namely, it comprises a hollow object which, although hollow, has such a high mechanical strength and a long strength retention period that this hollow object is usable as a biodegradable bone-uniting material and
in which the through-hole is filled with a porous material functioning as a bone substitute which itself has bone conductivity and bone inductivity and has a porous nature and mechanical strength similar to those of cancellous bones or as a scaffold which accelerates bone penetration and regeneration, the porous material containing a biological bone growth factor.

### 2. (Functions)

A) The composite, although comprising the three components, as a whole has a sufficient mechanical strength required of bone-uniting materials (torque strength required for insertion and strength required for the uniting of a bone separated for some reason, e.g., fracture) and has the ability to retain the strength over a period of at least three months, which is necessary for ordinary bone adhesion.
B) The cancellous-bone-like porous object packed in the through-hole by itself exhibits bone conductivity and bone inductivity and is degraded and assimilated earlier than the hollow compact object, which has a strength not lower than that of the high-strength cortical bone on the outermost side. Prior to or simultaneously with this behavior, the biological bone growth factor supported is gradually released. Consequently, the porous object functions as a scaffold which efficiently accelerates the inductive formation of an autobone.
C) That period is considerably shorter than three months, which is necessary for ordinary bone adhesion. There is a possibility that bone adhesion might be completed in a period as short as several weeks. Consequently, the time period required for the patient to leave his bed is significantly shortened, and all of the patient, doctor, and hospital make a large profit.
D) Thereafter, the hollow biodegradable bone-uniting material and the porous scaffold, which constitute the bone-uniting material, are gradually degraded and assimilated in the living body and are completely replaced finally by a living bone. The bone is thus restored to the normal original state.
E) The biological bone growth factor, which is susceptible to thermal or chemical alteration, can be added as an injection or dripping preparation in a solution or suspension state to the porous object having interconnected pores and, hence, does not alter.

In the case where the bone-uniting material main body comprising the compact composite in this implant composite material of the fourth type for osteosynthesis is, for example, a screw, this main body is screwed into the bone of a fractured part to unite and fix the fractured part. In the case where the bone-uniting material main body is, for example, a pin, this main body is driven into the bone of a fractured part to unite and fix it. After the fractured part is thus united and fixed, the bone-uniting material main body, which comprises the compact composite of a biodegradable and bioabsorbable polymer containing bioceramic particles, retains its strength over a period of at least 3 months, which is necessary for ordinary bone adhesion, and can fix the osteosynthesis part without fail. This is because the main body has a sufficient mechanical strength, although it is a hollow object having a through-hole to be filled with the filler, and because it slowly undergoes hydrolysis by a body fluid. On the other hand, the filler which comprises the porous composite of a biodegradable and bioabsorbable polymer containing bioceramic particles and which has been packed in the hole of thebone-uniting material main body is a cancellous-bone-like porous object. This filler enables a body fluid and an osteoblast to penetrate into inner parts of the porous composite through interconnected pores, and is degraded and assimilated earlier than the bone-uniting material main body comprising the compact composite while exhibiting its bone conductivity and bone inductivity based on the bioactivity of the bioceramic particles. Prior to or simultaneously with this degradation/assimilation, the biological bone growth factor supported, such as a BMP, is gradually released. Because of this, the conductive formation of a living bone (autobone) is efficiently accelerated and bone adhesion is completed in about several weeks, which is considerably shorter than three months necessary for ordinary bone adhesion. Thereafter, the bone-uniting material main body and the filler further undergo degradation and assimilation and are finally replaced completely by a living bone formed by bone conduction or bone induction, whereby the bone is restored to the original state in which the hole of the bone-uniting material main body does not remain vacant. Furthermore, since the biological bone growth factor contained in the filler comprising the porous composite has not undergone the heat history attributable to the production of the bone-uniting material main body, it has no fear of having undergone thermal alteration and performs the function of accelerating bone growth. In addition, since the bioceramic particles contained in the filler and in the bone-uniting material main body are bioabsorbable, they neither remain/accumulate in the living bone which has replaced nor come into/remain in soft tissues or blood vessels.

This implant composite material of the fourth type for osteosynthesis in which the content of the bioceramic particles in the compact composite constituting the bone-uniting material main body is 30-60% by mass and the content of the bioceramic particles in the porous composite constituting the filler is 60-80% by mass has the following advantages. This bone-uniting material main body exhibits satisfactory bone conductivity while retaining the intact necessary strength and can be replaced by a living bone, while the filler also exhibits satisfactory bone inductivity and can be replaced by a living bone in an early stage. Furthermore, the implant composite material for osteosynthesis in which the porous composite constituting the filler has a porosity of 60-90%, at least 50% of all pores are accounted for by interconnected pores, and the interconnected pores have a pore diameter of 50-600 µm has the following advantage. An appropriate amount of a biological bone growth factor can be easily injected and infiltrated into the filler to facilitate the penetration of a body fluid or an osteoblast. Because of this, the hydrolysis of the filler and the inductive growth of bone tissues proceed in an early stage and the filler is wholly replaced by a living bone and disappears in a short period.

### BRIEF DESCRIPTION OF THE DRAWINGS

[Fig. 1]

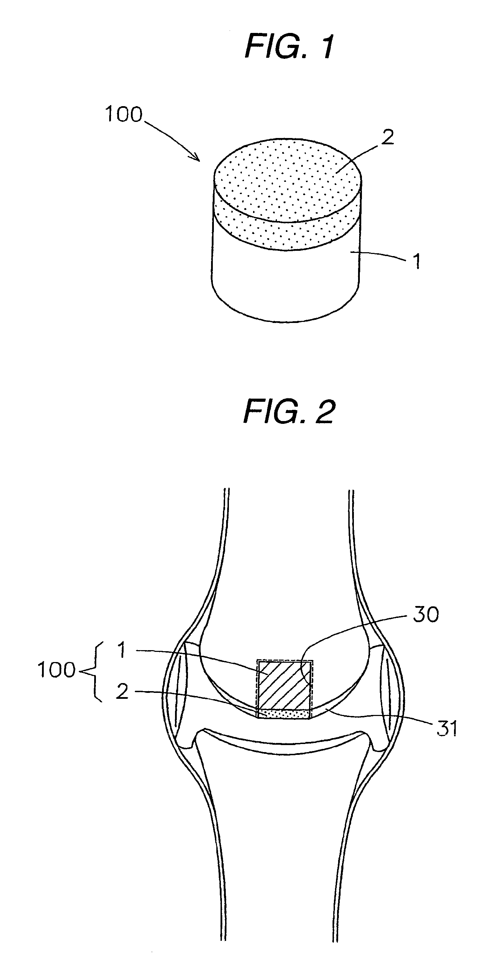

   Fig. 1 is a slant view of an implant composite material of the first type as one embodiment of the invention.
[Fig. 2]
   Fig. 2 is a view illustrating an example in which the implant composite material is used.
[Fig. 3]

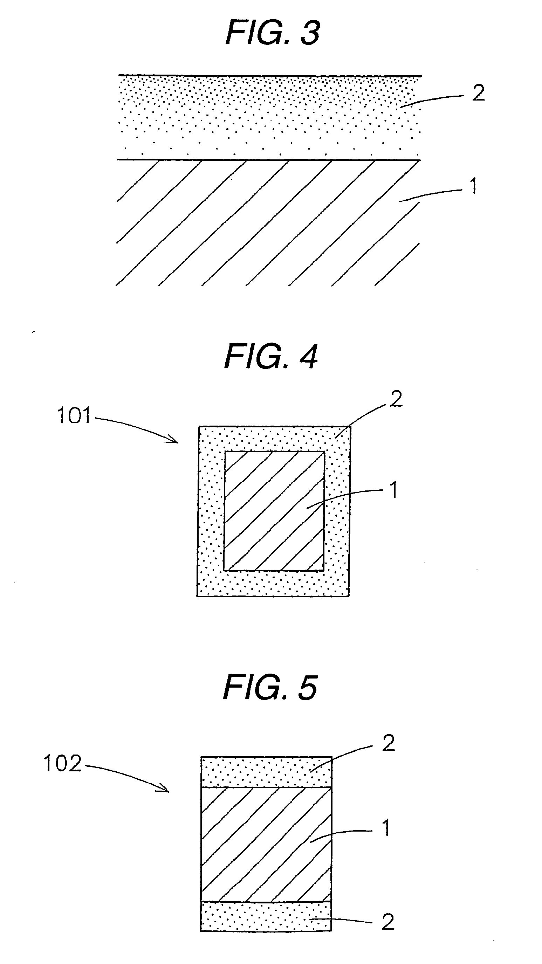

   Fig. 3 is an enlarged sectional view illustrating part of the implant composite material.
[Fig. 4]
   Fig. 4 is a sectional view of an implant composite material of the first type as another embodiment of the invention.
[Fig. 5]
   Fig. 5 is a sectional view of an implant composite material of the first type as still another embodiment of the invention.
[Fig. 6]

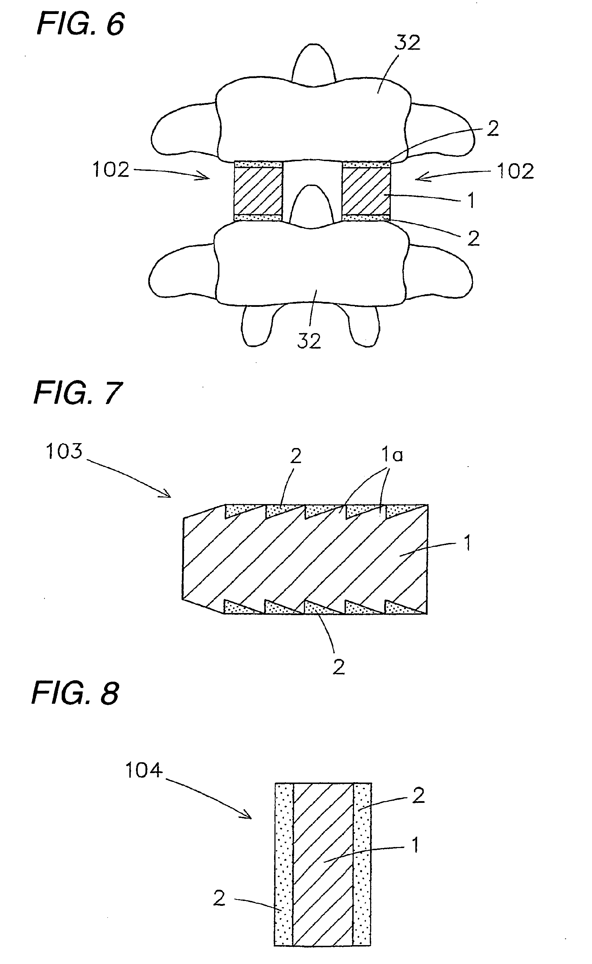

   Fig. 6 is a view illustrating an example in which the implant composite material is used.
[Fig. 7]
   Fig. 7 is a sectional view of an implant composite material of the first type as a further embodiment of the invention.
[Fig. 8]
   Fig. 8 is a sectional view of an implant composite material of the first type as still a further embodiment of the invention.
[Fig. 9]

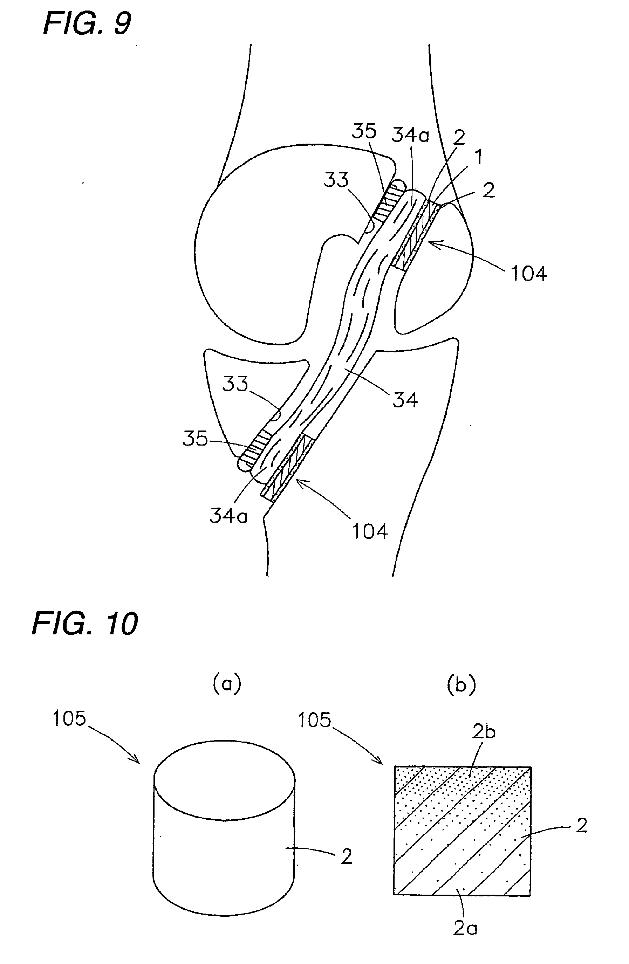

   Fig. 9 is a view illustrating an example in which the implant composite material is used.
[Fig. 10]
   Fig. 10 (a) is a slant view illustrating one example of modifications of implant composite materials of the first type, and Fig. 10 (b) is a diagrammatic sectional view of this implant composite material.
[Fig. 11]

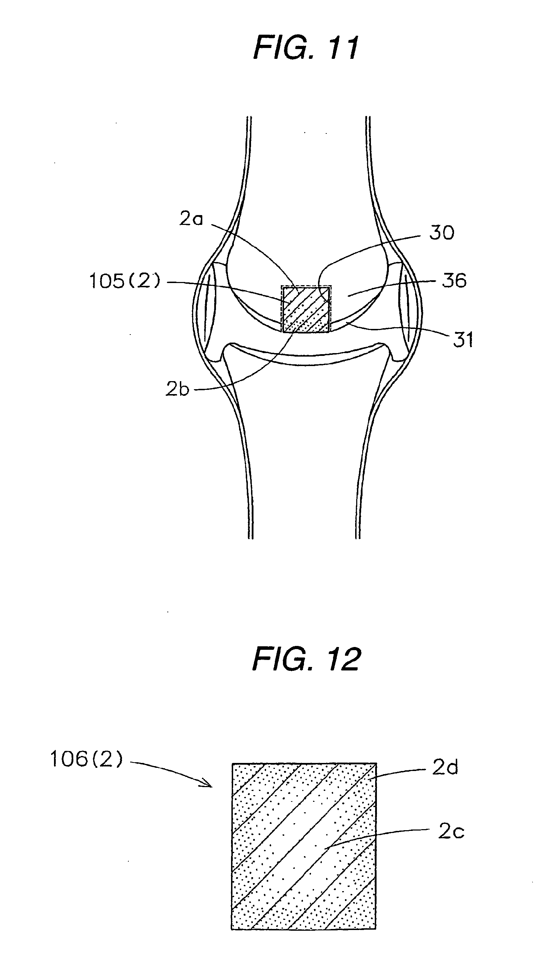

   Fig. 11 is a view illustrating an example in which the implant composite material is used.
[Fig. 12]
   Fig. 12 is a diagrammatic sectional view illustrating another example of the modifications of implant composite materials of the first type.
[Fig. 13]

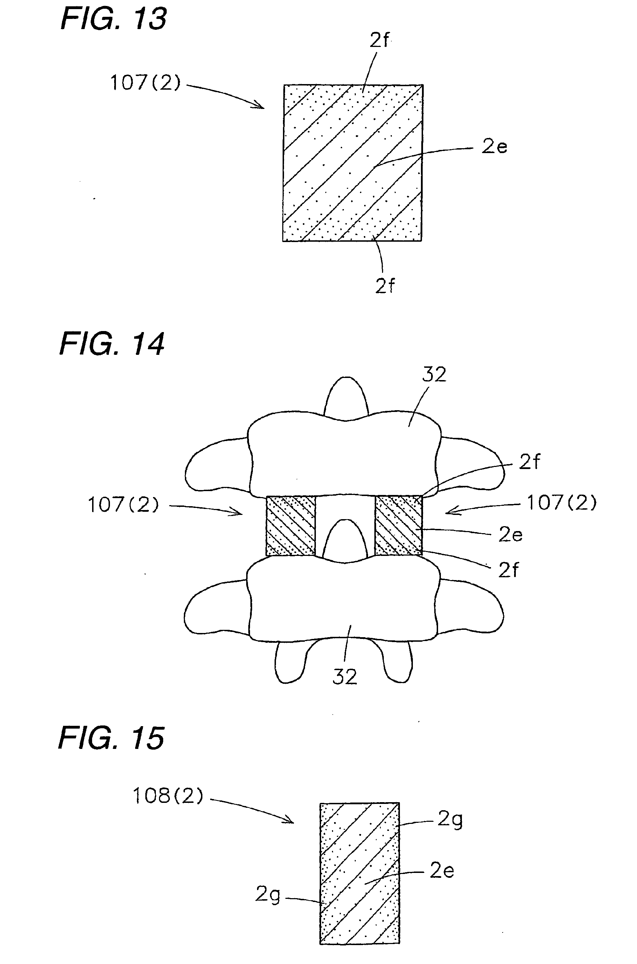

   Fig. 13 is a diagrammatic sectional view illustrating still another example of the modifications of implant composite materials of the first type.
[Fig. 14]
   Fig. 14 is a view illustrating an example in which the implant composite material is used.
[Fig. 15]
   Fig. 15 is a diagrammatic sectional view illustrating a further example of the modifications of implant composite materials of the first type.
[Fig. 16]

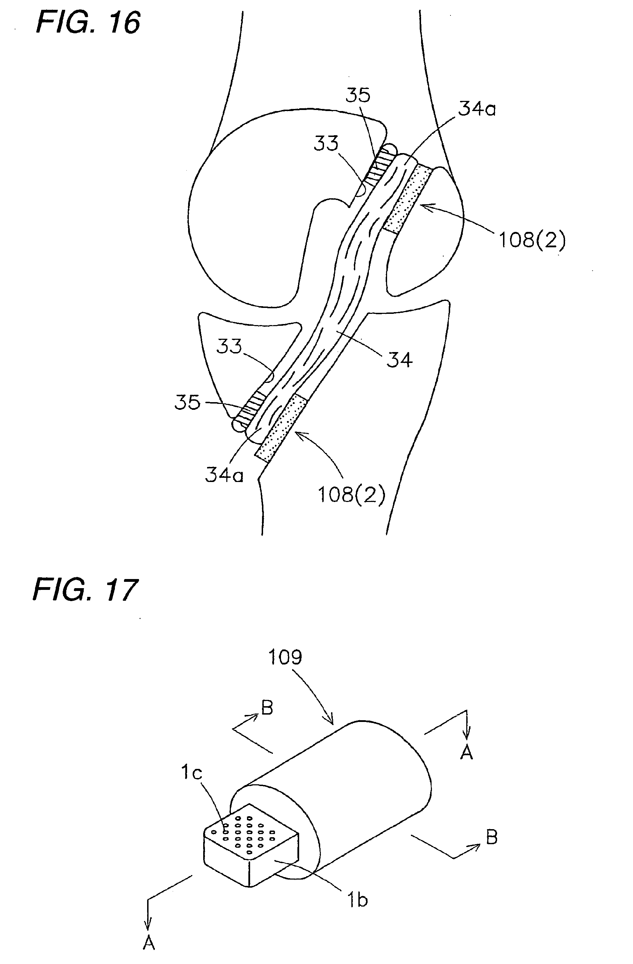

   Fig. 16 is a view illustrating an example in which the implant composite material is used.
[Fig. 17]
   Fig. 17 is a slant view of an implant composite material of the second type as still a further embodiment of the invention.
[Fig. 18]

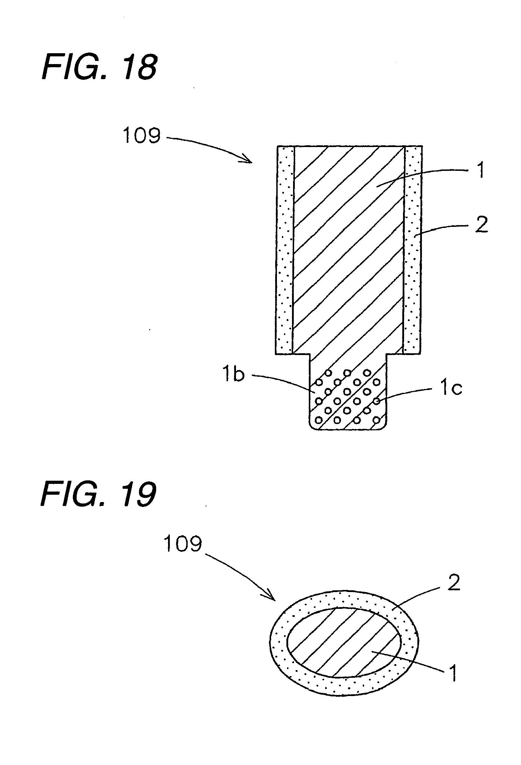

   Fig. 18 is a sectional view taken on the line A-A of Fig. 17.
[Fig. 19]
   Fig. 19 is a sectional view taken on the line B-B of Fig. 17.
[Fig. 20]

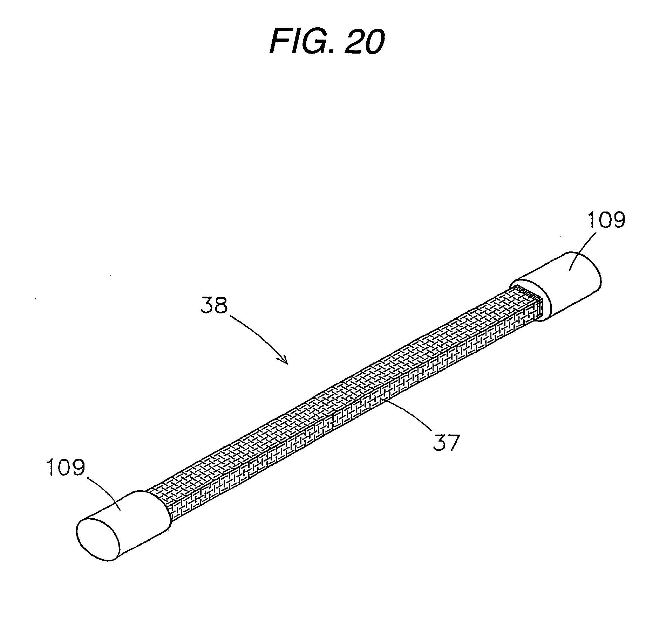

   Fig. 20 is a slant view of an artificial ligament having the implant composite material attached to each end thereof.
[Fig. 21]

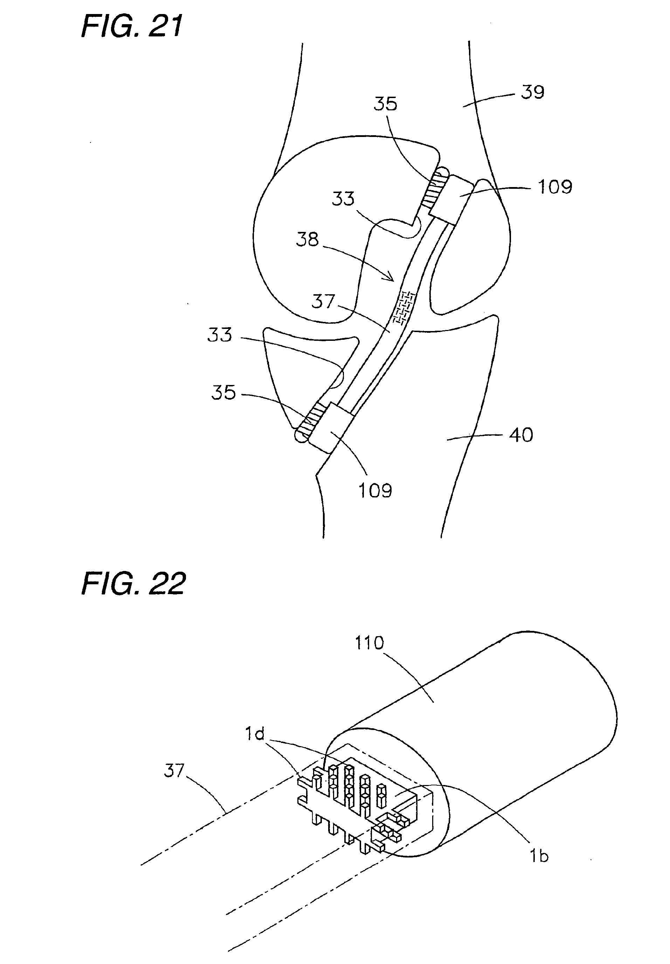

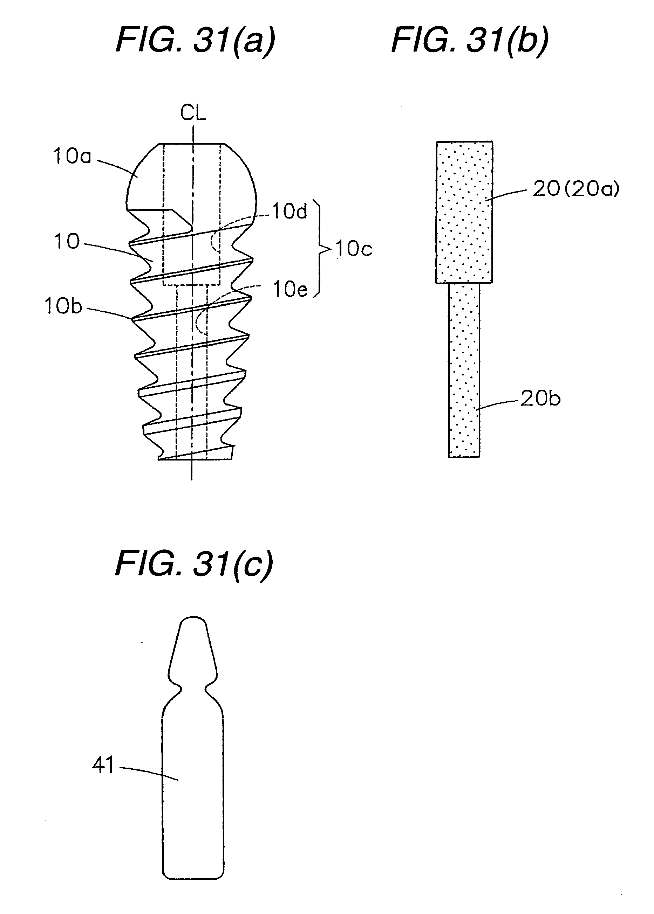

   Fig. 21 is a view illustrating an example in which the artificial ligament is used.
[Fig. 22]
   Fig. 22 is a slant view of an implant composite material of the second type as still a further embodiment of the invention.
[Fig. 23]

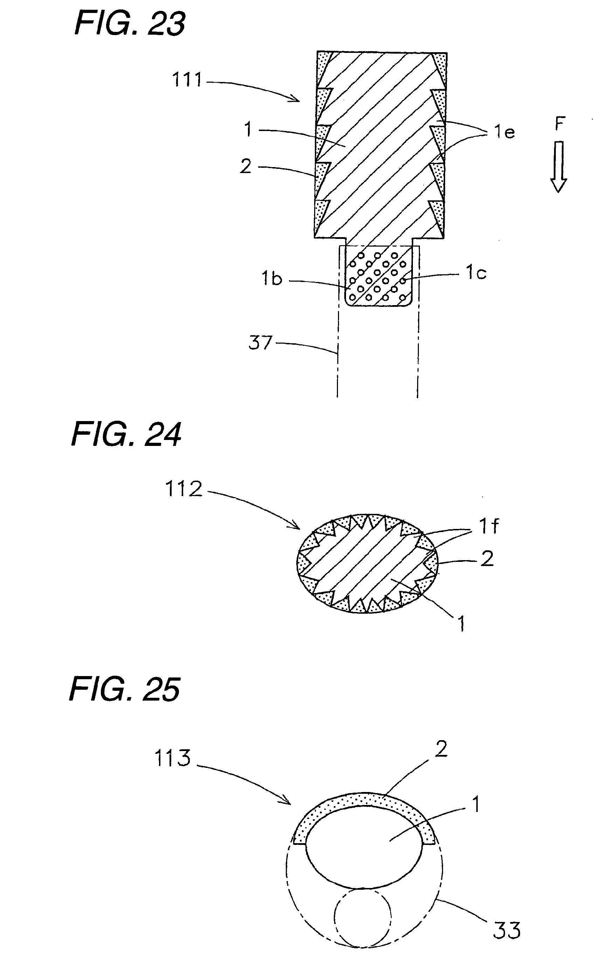

   Fig. 23 is a vertical sectional view of an implant composite material of the second type as still a further embodiment of the invention.
[Fig. 24]
   Fig. 24 is a cross-sectional view of an implant composite material of the second type as still a further embodiment of the invention.
[Fig. 25]
   Fig. 25 is a cross-sectional view of an implant composite material of the second type as still a further embodiment of the invention.
[Fig. 26]

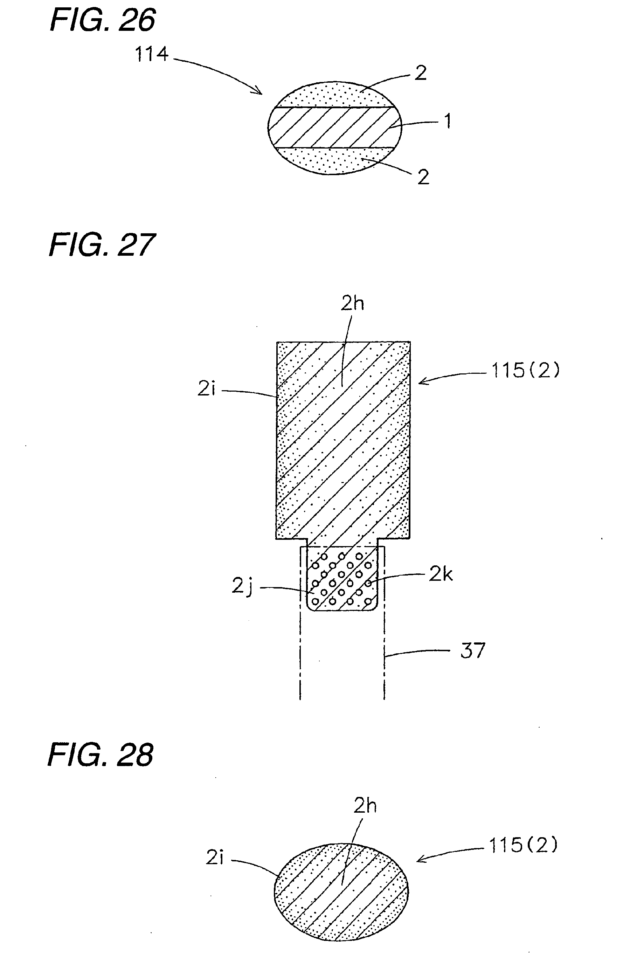

   Fig. 26 is a cross-sectional view of an implant composite material of the second type as still a further embodiment of the invention.
[Fig. 27]
   Fig. 27 is a vertical sectional view illustrating one example of modifications of implant composite materials of the second type.
[Fig. 28]
   Fig. 28 is a cross-sectional view of the implant composite material.
[Fig. 29]

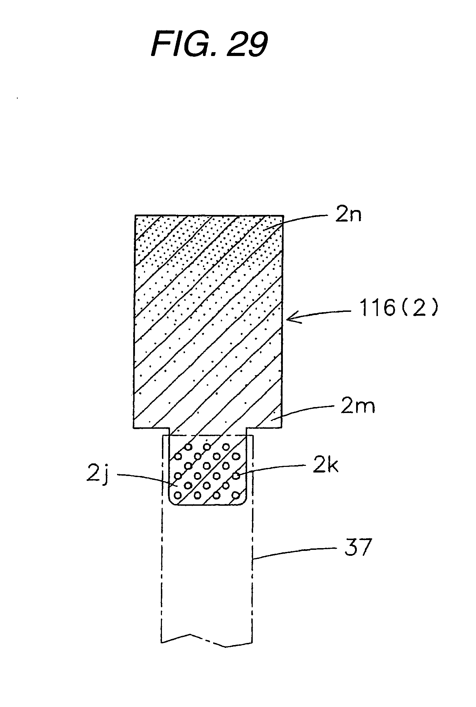

   Fig. 29 is a vertical sectional view illustrating another example of the modifications of implant composite materials of the second type.
[Fig. 30]

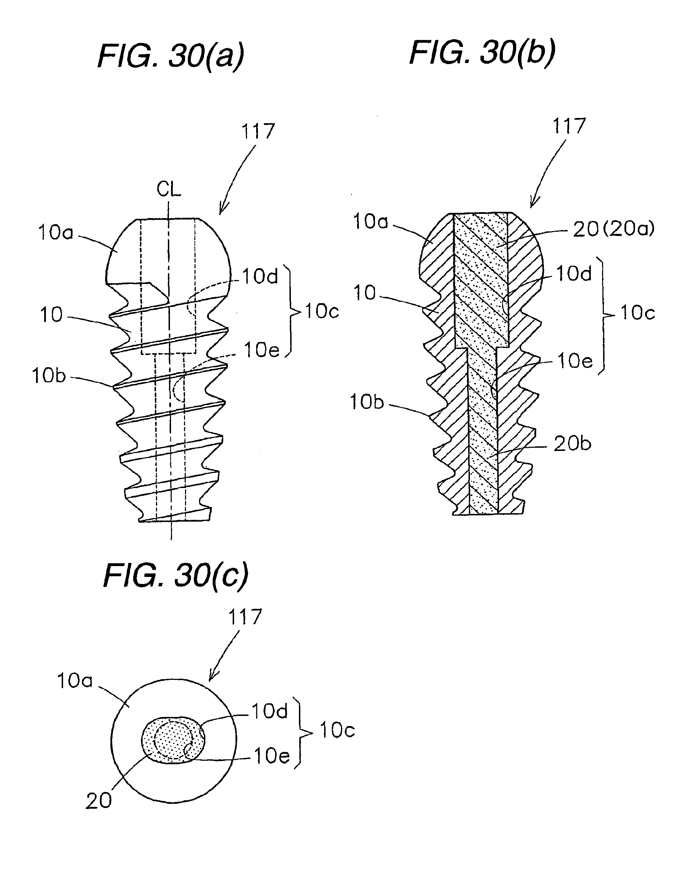

   Fig. 30 illustrates an implant composite material of the third type as still a further embodiment of the invention: (a), (b), and (c) are a front view, vertical sectional view, and plan view thereof, respectively.
[Fig. 31]
   Fig. 31 illustrates one example of sets for tendon or ligament fixing: (a) is a front view of an interference screw in the set; (b) is a front view of a packing in the set; and (c) is a front view of a container in the set, the container containing a biological bone growth factor.
[Fig. 32]

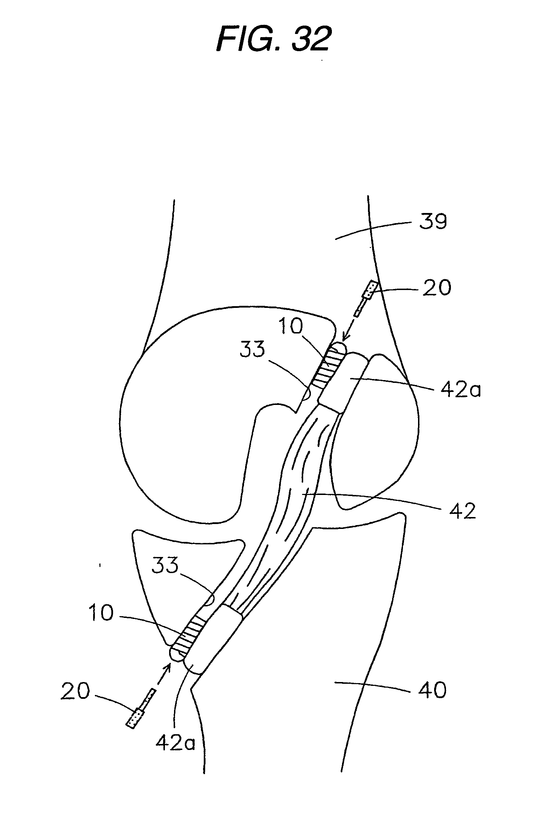

   Fig. 32 is a view illustrating an example in which the set for tendon or ligament fixing is used.
[Fig. 33]

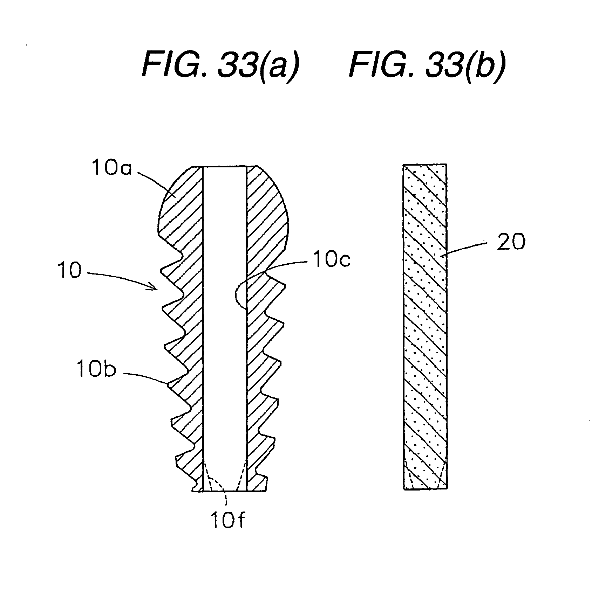

   Fig. 33 illustrates another example of the sets for tendon or ligament fixing: (a) is a vertical front view of a screw in the set and (b) is a vertical sectional view of a packing in the set.
[Fig. 34]

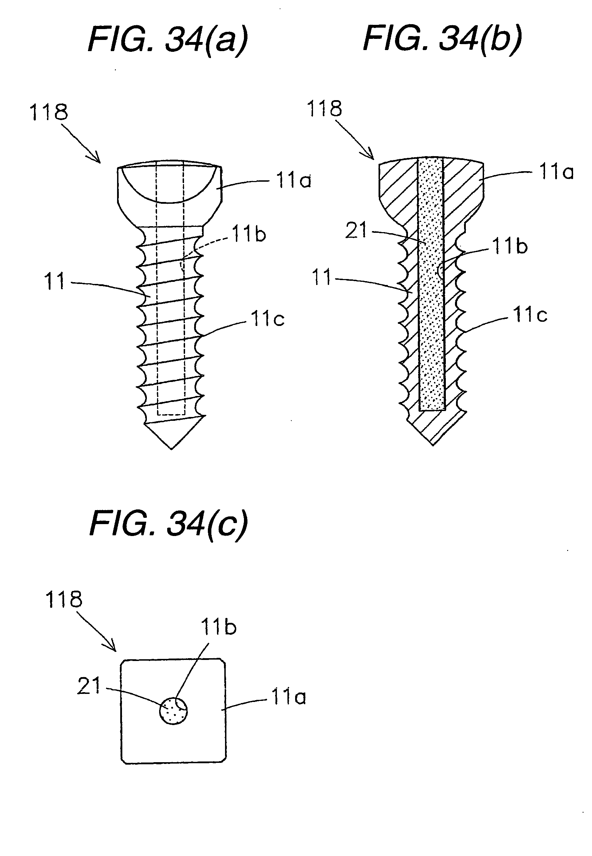

   Fig. 34 illustrates an implant composite material of the fourth type as still a further embodiment of the invention: (a), (b), and (c) are a front view, vertical sectional view, and plan view thereof, respectively.
[Fig. 35]

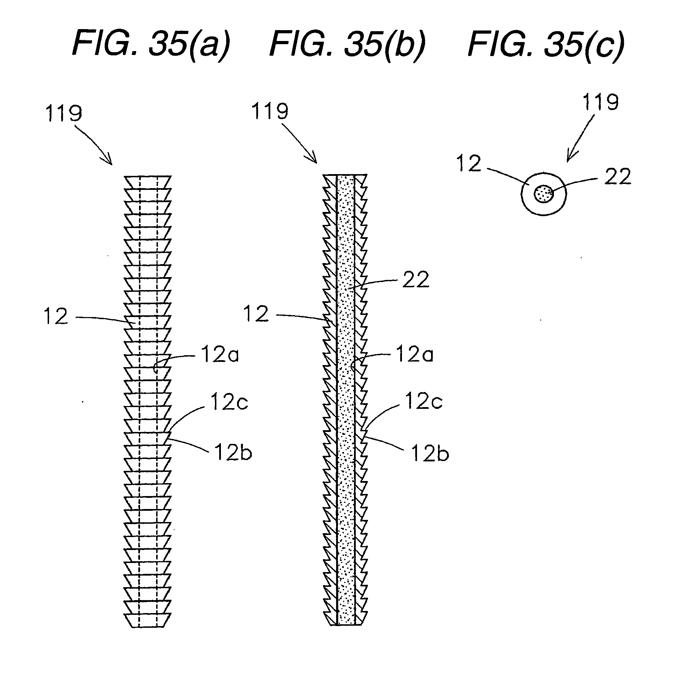

   Fig. 35 illustrates an implant composite material of the fourth type as still a further embodiment of the invention: (a), (b), and (c) are a front view, vertical sectional view, and plan view thereof, respectively.
[Fig. 36]

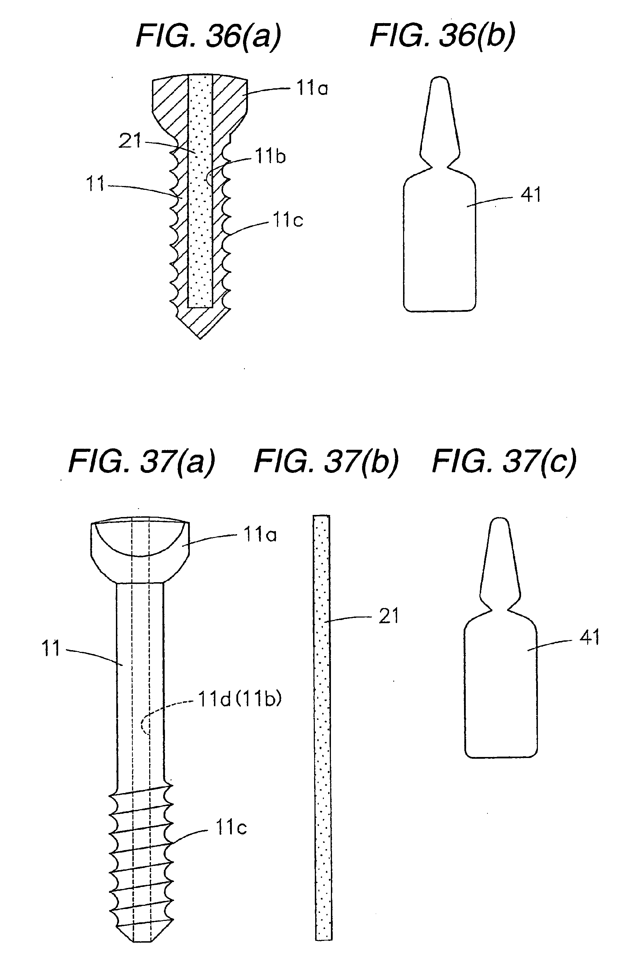

   Fig. 36 illustrates one example of sets of bone-uniting materials: (a) is a vertical sectional view of a filler-filled bone-uniting material main body in the set and (b) is a front view of a container in the set, the container containing a biological bone growth factor.
[Fig. 37]
   Fig. 37 illustrates another example of the sets of bone-uniting materials: (a) is a front view of a bone-uniting material main body in the set, (b) is a front view of a filler in the set, and (c) is a front view of a container in the set, the container containing a biological bone growth factor.
[Fig. 38]

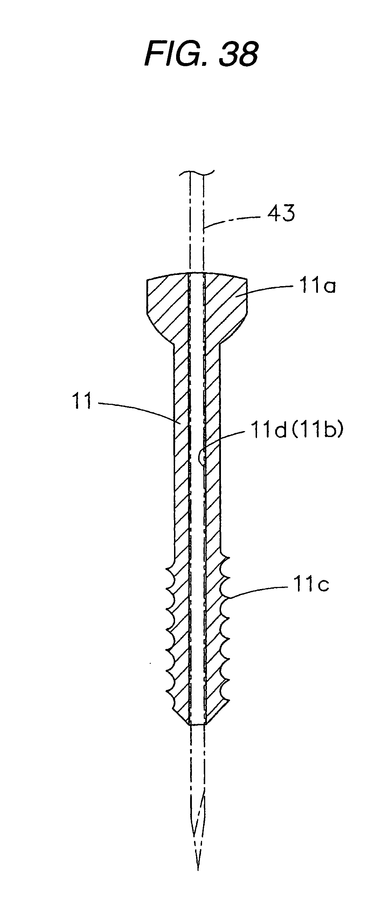

   Fig. 38 is a vertical sectional view of the bone-uniting material main body in the bone-uniting material set.
[Fig. 39]

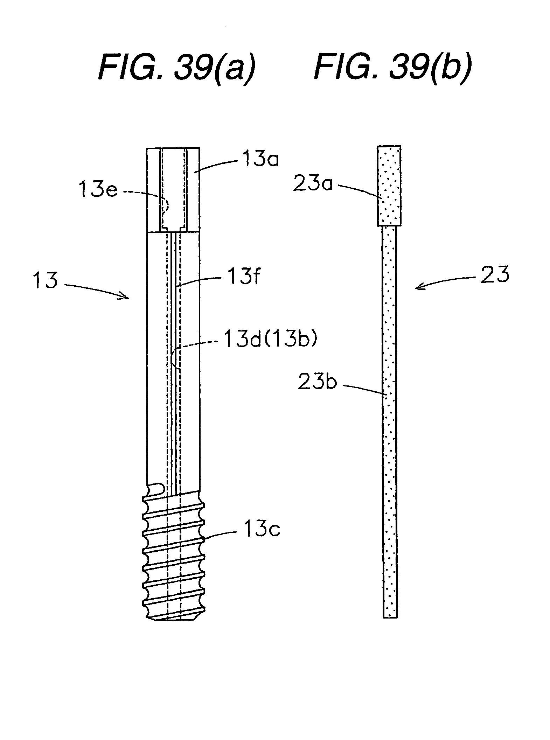

   Fig. 39 illustrates still another example of the sets of bone-uniting materials: (a) is a front view of a bone-uniting material main body in the set and (b) is a front view of a filler in the set.
[Fig. 40]

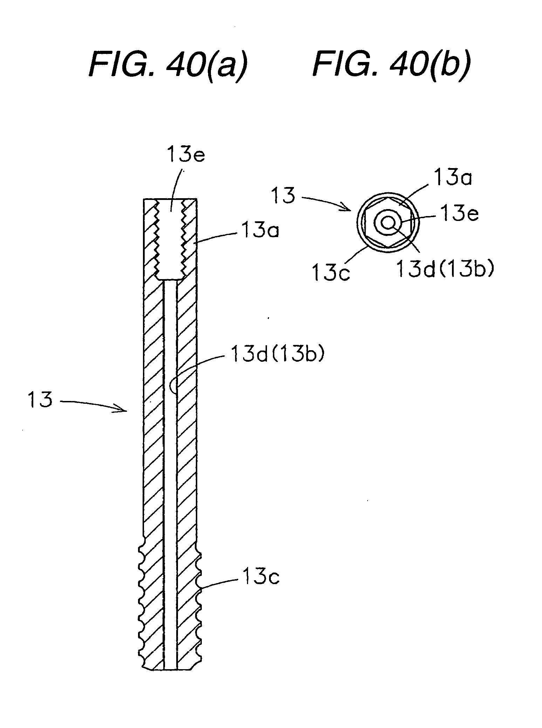

   Fig. 40 (a) is a vertical sectional view of the bone-uniting material main body in the bone-uniting material set and (b) is a plan view thereof.

### DESCRIPTION OF REFERENCE NUMERALS AND SINGS

1 compact composite
2 porous composite
10 interference screw
10c, 11d, 13d through-hole
11, 13 screw
11a screw head
11b, 12b, 13b hole
12 pin
20 packing
21, 22, 23 filler
37 ligamental member
43 Kirschner wire

### BEST MODE FOR CARRYING OUT THE INVENTION

Specific embodiments of the invention will be described below in detail by reference to drawings.

Fig. 1 is a slant view of an implant composite material of the first type as one embodiment of the invention; Fig. 2 is a view illustrating an example in which this implant composite material is used; and Fig. 3 is an enlarged sectional view illustrating part of the implant composite material.

The implant composite material 100 shown in Fig. 1 is an implant composite material of the first type which comprises a compact composite 1 and a porous composite 2 superposed on and united with one side (upper side in this embodiment) of a surface-layer part of the compact composite 1.

The compact composite 1 is a compact block composite comprising a biodegradable and bioabsorbable polymer containing bioabsorbable and bioactive bioceramic particles. Although the compact composite 1 in this embodiment is in the form of a solid cylinder, it can have a quadrangular solid prism, elliptic solid cylinder, or flat plate shape or any of other various shapes according to the joint part into which the implant composite material is to be implanted. The size of the compact composite 1 also is not limited, and may be one suitable for the joint part into which the implant composite material is to be implanted.

This compact composite 1 is required to have a high strength which is equal to or higher than that of the hard bone of the joint. Because of this, the biodegradable and bioabsorbable polymer to be used as a raw material preferably is a crystalline polymer such as poly(L-lactic acid) or poly(glycolic acid). Especially suitable is the compact composite 1 obtained from poly(L-lactic acid) having a viscosity-average molecular weight of about 150, 000 or higher, preferably about 200,000-600,000.

The bioceramic particles to be incorporated into this compact composite 1 preferably are particles which have bioactivity, are bioabsorbable and wholly assimilated by the living body and completely replaced by bone tissues, and have satisfactory bone conductivity (inductivity) and satisfactory biocompatibility. Example thereof include uncalcined and unsintered particles of hydroxyapatite, dicalcium phosphate, tricalcium phosphate, tetracalcium phosphate, octacalcium phosphate, calcite, Ceravital, diopside, and natural coral. Of these, uncalcined and unsintered hydroxyapatite, tricalcium phosphate, and octacalcium phosphate are optimal because they have exceedingly high bioactivity and excellent bone conductivity, are low invasive, and are assimilated by the living body in a short time period. The particles of any of these bioceramics to be used have a particle diameter of 30 µm or smaller, preferably 10 µm or smaller, more preferably about 0.1-5 µm, from the standpoints of dispersibility in the biodegradable and bioabsorbable polymer and bioabsorbability. The content of the bioceramic particles will be explained later.

The compact composite 1 is produced, for example, by a method in which a biodegradable and bioabsorbable polymer containing bioceramic particles is injection-molded into a solid cylinder or another given shape or a method in which a molded object of a biodegradable and bioabsorbable polymer containing bioceramic particles is cut into a solid cylinder or another given shape. In particular, the compact composite 1 obtained by the latter method in which a molded object in which polymer molecules and crystals have been oriented is formed by compression molding or forging and this molded object is cut is exceedingly suitable. This is because this compact composite 1 is highly compact due to the compression and has a further enhanced strength due to the three-dimensionally oriented polymer molecules and crystals. Also usable besides these is a compact composite obtained by cutting a molded object obtained by stretch forming.

On the other hand, the porous composite 2 is a porous object which has interconnected pores inside and comprises a biodegradable and bioabsorbable polymer containing bioabsorbable and bioactive bioceramic particles. Part of the bioceramic particles are exposed in the surfaces of this porous composite 2 and in inner surfaces of the interconnected pores. Although the porous composite 2 in this embodiment is in a disk form so as to conform to the cylindrical compact composite 1, it can have any of various shapes such as a square platy shape and an elliptic platy shape according to the shape of the compact composite. Furthermore, the thickness of this porous composite 2 is not particularly limited as long as this composite is thinner than the compact composite 1. However, when the inductive growth of (cartilage) bone tissues and the property of bonding with living (cartilage) bones are taken into account, the thickness of the porous composite 2 is preferably about 0.5-15 mm. The thickness thereof may differ from part to part to form recesses and protrusions.

This porous composite 2 need not have a high strength such as that of the compact composite 1, and a strength and flexibility such as those of cartilages suffice for the composite 2. This porous composite 2 is required to be rapidly degraded and undergo bonding with and complete replacement by a living (cartilage) bone in an early stage. Because of this, a biodegradable and bioabsorbable polymer which is safe, can be rapidly degraded, is not so brittle, and is amorphous or a mixture of crystalline and amorphous phases is suitable for use as a raw material for the porous composite 2. Examples thereof include poly(D,L-lactic acid), copolymers of L-lactic acid and D,L-lactic acid, copolymers of a lactic acid and glycolic acid, copolymers of a lactic acid and caprolactone, copolymers of a lactic acid and ethylene glycol, and copolymers of a lactic acid and p-dioxanone. These may be used alone or as a mixture of two or more thereof. When the strength required of the porous composite 2, the period of biodegradation, etc. are taken into account, those biodegradable and bioabsorbable polymers to be used preferably have a viscosity-average molecular weight of about 50,000-600,000.

It is desirable that the porous composite 2 should be one in which the porosity thereof is 50-90%, preferably 60-80%, interconnected pores account for 50-90%, preferably 70-90%, of all pores, and the interconnected pores have a pore diameter of 50-600 µm, preferably 100-400 µm, when physical strength, osteoblast penetration, stabilization, etc. are taken into account. In case where the porous composite 2 has a porosity exceeding 90% and a pore diameter larger than 600 µm, this porous composite 2 has reduced physical strength and is brittle. On the other hand, when the porosity thereof is lower than 50%, the proportion of interconnected pores is lower than 50% based on all pores, and the pore diameter is smaller than 50 µm, then the penetration of a body fluid or osteoblast becomes difficult. In this case, the hydrolysis of the porous composite 2 and the inductive growth of bone tissues therein become slow, and the time period required for bonding with a living bone and for complete replacement by (cartilage) bone tissues is prolonged. However, it has been found that bone inductivity is exhibited when fine interconnected pores on submicron order of 1-0.1 µm coexist with interconnected pores having that preferred pore diameter.

The porosity of the porous composite 2 may be even throughout the whole composite. However, when the property of bonding with a living (cartilage) bone and conductive/inductive growth are taken into account, it is preferred that the porosity thereof should gradually change continuously so that it increases from an inner-layer part to a surface-layer part of the porous composite 2 as shown in Fig. 3. In the porous composite 2 having such a porosity inclination, it is desirable that the porosity thereof should gradually increase continuously from an inner-layer part to a surface-layer part in the range of 50-90%, preferably in the range of 60-80%, and that the pore diameter of the interconnected pores should gradually increase from the inner-layer part to the surface-layer part in the range of 100-400 µm. In the porous composite 2 having such properties, hydrolysis proceeds rapidly on its surface-layer side and osteoblast penetration and the inductive growth of (cartilage) bone tissues are enhanced. This porous composite 2 bonds with a living (cartilage) bone in an early stage. Consequently, the implant composite material can be further improved in the property of bonding with and being replaced by a living (cartilage) bone in an early stage after implantation.

The bioceramic particles to be incorporated into this porous composite 2 may be the same as the bioceramic particles contained in the compact composite 1 described above. However, bioceramic particles having a particle diameter of about 0.1-5 µm are especially preferred because use of such bioceramic particles is free from the possibility of cutting the fibers to be formed, e.g., by spraying in producing the porous composite by the method which will be described later, and because such bioceramic particles have satisfactory bioabsorbability.

The content of the bioceramic particles in the porous composite 2 may be even throughout the whole porous composite 2 or may be uneven. In the former case, in which the content is even, it is preferred that the content of the bioceramic particles should be 60-80% by mass. Contents thereof exceeding 80% by mass result in a trouble that such a high bioceramic-particle content coupled with the high porosity of the porous composite 2 leads to a decrease in the physical strength of the porous composite 2. Contents thereof lower than 60% by mass cause the following trouble. This porous composite 2 has reduced bioactivity and, hence, the inductive growth of (cartilage) bone tissues becomes slow. As a result, bonding with and complete replacement by a living (cartilage) bone take too much time. A more preferred range of the content of the bioceramic particles is 60-70% by mass.

On the other hand, in the latter case, in which the content is uneven, it is preferred that the content of the bioceramic particles in the porous composite 2 should be higher than the bioceramic-particle content in the compact composite 1 and gradually change to have an inclination so that it increases from an inner-layer part to a surface-layer part of the porous composite 2 with interconnected pores in the range of 30-80% by mass. Namely, it is preferred that the bioceramic particle/biodegradable and bioabsorbable polymer proportion by mass in the porous composite 2 should be larger than that mass proportion in the compact composite 1 and gradually change to have an inclination so that it increases from the inner-layer part to the surface-layer part of the porous composite 2 in the range of from 30/70 to 80/20. In the porous composite 2 having such an inclination of bioceramic-particle content, bioactivity is high in the surface-layer side having a high content and the inductive growth of an osteoblast and (cartilage) bone tissues is enhanced especially in the surface-layer side. This porous composite 2 bonds with a living (cartilage) bone and is replaced thereby in an early stage.

In contrast, the content of the bioceramic particles in the compact composite 1 preferably is lower than the bioceramic-particle content in the porous composite 2 and is in the range of 30-60% by mass. Contents thereof exceeding 60% by mass result in a trouble that the compact composite 1, which is required to be strong, becomes brittle and come to have a deficiency in strength. Contents thereof lower than 30% by mass result in a trouble that the conductive bone formation by the action of the bioceramic particles becomes insufficient and complete replacement by (cartilage) bone tissues requires much time. The content of the bioceramic particles therein may be even throughout the whole compact composite 1 or may change to have an inclination so that it gradually increases from a central part toward a surrounding surface-layer part of the compact composite 1 or from the bottom side toward the upper side of the compact composite 1, provided that the content thereof is lower than in the porous composite 2 as stated above and is in the range of 30-60% by mass. In the compact composite 1 having such an inclination of bioceramic-particle content, the surface-layer part or upper side having a high content undergoes the conductive growth of (cartilage) bone tissues while the central part or bottom side having a low content retains strength. Finally, this compact composite 1 is wholly replaced.

Incidentally, in the case where the content of the bioceramic particles in each of the compact composite 1 and the porous composite 2 is to be inclined, it is preferred that the content of the bioceramic particles should be gradually changed continuously so that it increases from the bottom side of the compact composite 1 to the upper side of the porous composite 2 or that it increases from a central part of the compact composite 1 to the upper side of the porous composite 2 and to the lateral sides and bottom side of the compact composite 1, in the range of 30-80% by mass.

It is preferred that this porous composite 2 should be impregnated with at least one biological bone growth factor selected from a BMP (Bone Morphogenic Protein), TGF-β (Transforming Growth Factor β), EP4 (Prostanoid Receptor), b-FGF (basic Fibroblast Growth Factor), and PRP (platelet-rich plasma) and/or an osteoblast derived from a living organism. By impregnating the composite 2 with any of these biological bone growth factors or the osteoblast, osteoblast multiplication and growth are greatly accelerated. As a result, (cartilage) bone tissues come to grow in the surface side of the porous composite 2 in an extremely short time period (about 1 week) and the porous composite 2 is wholly replaced by (cartilage) bone tissues rapidly thereafter, whereby the living (cartilage) bone is repaired/reconstructed. Of those factors, TGF-β and b-FGF are especially effective in cartilage growth and BMPs and EP4 are especially effective in hard-bone growth. It is therefore preferred that the composite 2 should be impregnated with TGF-β or b-FGF when the living bone to be regenerated is a cartilage and with a BMP or EP4 when the living bone to be regenerated is a hard bone. On the other hand, PRP is a plasma having a highly elevated platelet concentration and addition thereof accelerates the growth of a newly regenerated bone. In some cases, another growth factor such as IL-1, TNF-α, TNF-β, or IFN-γ or a drug may be infiltrated.

The surface of this porous composite 2 may be subjected to an oxidation treatment such as corona discharge, plasma treatment, or hydrogen peroxide treatment. Such an oxidation treatment has an advantage that bonding with a living (cartilage) bone and total replacement thereby are further accelerated because the wettability of the surface of the porous composite 2 is improved to enable an osteoblast to more effectively penetrate into and grow in interconnected pores of this composite 2. The surface of the compact composite 1 may, of course, be subjected to such an oxidation treatment.

The porous composite 2 is produced, for example, by the following process. First, a biodegradable and bioabsorbable polymer is dissolved in a volatile solvent and bioceramic particles are mixed with the solution to prepare a suspension. This suspension is formed into fibers by spraying or another technique to produce a fibrous mass composed of fibers intertwined with one another. This fibrous mass is immersed in a volatile solvent such as methanol, ethanol, isopropanol, dichloroethane(methane), or chloroform to bring it into a swollen or semi-fused state. The fibrous mass in this state is pressed to obtain a porous fusion-bonded fibrous mass in a disk form such as that shown in Fig. 1. The fibers in this fusion-bonded fibrous mass are shrunk and fused, and are thereby deprived substantially of their fibrous shape to form a matrix. Thus, the fibrous mass is changed in form into a porous composite in which the spaces among the fibers have been changed into rounded interconnected pores.

In the case where a porous composite in which the porosity increases from an inner-layer part to a surface-layer part is to be produced by that process, a method, wherein when the fibrous mass is immersed in the volatile solvent to bring it into a swollen or semi-fused state and is then pressed to obtain a porous fusion-bonded fibrous mass, the amount of the fibrous mass is regulated so as to decrease from the inner-layer part to the surface-layer part, may be used. On the other hand, in the case where a porous composite which has interconnected pores and in which the content of bioceramic particles increases from an inner-layer part to a surface-layerpart is tobe produced, a method which comprises preparing several suspensions differing in the amount of bioceramic particles incorporated, forming several fibrous masses differing in bioceramic-particle content, superposing these fibrous masses in order of increasing bioceramic-particle content, bringing this assemblage into a swollen or semi-fused stage, and pressing it, may be used.

The implant composite material 100 shown in Fig. 1 is one obtained by superposing the porous composite 2 in a disk form on the upper side of the compact composite 1 in a solid cylinder form and uniting these by, e.g., thermal fusion bonding or another technique. Techniques for uniting the compact composite 1 with the porous composite 2 are not limited to thermal fusion bonding. For example, the two members may be united by bonding with an adhesive, or a method may be used which comprises forming a dovetail groove in one of the contact surfaces of the compact composite 1 and the porous composite 2, forming a dovetail on the other contact surface, and fitting the dovetail into the dovetail groove to unite the two composites.

When the implant composite material 100 described above is used for the treatment of an articular cartilage disorder such as, e.g., knee bone head necrosis, it is used in the manner shown in Fig. 2. Namely, the necrotized part of the knee bone head is excised, and the compact composite 1 (preferably one containing a BMP, EP4, or PRP, which each are effective in hard-bone growth) of the implant composite material 100 is implanted in the excised part 30 and fixed. The porous composite 2 (preferably one containing TGF-β or b-FGF, which each are a biological growth factor effective for cartilages) is disposed on the cartilage 31 side so as to be flush therewith. After the implant material 100 is thus implanted, the hydrolysis of the porous composite 2 by a body fluid in contact with the surface of the composite 2 and by a body fluid which has penetrated into interconnectedpores inside proceeds rapidly from the surface and inner parts thereof. By the action of the bioactive bioceramic particles, cartilage tissues inductively grow in an extremely short time period in that peripheral lateral surface of the porous composite 2 which is in contact with the cartilage 31, whereby the porous composite 2 bonds with the cartilage 31. The porous composite 2 is wholly replaced by cartilage tissues and disappears rapidly thereafter. On the other hand, the compact composite 1 undergoes hydrolysis to some degree. However, it retains a sufficient strength until the porous composite 2 is nearly replaced by cartilage tissues. Thereafter, hydrolysis of the compact composite 2 further proceeds and, with this hydrolysis, hard-bone tissues of the knee joint bone head conductively grow in inner parts of the compact composite 1 due to the bone conductivity of the bioceramic particles. Finally, the compact composite 1 is replaced by the hard-bone tissues and disappears. Furthermore, the bioceramic particles contained in the porous composite 2 and compact composite 1 also are completely assimilated and disappear. Thus, the knee joint cartilage disorder part is completely repaired/reconstructed.

Fig. 4 is a sectional view of an implant composite material of the first type as another embodiment of the invention.

This implant composite material 101 is an implant composite material of the first type in which a porous composite 2 has been superposed on and united with all surfaces of the surface-layer parts, i.e., the upper side, lateral sides, and bottom side, of a compact composite 1. The compact composite 1 and the porous composite 2 have the same constitutions as those in the implant composite material 100 described above, and explanations thereon are hence omitted.

This implant composite material 101 has the following advantage besides the effects and advantages of the implant composite material 100 described above. After the compact composite 1 is implanted in an excised part of an articular bone, hard-bone tissues conductively grow in the porous composite 2 superposed on and united with the lateral sides and bottom side of the compact composite 1. As a result, the compact composite 1 bonds with the inner surface of the excised part of the articular bone and is fixed thereto in an extremely short time period.

Fig. 5 is a sectional view of an implant composite material of the first type as still another embodiment of the invention, and Fig. 6 is a view illustrating an example in which this implant composite material is used.

This implant composite material 102 is an implant composite material of the first type which comprises a compact composite 1 of a solid prism shape and porous composites 2 and 2 of a square plate shape which are thinner than the composite 1 and have been superposed on and united with the upper and lower sides, respectively, of the surface-layer part of the compact composite 1. The compact composite 1 and each porous composite 2 have the same constitutions as those in the implant composite material 100 described above, and explanations thereon are hence omitted.

This implant composite material 102 is, for example, inserted as a spacer between vertebrae in a joint part, such as the vertebral column, lumbar vertebrae, or cervical vertebrae, as shown in Fig. 6. After the implant composite material 102 is thus inserted, the porous composites 2 and 2 respectively in contact with the upper and lower vertebrae 32 and 32 rapidly hydrolyze, and bone tissues conductively grow from the upper and lower vertebrae 32 and 32 and penetrate into surface-layer parts of the porous composites 2 and 2. The porous composites 2 and 2 hence bond with the vertebrae in a short time period, whereby the implant composite material 102 does not detach from the joint part. These porous composites 2 and 2 are wholly replaced by bone tissues and disappear in an early stage. On the other hand, the compact composite 1 retains a strength over a certain time period. Thereafter, however, the conductive growth of bone tissues proceeds in the compact composite 1, which finally is wholly replaced by the bone tissues and disappears.

In the implant composite material 102 to be inserted as a spacer between vertebrae as described above, too large thicknesses of the porous composites 2 and 2 result in a possibility that these porous composites 2 and 2 are compressed from the upper and lower directions to narrow the vertical space between the vertebrae 32 and 32. Consequently, it is preferred to regulate the thickness of each of the porous composites 2 and 2 so as to be as small as about 0.1-2.0 mm. That possibility may be completely eliminated by rotating this implant composite material 102 by 90 degrees and inserting it between vertebrae 32 and 32, with the porous composites 2 and 2 located respectively on the left and right sides of the compact composite 1. When the implant composite material 102 is implanted in this manner, the upper and lower edges of the porous composites 2 and 2 respectively on the left and right sides serve in a bridging stage to bond with the upper and lower vertebrae 32 and 32 in an early stage while maintaining the space between the upper and lower vertebrae 32 and 32 with the compact composite 1 without fail. Thus, the implant composite material 102 can be prevented from detaching.

Fig. 7 is a sectional view of an implant composite material of the first type as a further embodiment of the invention.

This implant composite material 103 also is one to be inserted as a spacer between vertebrae in a joint part, such as the vertebral column, lumbar vertebrae, or cervical vertebrae. It comprises a compact composite 1 which has projections 1a formed in a serrate arrangement on the upper and lower sides of the surface-layer part thereof and a porous composite 2 superposed on and united with each of the upper and lower sides of the compact composite 1 so that the porous composite 2 fills the recesses between the projections 1a and 1a. The compact composite 1 and the porous composite 2 have the same constitutions as those in the implant composite material 100 described above, and explanations thereon are hence omitted.

This implant composite material 103 is inserted between vertebrae so that the inclined faces of the serrate projections 1a face the front side, whereby the following advantage is brought about besides the effects and advantages of the implant composite material 102 described above. The projections 1a of the compact composite 1 slightly bite into the upper and lower vertebrae and, hence, the implant composite material 103 can be prevented from coming out from the space between the vertebrae just after the insertion.

In each of the implant composite materials 102 and 103 which are inserted as spacers between vertebrae, the compact composite 1 is solid. However, a hollow compact composite filled inside with a porous composite, living-bone powder, or the like may be employed. This has an advantage that the replacement of the compact composite 1 by bone tissues proceeds rapidly.

Fig. 8 is a sectional viewof an implant compositematerial of the first type as still a further embodiment of the invention, and Fig. 9 is a view illustrating an example in which this implant composite material is used.

This implant composite material 104 is a composite material in a piece form (small piece form) which comprises a compact composite 1 and a porous composite 2 which is thinner than the composite 1 and has been superposed on and united with each of two opposed lateral sides of the surface-layer part of the composite 1. It is for use in the reconstruction or reinforcement of a ligament part adherent to a joint. The compact composite 1 and the porous composite 2 have the same constitutions as those in the implant composite material 100 described above, and explanations thereon are hence omitted.

In the case where this implant composite material 104 is used to conduct the reconstruction or reinforcement of a ligament part adherend to a joint, the following method may, for example, be used as shown in Fig. 9. Holes 33 and 33 are formed respectively in the two bones of a joint, and both ends 34a and 34a of a ligament 34 are inserted into the holes 33 and 33. The implant composite materials 104 and 104 are sandwiched between the two end parts 34a and 34a of the ligament 34 and one side of the inner surfaces of the holes 33 and 33. Interference screws 35 and 35 are screwed into the space between the two end parts 34a and 34a and the opposite side of the inner surfaces of the holes 33 and 33 to fix the ligament 34. In this case, the porous composites 2 and 2 on the two lateral sides of each of the implant composite materials 104 bond with the two end parts 34a and 34a of the ligament 34 and with the inner surfaces of the holes 33 and 33, and are wholly replaced by bone tissues and disappear rapidly thereafter. In addition, the compact composite 1 also is wholly replaced by bone tissues and disappears shortly thereafter. Consequently, the two end parts 34a and 34a of the ligament 34 bond with the holes 33 and 33 through the bone tissues which have wholly replaced. In this case, when the interference screws 35 and 35 are ones comprising a biodegradable and bioabsorbable polymer containing bioactive bioceramic particles, then these screws 35 and 35 also are shortly replaced by bone tissues and bond with the inner surfaces of the holes 33 and 33 and with the two end parts 34a and 34a of the ligament 34, whereby the adherent parts of the ligament have a further improved fixing strength.

Next, implant composite material modifications are explained which eliminate the same problems as those eliminated by the implant composite materials 100, 101, 102, and 104 of the first type described above and can bring about the same effects and advantages as those implant composite materials.

The implant composite materials as such modifications include: (1) one characterized in that it comprises a porous composite of a biodegradable and bioabsorbable polymer containing bioabsorbable and bioactive bioceramic particles and that the porosity gradually changes to have an inclination so that it increases from a surface-layer part on one side of the composite to a surface-layer part on the other side thereof in the range of 10-90%; and (2) one characterized in that it comprises a porous composite of a biodegradable and bioabsorbable polymer containing bioabsorbable and bioactive bioceramic particles and that the porosity gradually changes to have an inclination so that it increases from an intermediate part of the composite to surface-layer parts on both sides thereof or from a central part of the composite to surrounding surface-layer parts thereof, in the range of 10-90%.

The implant composite materials as such modifications (implant inclination materials) have the following advantages. A body fluid is apt to penetrate into interconnected pores in those surface-layer parts of the porous composite which have a high porosity. This porous composite is hence rapidly hydrolyzed from the surfaces and inner parts thereof by a body fluid in contact with the surfaces of the surface-layer parts and by a body fluid which has penetrated into the interconnected pores. Since an osteoblast also is apt to penetrate into the surface-layer parts having a high porosity, the inductive growth of (cartilage) bone tissues is triggered by the bioactive bioceramic particles and proceeds, with the hydrolysis, from the surface-layer parts having a high porosity to inner parts. Thus, the surface-layer parts and the inner-layer parts connected thereto which have a relatively high porosity are replaced by (cartilage) bone tissues in a short time period. On the other hand, that surface-layer part on one side or that intermediate part or central part of the porous composite which has a low porosity has a strength and hydrolyzes far more slowly than the surface-layer parts having a high porosity. It retains the strength until the hydrolysis proceeds to a certain degree and is wholly degraded finally. A living bone is conductively formed by the action of the bioactive bioceramic particles and the low-porosity part is thus replaced by bone tissues. Since the bioceramic particles contained in this porous composite are bioabsorbable, they neither remain/accumulate in the (cartilage) bone tissues which have replaced and regenerated nor come into soft tissues or blood vessels.

As described above, those implant composite materials as modifications (inclination materials) have the properties or functions required of scaffold materials for use in, e.g., the treatment of articular cartilage disorders. Because of this, when the former implant composite material (inclination material), in which the porosity gradually changes to have an inclination so that it increases from a surface-layer part on one side to a surface-layer part on the other side, is implanted in and fixed to, for example, a part where a necrotized part of an articular bone head has been excised, so that the surface-layer part having a high porosity is located on the cartilage side of the articular bone head surface, then it functions by the following mechanism. The surface-layer part having a high porosity and the inner-layer parts connected thereto which have a relatively high porosity are wholly replaced by cartilage tissues inductively grown in an early stage and disappear. Furthermore, the part on the opposite side, which has a low porosity and has a strength, also is wholly replaced finally by conductively grown hard-bone tissues and disappears. The bioceramic particles also are completely assimilated. Thus, the hard-bone part and cartilage part of the necrotized articular bone head part are regenerated. On the other hand, when the latter implant composite material (inclination material), in which the porosity gradually changes to have an inclination so that it increases from an intermediate part of the composite to surface-layer parts on both sides thereof or from a central part of the composite to surrounding surface-layer parts thereof, is implanted in an excised part of an articular bone head, the following effect/advantage is brought about besides those described above. Hard-bone tissues rapidly grow inductively in the surface-layer parts having a high porosity which are in contact with the hard bone in the excised part, whereby the implant composite material (inclination material) bonds with and is fixed to the excised part of the articular bone head in a short time period.

The content of the bioceramic particles may be even throughout the porous composite. However, in the former implant composite material (inclination material), it is preferred that the content of the bioceramic particles should gradually change to have an inclination so that it increases from a surface-layer part on one side of the porous composite to a surface-layer part on the opposite side thereof in the range of 30-80% by mass. In the latter implant composite material (inclination material), it is preferred that the content of the bioceramic particles should gradually change to have an inclination so that it increases from an intermediate part of the porous composite to surface-layer parts on both sides thereof or from a central part of the composite to surrounding surface-layer parts thereof, in the range of 30-80% by mass. In these implant composite materials (inclination materials) in which the content of the bioceramic particles changes to have such an inclination, the surface-layer parts having a high bioceramic-particle content have higher bioactivity. Because of this, the inductive growth of an osteoblast and (cartilage) bone tissues in the surface-layerparts is especially enhanced and replacement by (cartilage) bone tissues is further accelerated.

It is preferred to incorporate at least one biological bone growth factor selected from a BMP, TGF-β, EP4, b-FGF, and PRP and/or an osteoblast derived from a living organism into those implant composite materials (inclination materials) as modifications. In the implant composite materials (inclination materials) containing any of those growth factors or the osteoblast, osteoblast multiplication/growth is greatly accelerated and, hence, (cartilage) bone tissues grow vigorously. Thus, regeneration proceeds more rapidly.

Examples of such implant composite material (inclination material) modifications will be explained below by reference to drawings.

Fig. 10 (a) is a slant view illustrating one example of modifications of implant composite materials of the first type, and Fig. 10 (b) is a diagrammatic sectional view of this implant composite material. Fig. 11 is a view illustrating an example in which this implant composite material is used.

This implant composite material (inclination material) 105 comprises a porous composite 2 of a biodegradable and bioabsorbable polymer containing bioabsorbable and bioactive bioceramic particles, and the porosity thereof gradually changes to have an inclination so that it increases from a surface-layer part 2a on one side (surface-layer part on the lower side) of the composite to a surface-layer part 2b on the opposite side (surface-layer part on the upper side) in the range of 10-90%. Although the implant composite material (inclination material) 105 comprising this porous composite 2 is in the form of a solid cylinder as shown in Fig. 10 (a), the shape thereof is not limited to it. The implant composite material can have a quadrangular solid prism, elliptic solid cylinder, or flat plate shape or any of other various shapes according to the joint part into which the implant composite material is to be implanted. The size thereof also may be one optimal for the joint part into which the implant composite material is to be implanted.

The biodegradable and bioabsorbable polymer to be used as a raw material for this porous composite 2 may be a crystalline polymer such as poly(L-lactic acid) or poly(glycolic acid). However, a biodegradable and bioabsorbable polymer which is safe, can be rapidly degraded, is not so brittle, and is amorphous or a mixture of crystalline and amorphous phases is suitable for use as a raw material for the porous composite 2. This is because the surface-layer part 2b having a high porosity and the inner-layer parts connected thereto having a relativelyhighporosity in this porous composite 2 are required to have a strength and flexibility such as those of cartilages and are further required to be rapidly degraded and undergo bonding with and complete replacement by a living (cartilage) bone in an early stage. Examples of the suitable polymer include poly(D,L-lactic acid), copolymers of L-lactic acid and D, L-lactic acid, copolymers of a lactic acid and glycolic acid, copolymers of a lactic acid and caprolactone, copolymers of a lactic acid and ethylene glycol, and copolymers of a lactic acid and p-dioxanone. These may be used alone or as a mixture of two or more thereof. When the strength required of the porous composite 2, the period of biodegradation, etc. are taken into account, those biodegradable and bioabsorbable polymers to be used preferably have a viscosity-average molecular weight of about 50,000-600,000.

The porous composite 2 constituting this implant composite material 105 has interconnected pores inside and contains bioceramic particles, part of which are exposed in inner surfaces of the interconnected pores and in the surfaces of the composite 2. As stated above, the porosity of this porous composite 2 gradually changes continuously so that it increases from a surface-layer part 2a on one side (surface-layer part on the lower side) to a surface-layer part 2b on the opposite side (surface-layer part on the upper side) in the range of 10-90%, preferably in the range of 20-80%. It is preferred that the interconnected pores should account for 50-90%, especially 70-90%, of all pores. The pore diameter of the interconnected pores has been regulated so as to be in the range of 50-600 µm, preferably in the range of 100-400 µm; the pore diameter increases toward that surface-layer part 2b on one side which has a high porosity.

Such inclinations of porosity, etc. have the following advantages. The surface-layer part 2b on one side having a high porosity (hereinafter referred to as high-porosity surface-layer part) of the porous composite 2 is rapidly hydrolyzed because a body fluid easily penetrates thereinto. In addition, an osteoblast is apt to penetrate thereinto and this, coupled with the high content of the bioactive bioceramic particles as will be described later, enables (cartilage) bone tissues to inductively grow in an early stage. This porous composite 2 thus bonds with a living (cartilage) bone and is replaced thereby. In case where the high-porosity surface-layer part 2b has a porosity exceeding 90% and a pore diameter larger than 600 µm, this high-porosity surface-layer part 2b is undesirable because it has a reduced physical strength and is brittle. In case where the interconnected pores account for less than 50% of all pores and have a pore diameter smaller than 50 µm, this surface-layer part 2b is undesirable because the penetration of a body fluid and an osteoblast thereinto is difficult and hydrolysis and the inductive growth of bone tissues are slow, resulting in a prolonged time period required for bonding with and replacement by a living (cartilage) bone. However, it has been found that bone inductivity is exhibited when fine interconnected pores on submicron order of 1-0.1 µm coexist with interconnected pores having that preferred pore diameter.

On the other hand, that surface-layer part 2a on the opposite side (lower side) which has a low porosity (hereinafter referred to as low-porosity surface-layer part) of the porous composite 2 improves in strength as the porosity decreases. However, since an extremely high strength is not required of implant composite materials to be applied as a scaffold to an articular part, there is no need of regulating the porosity of the low-porosity surface-layer part 2a to a value close to zero. Because of this, the lower limit of the porosity of the low-porosity surface-layer part 2a is regulated to 10%, preferably 20%. Thus, a strength suitable for scaffolds is imparted and the time period required for hydrolysis and complete replacement by (cartilage) bone cells can be reduced.

The bioceramic particles to be incorporated into this porous composite 2 are the same as the bioceramic particles described above, and an explanation thereon is hence omitted.

The content of the bioceramic particles in the porous composite 2 may be even throughout the whole porous composite 2. It is, however, preferred that the content thereof should gradually change to have an inclination so that it increases from the low-porosity surface-layer part 2a to the high-porosity surface-layer part 2b in the range of 30-80% by mass. Namely, it is preferred that the bioceramic particle/biodegradable and bioabsorbable polymer proportion by mass should gradually change to have an inclination so that it increases from the low-porosity surface-layer part 2a to the high-porosity surface-layer part 2b in the range of from 30/70 to 80/20. Such an inclination of bioceramic-particle content has an advantage that the high-porosity surface-layer part 2b has high bioactivity and the inductive growth of an osteoblast and (cartilage) bone tissues therein is especially enhanced, whereby bonding with a living (cartilage) bone and replacement thereby are further accelerated.

In case where the content of the bioceramic particles in the high-porosity surface-layer part 2b exceeds 80% by mass, this arouses a trouble that the high-porosity surface-layer part 2b has a reduced physical strength. In case where the content thereof in the low-porosity surface-layer part 2a is lower than 30% by mass, this arouses a trouble that the inductive growth of (cartilage) bone tissues in the low-porosity surface-layer part 2a by the action of the bioceramic particles becomes slow and, hence, bonding with a living (cartilage) bone and complete replacement thereby take too much time. A more preferred upper limit of the content of the bioceramic particles is 70% by mass.

It is preferred that this porous composite 1 should be impregnated with at least one of the biological bone growth factors described above and/or an osteoblast derived from a living organism. By impregnating the porous composite 2 with these substances, osteoblast multiplication and growth are greatly accelerated. As a result, (cartilage) bone tissues come to grow in the high-porosity surface-layer part 2b of the porous composite 2 in an extremely short time period (about 1 week) and the porous composite 2 is wholly replaced by (cartilage) bone tissues thereafter, whereby the living (cartilage) bone is repaired/reconstructed. Incidentally, the biological bone growth factors which may be infiltrated are the same as the biological bone growth factors described above and an explanation thereon is hence omitted. In some cases, another growth factor such as IL-1, TNF-α, TNF-β, or IFN-γ or a drug may be infiltrated.

The surface of this porous composite 2 may be subjected to an oxidation treatment such as corona discharge, plasma treatment, or hydrogen peroxide treatment. Such an oxidation treatment has an advantage that bonding with a living (cartilage) bone and total replacement thereby are further accelerated because the wettability of the surface of the porous composite 2 is improved to enable an osteoblast to more effectively penetrate into and grow in interconnectedpores of this composite 2.

The implant composite material (inclination material) 105 comprising the porous composite 2 may be produced by substantially the same method as for the porous composite 2 in, e.g., the implant composite material 100 described above. Namely, it may be produced in the following manner. A biodegradable and bioabsorbable polymer is dissolved in a volatile solvent and bioceramic particles are mixed with the solution to prepare a suspension. This suspension is formed into fibers by spraying or another technique to produce a fibrous mass composed of fibers intertwined with one another. This fibrous mass is immersed in a volatile solvent such as methanol, ethanol, isopropanol, dichloroethane(methane), or chloroform to bring it into a swollen or semi-fused state. The fibrous mass in this state is pressed to obtain a porous fusion-bonded fibrous mass in a solid cylinder form such as that shown in Fig. 10. The fibers in this fusion-bonded fibrous mass are shrunk and fused, and are thereby deprived substantially of their fibrous shape to form a matrix. Thus, the fibrous mass is changed in form into a porous composite in which the spaces among the fibers have been changed into rounded interconnected pores. In this operation, the step in which the fibrous mass is immersed in a volatile solvent to bring it into a swollen or semi-fused state and is then pressed to obtain a fusion-bonded fibrous mass may be conducted in such a manner that the amount of the fibrous mass is regulated so as to decrease from one side (lower side) to the opposite side (upper side). As a result, the porous composite 2 in which the porosity gradually increases from a surface-layer part 2a on one side to a surface-layer part 2b on the opposite side can be obtained. In the case where the porous composite 2 in which the content of bioceramic particles increases from a low-porosity surface-layer part 2a to a high-porosity surface-layer part 2b is to be produced, use may be made of a method which comprises preparing several suspensions differing in the amount of bioceramic particles incorporated, forming several fibrous masses differing in bioceramic-particle content, superposing these fibrous masses in order of increasing bioceramic-particle content, bringing this assemblage into a swollen or semi-fused stage, and pressing it.

When the implant composite material (inclination material) 105 described above is used for the treatment of an articular cartilage disorder such as, e.g., knee bone head necrosis, it is used in the manner shown in Fig. 11. Namely, the necrotized part of the knee bone head 36 is excised, and the implant composite material 105 (preferably one in which a BMP, EP4, or PRP, which each are effective in hard-bone growth, has been incorporated in the low-porosity surface-layer part 2a and inner-layer parts connected thereto having a relatively low porosity and TGF-β or b-FGF, which each are a biological growth factor effective for cartilages, has been incorporated in the high-porosity surface-layer part 2b and inner-layer parts connected thereto having a relatively high porosity) is implanted in the excised part 30 and fixed so that the high-porosity surface-layer part 2b is located on the cartilage 31 side and is flush therewith. After the implant composite material 105 is thus implanted, the high-porosity surface-layer part 2b is rapidly hydrolyzed from the surface and inner parts thereof by a body fluid in contact with the surface of the surface-layer part 2b and by a body fluid which has penetrated into interconnected pores inside. By the action of the bioactive bioceramic particles, cartilage tissues inductively grow in an extremely short time period in that peripheral lateral surface of the high-porosity surface-layer part 2b which is in contact with the cartilage 31, whereby the high-porosity surface-layer part 2b bonds with the cartilage 31. Thereafter, the high-porosity surface-layer part 2b and inner-layer parts connected thereto having a relatively high porosity are wholly replaced by cartilage tissues and disappear rapidly. On the other hand, the low-porosity surface-layer part 2a and inner-layer parts connected thereto having a relatively low porosity, in the porous composite 2, undergo hydrolysis to some degree. However, they retain a sufficient strength until the high-porosity surface-layer part 2b is almost replaced by cartilage tissues. Thereafter, with further progress of the hydrolysis, hard-bone tissues of the knee bone head 36 conductively grow in the low-porosity surface-layer part 2a and the inner-layer parts connected thereto having a relatively low porosity due to the bone conductivity of the bioceramic particles. Finally, these parts are replaced by the hard-bone tissues and disappear. Furthermore, the bioceramic particles contained in this porous composite 2 also are completely assimilated and disappear. Thus, the knee joint cartilage disorder part is completely repaired/reconstructed.

Fig. 12 is a diagrammatic sectional view illustrating another example of the modifications of implant composite materials of the first type.

This implant composite material (inclination material) 106 is a cylindrical one comprising a porous composite 2 of a biodegradable and bioabsorbable polymer containing bioabsorbable and bioactive bioceramic particles, like the implant composite material 105 described above. However, it differs from the implant composite material 105 described above in that the porosity thereof gradually changes to have an inclination so that it increases from a central part 2c of the porous composite 2 to surrounding surface-layer parts 2d thereof in the range of 10-90%, preferably in the range of 20-80%, and that the content of the bioceramic particles gradually changes to have an inclination so that it increases from the central part 2c of the porous composite 2 to the surrounding surface-layer parts 2d thereof in the range of 30-80% by mass.

When this implant composite material (inclination material) 106 is implanted in an excised part 30 of an articular bone, the following advantage is brought about besides the effects and advantages of the implant composite material 105 described above. Hard-bone tissues conductively grow rapidly in the surface-layer parts 2d which have a high porosity and a high bioceramic-particle content and are in contact with the inner surface of the excised part 30. As a result, the surface-layer parts 2d bond with the inner surface of the excised part 30 of the articular bone and are fixed thereto in a short time period.

Fig. 13 is a diagrammatic sectional view illustrating still another example of the modifications of implant composite materials of the first type, and Fig. 14 is a view illustrating an example in which this implant composite material is used.

Like the implant composite material 105 described above, this implant composite material (inclination material) 107 is one comprising a porous composite 2 of a biodegradable and bioabsorbable polymer containing bioabsorbable and bioactive bioceramic particles. However, it differs from the implant composite material 105 described above in that it is in the form of a prism, that the porosity thereof gradually changes to have an inclination so that it increases from an intermediate part 2e of the porous composite 2 to surface-layer parts 2f and 2f on the upper and lower sides in the range of 10-90%, preferably in the range of 20-80%, and that the content of the bioceramic particles gradually changes to have an inclination so that it increases from the intermediate part 2e of the porous composite 2 to the surface-layer parts 2f and 2f on the upper and lower sides in the range of 30-80% by mass.

This implant composite material (inclination material) 107 is, for example, inserted as a spacer between vertebrae 32 and 32 in a joint part, such as the vertebral column, lumbar vertebrae, or cervical vertebrae, as shown in Fig. 14. After the implant composite material 107 is thus inserted, the surface-layer parts 2f and 2f, which are high in porosity and bioceramic-particle content, respectively in contact with the upper and lower vertebrae 32 and 32 rapidly hydrolyze, and bone tissues conductively grow from the upper and lower vertebrae 32 and 32 and penetrate into the surface-layer parts 2f and 2f. The surface-layer parts 2f and 2f hence bond with the vertebrae in a short time period, whereby the implant composite material 107 does not detach from the joint part. These surface-layer parts 2f and 2f are wholly replaced by bone tissues and disappear in an early stage. On the other hand, the intermediate part 2e retains a strength over a certain time period. Thereafter, however, the conductive growth of bone tissues proceeds in the intermediate part 2e, which finally is wholly replaced by the bone tissues and disappears.

In the implant composite material (inclination material) 107 to be inserted as a spacer between vertebrae as described above, too large thicknesses of the upper and lower surface-layer parts 2f and2f, whichhave ahighporosity, result inapossibility that these surface-layer parts 2f and 2f are compressed from the upper and lower directions to narrow the vertical space between the vertebrae 32 and 32. Consequently, it is preferred to regulate the thickness of each of the upper and lower surface-layer parts 2f and 2f so as to be as small as about 0.1-2.0 mm. That possibility may be completely eliminated by rotating this implant composite material 107 by 90 degrees and inserting it between vertebrae 32 and 32, with the high-porositysurface-layer parts2fand2flocated respectively on the left and right sides of the low-porosity intermediate part 2e. When the implant composite material 107 is implanted in this manner, the upper and lower edges of the high-porosity surface-layer parts 2f and 2f respectively on the left and right sides serve in a bridging stage to bond with the upper and lower vertebrae 32 and 32 in an early stage while maintaining the space between the upper and lower vertebrae 32 and 32 without fail with the low-porosity intermediate part 2e having a strength. Thus, the implant composite material 107 can be prevented from detaching.

Fig. 15 is a diagrammatic sectional view illustrating a further example of the modifications of implant composite materials of the first type, and Fig. 16 is a view illustrating an example in which this implant composite material is used.

Like the implant composite material 105 described above, this implant composite material (inclination material) 108 is one comprising a porous composite 2 of a biodegradable and bioabsorbable polymer containing bioabsorbable and bioactive bioceramic particles. However, it differs from the implant composite material 105 described above in that it is in the form of a piece (small piece) so as to be suitable for the reconstruction or reinforcement of a ligament part adherent to a joint, that the porosity thereof gradually changes to have an inclination so that it increases from an intermediate part 2e of the porous composite 2 to surface-layer parts 2g and 2g on the left and right sides in the range of 10-90%, preferably in the range of 20-80%, and that the content of the bioceramic particles gradually changes to have an inclination so that it increases from the intermediate part 2e of the porous composite 2 to the surface-layer parts 2g and 2g on the left and right sides in the range of 30-80% by mass.

This implant composite material (inclination material) 108 is for use in the reconstruction or reinforcement of a ligament part adherent to a joint as shown in Fig. 16. Namely, it is used in the following manner as shown in Fig. 16. Holes 33 and 33 are formed respectively in the two bones of a joint, and both ends 34a and 34a of a ligament 34 are inserted into the holes 33 and 33. The implant composite materials 108 and 108 are sandwiched between the two end parts 34a and 34a of the ligament 34 and one side of the inner surfaces of the holes 33 and 33. Interference screws 35 and 35 are screwed into the space between the two end parts 34a and 34a and the opposite side of the inner surfaces of the holes 33 and 33 to fix the ligament 34. In this case, the high-porosity surface-layer parts 2g and 2g on both sides of each of the implant composite materials 108 bond with the two end parts 34a and 34a of the ligament 34 and with the inner surfaces of the holes 33 and 33, and are wholly replaced by bone tissues and disappear rapidly thereafter. In addition, the low-porosity intermediate part 2e having a strength also is wholly replaced by bone tissues and disappears shortly thereafter. Consequently, the two end parts 34a and 34a of the ligament 34 bond with the holes 33 and 33 through the bone tissues which have wholly replaced. In this case, when the interference screws 35 and 35 are ones comprising a biodegradable and bioabsorbable polymer containing bioactive bioceramic particles, then these screws 35 and 35 also are shortly replaced by bone tissues and bond with the inner surfaces of the holes 33 and 33 and with the two end parts 34a and 34a of the ligament 34, whereby the adherent parts of the ligament have a further improved fixing strength.

It is a matter of course that it is preferred to impregnate each of the implant composite materials (inclination materials) 106, 107, and 108 with any of the biological bone growth factors described above and/or an osteoblast derived from a living organism.

The implant composite material of the second type of the invention, which is to be attached as an anchor member to an end part of a ligamental member or tendinous member, will be explained next by reference to drawings.

Fig. 17 is a slant view of an implant composite material of the second type as still a further embodiment of the invention. Fig. 18 is a sectional view taken on the line A-A of Fig. 17. Fig. 19 is a sectional view taken on the line B-B of Fig. 17. Fig. 20 is a slant view of an artificial ligament having this implant composite material attached to each end thereof.

The implant composite material 109 of the second type shown in Fig. 17 to Fig. 19 is to be attached as an anchor member to each end of a ligamental member 37 as shown in Fig. 20 or to each end of a tendinous member. It serves to tenaciously fix both ends of the ligamental member 37 to holes respectively formed in the two living bones of a joint. As shown in Fig. 17, this implant composite material (anchor member) 109 is a member in the form of an elliptic solid cylinder which has a projecting piece 1b formed on a central part of one edge face (edge face on the ligamental member 37 side), and the projecting piece 1b has many small holes 1c bored for attaching the ligamental member 37 thereto. Organic fibers of the ligamental member 37, which will be described later, are passed through these small holes 1c and hitched on, whereby the implant composite material 109 can be attached to the end part of the artificial ligamental member 37 while preventing it from detaching. The shape of the implant composite material 109 is not limited to the elliptic solid cylinder, and the implant composite material 109 can have any shape as long as it can be easily inserted into the holes formed in the upper and lower living bones of a joint such as a knee joint and can be stably fixed with the interference screw to be screwed into the space between the inner surface of each hole and the implant composite material 109.

As shown in Fig. 18 and Fig. 19, this implant composite material 109 for use as an end anchor for ligamental members comprises a compact composite 1 in an elliptic solid cylinder form comprising a biodegradable and bioabsorbable polymer containing bioabsorbable and bioactive bioceramic particles and a porous composite 2 of a biodegradable and bioabsorbable polymer containing bioabsorbable and bioactive bioceramic particles, the porous composite 2 being superposed on and united with part of the surfaces of the compact composite 1, i.e., the peripheral surface of the composite 1 in this embodiment. The projecting piece 1b has been united with and projects from one edge face of the compact composite 1 in an elliptic solid cylinder form.

The compact composite 1, which serves as a core material in the implant composite material 109 for anchoring, is required to have high a strength. Because of this, the biodegradable and bioabsorbable polymer to be used as a raw material preferably is the same as the biodegradable and bioabsorbable polymer used for the compact composite 1 of the implant composite material 100 described above. The bioceramic particles to be incorporated into this compact composite 1 also are the same as the bioceramic particles contained in the compact composite 1 of the implant composite material 100 described above, and an explanation thereon is hence omitted.

This compact composite 1 is produced, for example, by a method in which a biodegradable and bioabsorbable polymer containing bioceramic particles is injection-molded into an elliptic solid cylinder having a projecting piece 1b on one edge face thereof and this projecting piece 1b is subjected to drilling or by a method in which a molded object of a biodegradable and bioabsorbable polymer containing bioceramic particles is cut into an elliptic solid cylinder having a projecting piece 1b on one edge face thereof and this projecting piece 1b is subjected to drilling. In particular, the compact composite 1 obtained by the latter method in which a molded obj ect in which polymer molecules and crystals have been oriented is formed by compression molding or forging and this molded object is cut is exceedingly suitable. This is because this compact composite 1 is highly compact due to the compression and has a further enhanced strength due to the three-dimensionally oriented polymer molecules and crystals. Also usable besides these is a compact composite obtained by cutting a molded object obtained by stretch forming.

On the other hand, the layer of the porous composite 2 is a porous object which has interconnected pores inside and comprises a biodegradable and bioabsorbable polymer containing bioabsorbable and bioactive bioceramic particles. Part of the bioceramic particles are exposed in the surfaces of this porous composite 2 and in inner surfaces of the interconnected pores. Although the porous composite 2 in this implant composite material (anchor member) 109 as an embodiment has been superposed only on the peripheral surface of the compact composite 1 in an elliptic solid cylinder form, the porous composite 2 may be superposed on and united with all surfaces of the compact composite 1 except the projecting piece 1b, i.e., the peripheral surface and both edge faces of the compact composite 1.

The thickness of the layer of the porous composite 2 is not particularly limited as long as this composite 2 is thinner than the compact composite 1. However, when the inductive growth of bone tissues and the property of bonding with living bones are taken into account, the thickness thereof is preferably about 0.5-15 mm. Furthermore, the thickness of the layer of the porous composite 2 need not be always even, and may differ from part to part as in, e.g., a porous-composite layer having recesses and protrusions.

The layer of the porous composite 2 need not have a high strength such as that of the compact composite 1, but is required to be rapidly hydrolyzed and undergo bonding with and complete replacement by a living bone in an early stage. Because of this, the biodegradable and bioabsorbable polymer to be used as a raw material for the porous composite 2 preferably is the same as the biodegradable and bioabsorbable polymer used for the porous composite 2 of the implant composite material 100 described above.

It is desirable that the layer of the porous composite 2 shouldbe one inwhich the porosity thereof is 50-90%, preferably 60-80%, interconnected pores account for 50% ormore, preferably 70-90%, of all pores, and the interconnected pores have a pore diameter of 50-600 µm, preferably 100-400 µm, when physical strength, osteoblastpenetration, stabilization, etc. are taken into account, as in the case of the porous composite 2 of the implant composite material 100 described above. The reasons for this are as described above with regard to the porous composite 2 of the implant composite material 100, and an explanation thereon is hence omitted.

The porosity of the porous composite 2 may be even throughout the whole composite. However, when the property of bonding with a living bone and conductive/inductive growth are taken into account, it is preferred that the porosity thereof should gradually change to have an inclination so that it increases from an inner-layer part to a surface-layer part of the layer of the porous composite 2 as in the case of the porous composite 2 of the implant composite material 100. In the layer of the porous composite 2 having such a porosity inclination, it is desirable that the porosity thereof should gradually increase continuously from an inner-layer part to a surface-layer part in the range of 50-90%, preferably in the range of 60-80%, and that the pore diameter of the interconnected pores should gradually increase from the inner-layer part to the surface-layer part in the range of 50-600 µm. In the layer of the porous composite 2 having such properties, hydrolysis proceeds rapidly on its surface-layer side and osteoblast penetration and the inductive growth of bone tissues are enhanced. This porous composite 2 bonds with a living bone in an early stage. Consequently, the strength of fixing of the implant composite material (anchor member) 109 to the upper or lower living bone of a knee joint or the like can be heightened in an early stage.

The bioceramic particles to be incorporated into this porous composite 2 may be the same as the bioceramic particles contained in the compact composite 1 described above. However, bioceramic particles having a particle diameter of about 0.1-5 µm are especially preferred because use of such bioceramic particles is free from the possibility of cutting the fibers to be formed, e.g., by spraying in producing the porous composite 2 by the method which will be described later, and because such bioceramic particles have satisfactory bioabsorbability.

The content of the bioceramic particles in the porous composite 2 may be even throughout the whole porous-composite layer 21d as in the case of the porous composite 2 of the implant composite material 100 described above, or may be uneven. In the former case, in which the content is even, it is preferred that the content of the bioceramic particles should be 60-80% by mass. The reasons for this are as described hereinabove with regard to the porous composite 2 of the implant composite material 100, and an explanation thereon is hence omitted. A more preferred range of the content of the bioceramic particles is 60-70% by mass.

On the other hand, in the latter case, in which the content is uneven, it is preferred that the content of the bioceramic particles in the porous composite 2 should be higher than the bioceramic-particle content in the compact composite 1 and gradually change to have an inclination so that it increases from an inner-layer part to a surface-layer part of the layer of the porous composite 2 in the range of 30-80% by mass as in the case of the porous composite 2 of the implant composite material 100. Namely, it is preferred that the bioceramic particle/biodegradable and bioabsorbable polymer proportion by mass in the porous composite 2 should be larger than that mass proportion in the compact composite 1 and gradually change to have an inclination so that it increases from the inner-layer part to the surface-layer part of the layer of the porous composite 2 in the range of from 30/70 to 80/20. In the layer of the porous composite 2 having such an inclination of bioceramic-particle content, bioactivity is high in the surface-layer side having a high content and the inductive growth of an osteoblast and bone tissues is enhanced especially in the surface-layer side. This porous composite 2 bonds with a living bone and is replaced thereby in an early stage. Consequently, the strength of fixing of the implant composite material (anchor member) 109 to the upper or lower living bone of a knee joint or the like can be heightened in an early stage.

In contrast, the content of the bioceramic particles in the compact composite 1 preferably is lower than the bioceramic-particle content in the porous-composite layer 2 and is in the range of 30-60% by mass as in the case of the compact composite 1 of the implant composite material 100 described above. The reasons for this are as described above with regard to the compact composite 1 of the implant composite material 100.
The content of the bioceramic particles in the compact composite 1 may be even throughout the whole compact composite 1, provided that the content thereof is lower than in the porous composite 2 and in the range of 30-60% by mass as stated above.
Or, the content of the bioceramic particles in the compact composite 1 may gradually change to have an inclination so that it gradually increases from an axial core part toward a peripheral part of the compact composite 1, provided that the content thereof is lower than in the porous composite 2 and in the range of 30-60% by mass as stated above.
In the compact composite 1 having such an inclination of bioceramic-particle content, the peripheral part having a high content undergoes the conductive growth of bone tissues while the axial core part having a low content retains a strength. Finally, this compact composite 1 is wholly replaced.

Incidentally, in the case where the content of the bioceramic particles in each of the compact composite 1 and the porous composite 2 is to be inclined, it is preferred that the content thereof should be gradually changed continuously to have an inclination so that it increases from the axial core part of the compact composite 1 to the surface-layer part of the porous composite 2 in the range of 30-80% by mass.

The presence of the layer of the porous composite 2 having such properties on the surfaces of the compact composite 1 is useful also from the standpoint that it can be impregnated with bone growth factors and various drugs. Namely, it is desirable that this porous-composite layer 21d should be impregnated with at least one of the biological bone growth factors described above, i.e., a BMP, TGF-β, EP4, b-FGF, and PRP, and/or an osteoblast derived from a living organism. By impregnating the porous-composite layer with any of these biological bone growth factors and/or the osteoblast, osteoblast multiplication and growth are greatly accelerated. As a result, bone tissues come to grow in the surface-layer part of the porous-composite layer 21d in an extremely short period (about 1 week). The porous-composite layer 21d thus bonds with a living bone and is wholly replaced by the living bone rapidly thereafter. Of those factors, BMPs and EP4 are especially effective in hard-bone growth. It is therefore preferred that the porous composite 2 of the implant composite material (anchor member) 109 to be implanted and fixed into a hole formed in a hard bone such as, e.g., the thighbone or shinbone of a knee joint should be impregnated especially with any of BMPs and EP4s among those factors. On the other hand, PRP is a plasma having a highly elevated platelet concentration and addition thereof accelerates the growth of a newly regenerated bone. In some cases, another growth factor such as IL-1, TNF-α, TNF-β, or IFN-γ or a drug may be infiltrated.

The surface of the layer of the porous composite 2 may be subjected to an oxidation treatment such as corona discharge, plasma treatment, or hydrogen peroxide treatment. Such an oxidation treatment has an advantage that bonding with a living bone and replacement thereby are further accelerated because the wettability of the surface of the layer of the porous composite 2 is improved to enable an osteoblast to more effectively penetrate into and grow in interconnected pores of this composite 2. The fixing strength of this implant composite material (anchor member) 109 improves in an earlier stage. The surface of the compact composite 1 may, of course, be subjected to such an oxidation treatment.

The layer of the porous composite 2 may be produced by substantially the same method as for the porous composite 2 of the implant composite material 100. First, a biodegradable and bioabsorbable polymer is dissolved in a volatile solvent and bioceramic particles are mixed with the solution to prepare a suspension. This suspension is formed into fibers by spraying or another technique to produce a fibrous mass composed of fibers intertwined with one another. This fibrous mass is immersed in a volatile solvent such as methanol, ethanol, isopropanol, dichloroethane(methane), or chloroform to bring it into a swollen or semi-fused state. The fibrous mass in this state is pressed to obtain a porous fusion-bonded fibrous mass in an elliptic hollow cylinder form. The fibers in this fusion-bonded fibrous mass are shrunk and fused, and are thereby deprived substantially of their fibrous shape to form a matrix. Thus, the fibrous mass is changed into a porous-composite layer in an elliptic hollow cylinder form in which the spaces among the fibers have been changed into rounded interconnected pores. In this case, use may be made of a method in which two porous-composite layers in the form of a half of an elliptic hollow cylinder are formed and then united with each other.

In the case where a porous-composite layer in which the porosity increases from an inner-layer part to a surface-layer part is to be produced by that process, use may be made of a method in which when the fibrous mass is immersed in the volatile solvent to bring it into a swollen or semi-fused state and is then pressed to obtain a porous fusion-bonded fibrous mass in the form of an elliptic hollow cylinder or of a half of an elliptic hollow cylinder, the amount of the fibrous mass is regulated so as to decrease from the inner-layer part to the surface-layer part. On the other hand, in the case where a porous composite in which the content of bioceramic particles increases from an inner-layer part to a surface-layer part is to be produced, use may be made of a method which comprises preparing several suspensions differing in the amount of bioceramic particles incorporated, forming several fibrous masses differing in bioceramic-particle content, superposing these fibrous masses in order of increasing bioceramic-particle content, bringing this assemblage into a swollen or semi-fused stage, and pressing it.

The implant composite material (anchor member) 109 shown in Fig. 17 to Fig. 19 is one obtained in the following manner. The compact composite 1 in an elliptic solid cylinder form is fitted into a layer of the porous composite 2 in an elliptic hollow cylinder form. Alternatively, two layers of the porous composite 2 in the form of a half of an elliptic hollow cylinder are united with each other and superposed on the periphery of the compact composite 1 in an elliptic solid cylinder form. These members superposed are united by, e.g., thermal fusion bonding to obtain the target implant composite material. Techniques for uniting the compact composite 1 with the layer(s) of the porous composite 2 are not limited to thermal fusion bonding. For example, the two members may be united by bonding with an adhesive, or a method may be used which comprises forming a dovetail groove in one of the contact surfaces of the compact composite 1 and the layer(s) of the porous composite 2, forming a dovetail on the other contact surface, and fitting the dovetail into the dovetail groove to unite the two composites.

The artificial ligament 38 shown in Fig. 20 comprises an artificial ligamental member 37 formed from organic fibers as a raw material and the implant composite material 109 attached as an anchor member to each end of the ligamental member 37 so as not to detach therefrom.

More specifically, the artificial ligamental member 37 may comprise a structure which is either a three-dimensional woven structure or knit structure comprising organic fibers arranged along three or more axes or a structure comprising a combination of the woven structure and the knit structure. Alternatively, it may comprise a braid or the like comprising organic fibers. This artificial ligamental member 37 has a tensile strength and flexibility which are equal to or higher than those of living-body ligaments, and shows a deformation behavior similar to that of living-body ligaments. The structure constituting the artificial ligamental member 37 is the same as the structure described in Japanese Patent Application No. 6-254515 (Japanese Patent No. 3243679) filed by the present applicant. When the geometry of the structure employed is expressed in terms of the number of dimensions and the number of directions of fiber arrangement is expressed in terms of the number of axes, then the structure is a three-dimensional structure with three or more axes as stated above.

A three-axis three-dimensional structure is a structure made up of three-dimensionally arranged fibers extending in three axial directions, i.e., length, breadth, and vertical directions. A typical shape of this structure is a thick strip shape such as that shown in Fig. 20. However, a hollow cylindrical shape is also possible. This kind of three-axis three-dimensional structures are classified, according to structure differences, into orthogonal structure, non-orthogonal structure, leno structure, cylindrical structure, etc. A three-dimensional structure with four or more axes has an advantage that the strength isotropy of the structure can be improved by arranging fibers in directions along 4, 5, 6, 7, 9, or 11 axes, etc. By selecting these, an artificial ligamental member 37 akin to ligaments of the living body can be produced.

The artificial ligamental member 37 comprising the structure described above preferably has an internal porosity in the range of 20-90%. In case where the internal porosity thereof is lower than 20%, this ligamental member 37 is too compact and is impaired in flexibility and deformability. This ligamental member is hence unsatisfactory as a substitute for a bio-derived ligament. On the other hand, in case where the internal porosity thereof exceeds 90%, this ligamental member 37 is reduced in shape retention and shows too high elongation. This ligamental member also is hence unsatisfactory as a substitute for a bio-derived ligament.

The organic fibers to be used as a material for the artificial ligamental member 37 preferably are, for example, bioinert synthetic-resin fibers such as, e.g., fibers of polyethylene, polypropylene, and polytetrafluoroethylene, or coated fibers obtained by coating organic core fibers with any of these bioinert resins to impart bioinertness thereto. In particular, coated fibers having a diameter of about 0.2-0.5 mm obtained by coating core fibers of ultrahigh-molecular polyethylene with linear low-density polyethylene are optimal fibers from the standpoints of strength, hardness, elasticity, suitability for weaving/knitting, etc.

The structure of organic fibers which constitute the artificial ligamental member 37 is disclosed in detail in Japanese Patent Application No. 6-254515 (Japanese Patent No. 3243679), which was cited above. A further explanation thereon is hence omitted.

A braid or three-dimensional woven fabric formed from bioabsorbable poly(lactic acid) fibers is also usable as the artificial ligamental member 1 besides the organic-fiber structure or braid described above.

The implant composite material 109 has been attached as an anchor member to each end of the ligamental member 37 so as not to detach therefrom, by passing organic fibers of the ligamental member 37 through the many small holes 1c formed in the projecting piece 1b and hitching them on the projecting piece 1b.

Fig. 21 is a view illustrating an example in which the artificial ligament 38 described above is used.

This use example illustrates the case where the artificial ligament 38 is implanted in the knee joint between a thighbone 39 and a shinbone 40 to conduct reconstruction in the following manner. First, holes 33 and 33 are formed respectively in the thighbone 39 and shinbone 40. The artificial ligament 38 is inserted into the knee joint through these holes, and the implant composite materials (anchor members) 109 and 109 on both ends of the artificial ligamental member 37 are inserted respectively into the two holes 33 and 33. An interference screw 35 is screwed into the space between the inner surface of the hole 33 in the thighbone 39 and one implant composite material 109 to press this implant composite material 109 against that side of the inner surface of the hole 33 which is opposite to the screw 35 to thereby fix the implant composite material 109. Furthermore, an interference screw 35 is screwed into the space between the inner surface of the hole 33 in the shinbone 40 and the other implant composite material 109 while keeping the artificial ligamental member 37 moderately loose to press this implant composite material 109 against that side of the inner surface of the hole 33 which is opposite to the screw 35 and thereby fix this implant composite material 109. Thus, the artificial ligamental 38 is implanted in and fixed to the knee joint.

After the artificial ligament 38 is implanted in such manner, the layer of the porous composite 2 in each implant composite material (anchor member) 109 is rapidly hydrolyzed from the surface and inner parts thereof by a body fluid in contact with the surface thereof and by a body fluid which has penetrated into interconnected pores thereof. With this hydrolysis, bone tissues present in the hole 33 inductively grow in inner parts of the layer of the porous composite 2 due to the bone inductivity of the bioactive bioceramic particles and the layer of the porous composite 2 is replaced by the bone tissues in an early stage. The implant composite materials 109 thus bond with the bone tissues present in the holes 33 and 33 formed respectively in the thighbone 39 and shinbone 40. Consequently, the implant composite materials 109 and 109 respectively on both ends of the artificial ligament 38 have a greatly improved fixing strength as compared with the conventional case where both ends of a ligament are fixed with interference screws only. On the other hand, the compact composite 1 of each implant composite material 109 is hard and strong and hydrolyzes far more slowly than the layer of the porous composite 2. It retains a sufficient strength until the hydrolysis proceeds to a certain degree. Finally, the compact composite 1 is wholly hydrolyzed and disappears through replacement by a living bone conductively formed by the action of the bioactive bioceramic particles. Consequently, the holes 33 and 33 formed in the thighbone 39 and shinbone 40 of the knee joint are almost completely filled with the living bones. In addition, since the bioceramic particles contained in the layer of the porous composite 2 and compact composite 1 in each implant composite material 109 are bioabsorbable, the bioceramic particles neither remain/accumulate in the living bone which has replaced and regenerated nor come into soft tissues or blood vessels.

Furthermore, the artificial ligamental member 37 comprising a structure of bioinert organic fibers has a strength and flexibility which are equal to or higher than those of living-body ligaments and shows a deformation behavior similar to that of living-bone ligaments. Because of this, even when a tensile force is repeatedly applied to the artificial ligamental member 37 due to bends of the knee joint, this artificial ligamental member 37 has almost no fear of rupturing. It gives no uncomfortable feeling during bending and stretching.

Incidentally, when the interference screws 35 used are screws comprising the same biodegradable and bioabsorbable polymer containing bioceramic particles as that constituting the compact composite 1, there is an advantage that these screws also are hydrolyzed and replaced by the living bones, whereby the holes 33 and 33 are completely filled with the living bones.

Fig. 22 is a slant view of an implant composite material of the second type as still a further embodiment of the invention.

This implant composite material 110 is one which has a projecting piece 1b projecting from the end face on the ligamental member 37 side and in which the projecting piece 1b has many small projections 1d, in place of the small holes 1c, on the upper and lower sides and left and right sides thereof. Organic fibers of an artificial ligamental member 37 are hitched on the small projections 1d, whereby the implant composite material 110 can be attached as an anchor member to each of both ends of the artificial ligamental member 37 while preventing it from detaching. The small projections 1d each may have an even thickness throughout as shown in the figure. It is, however, preferred that the ends of the small projections should be expanded or bent so as to prevent the hitched organic fibers from detaching. The other constitutions of this implant composite material (anchor member) 110 are the same as those of the implant composite material (anchor member) 109 described above, which is shown in Fig. 17 to Fig. 19, and an explanation thereon is hence omitted.

This implant composite material 110 also can be attached to an artificial ligamental member 37 so as not to detach therefrom, by hitching organic fibers of the ligamental member 37 on the small projections 1d. This implant composite material 110 can be thus used for implantation/reconstruction. It is a matter of course that this implant composite material 110 produces the same effects and advantages as those of the implant composite material 109 in the artificial ligament 38 described above.

Fig. 23 is a vertical sectional view of an implant composite material of the second type as still a further embodiment of the invention.

This implant composite material (anchor member) 111 comprises: a compact composite 1 which has annular projections 1e having a serrate sectional shape and formed on the peripheral surface thereof; and the porous composite 2 superposed on and united with the peripheral surface of the compact composite 1 so that the porous composite 2 fills the recesses between the annular projections 1e. The other constitutions of this implant composite material 111 are the same as those of the implant composite material 109 in the artificial ligament 38 described above, and an explanation thereon is hence omitted.

This implant composite material 111 has the following advantage besides the effects and advantages of the implant composite material 109 in the artificial ligament 38 described above. When this implant composite material 111 is press-fixed into each of holes 33 and 33 formed respectively in the thighbone 39 and shinbone 40 of a knee joint with an interference screw 35 in the manner shown in Fig. 21, then the tips of the serrate annular projections 1e of the compact composite 1 of this implant composite material 111 slightly bite into the inner surface of each of the holes 33 and 33. Because of this, even when a tensile force caused by a bend of the knee joint is exerted on the artificial ligament and a force F in the direction indicated by the arrow in Fig. 23 is applied to the implant composite material 111 in which the porous composite 2 has not bonded with bone tissues present in each of the holes 33 and 33, then there is no fear that the implant composite material 111 may come out from each of the holes 33 and 33.

Fig. 24 is a cross-sectional view of an implant composite material of the second type as still a further embodiment of the invention.

This implant composite material (anchor member) 112 comprises: a compact composite 1 in the form of an elliptic solid cylinder which has ridges 1f each having a triangular sectional shape and formed on the peripheral surface thereof; and the porous composite 2 superposed on and united with the peripheral surface of the compact composite 1 so that the porous composite 2 fills the recesses between these ridges 1f. The other constitutions of this implant composite material 112 are the same as those of the implant composite material 109 in the artificial ligament 38 described above, and an explanation thereon is hence omitted.

A structure obtained by attaching this implant composite material 112 as an anchor member to each of both ends of an artificial ligamental member also has the following advantage. The tips of the ridges 1f slightly bite into the inner surface of each of holes formed in the upper and lower living bones of a knee joint. Consequently, this implant composite material 112 can have an improved fixing strength until the porous composite 2 of the implant composite material 112 bonds with bone tissues present in the hole of each living bone.

Fig. 25 is a cross-sectional view of an implant composite material of the second type as still a further embodiment of the invention.

This implant composite material (anchor member) 113 comprises: the compact composite 1 in the form of an elliptic solid cylinder described above; and a layer of the porous composite 2 superposed on and united with one side of the peripheral surface of the compact composite 1 in an elliptic solid cylinder form, i.e., that one side of the peripheral surface which is to be pressed against the inner surface of each of holes 33 respectively formed in the two living bones of a knee joint. The other constitutions of this implant composite material 113 are the same as those of the implant composite material 109 in the artificial ligament 38, and an explanation thereon is hence omitted.

When this implant composite material 113, in which a layer of the porous composite 2 has been superposed on and united with only one side of the peripheral surface the compact composite 1 as described above, is attached as an anchor member to each end of an artificial ligamental member 1, the following advantage is brought about. That layer of the porous composite 2 which is pressed against the inner surface of a hole 33 is rapidly hydrolyzed, is replaced by bone tissues, and bonds with the inner surface of the hole 33. Consequently, the implant composite material 113 can be improved in bonding strength in an early stage. It is, however, noted that the opposite side of the peripheral surface of the compact composite 1, which is not covered with a porous-composite layer, is slow in both hydrolysis and the conductive growth of bone tissues and, hence, considerable time is required for the hole 33 to be mostly filled with a living bone.

Fig. 26 is a cross-sectional view of an implant composite material of the second type as still a further embodiment of the invention.

This implant composite material (anchor member) 114 is a composite material having a three-layer structure comprising a core layer comprising a compact composite 1 and a layer of a porous composite 2 superposed on and united with each of the upper and lower sides of the core layer. This implant composite material 114 as a whole is in the form of an elliptic solid cylinder. It has a projecting piece (not shown) having the small holes or projections for hitching organic fibers of an artificial ligamental member 37, the projecting piece being formed on one edge face of the core layer comprising a compact composite 1.

When this implant composite material 114 is attached as an anchor member to each end of an artificial ligamental member 37, the following advantage is brought about. The layer of the porous composite 2 disposed on one side of the implant composite material 114 comes into close contact with the inner surface of the hole 33 formed in a living bone of a joint and is rapidly replaced by bone tissues while being hydrolyzed. The porous composite 2 on one side thus bonds rapidly with the inner surface of the hole and, hence, this implant composite material 114 can be improved in bonding strength in an early stage. On the other hand, the layer of the porous composite 2 on the other side also is hydrolyzed in an early stage and an osteoblast is induced in interconnected pores by the action of the bioceramic particles to grow bone tissues. Consequently, the hole 33 can be mostly filled with a living bone in a shorter time period than in the case of the implant composite material 113 shown in Fig. 25.

Next, implant composite material modifications are explained which eliminate the same problems as the implant composite materials of the second type described above, which are used as anchor members for artificial ligaments, etc., and can have the same effects and advantages as those implant composite materials.

The modifications of the implant composite materials of the second type include: (1) an implant composite material which is to be attached as an anchor member to an end part of a ligamental member or tendinous member so as not to detach therefrom, and is characterized in that it comprises a porous composite of a biodegradable and bioabsorbable polymer containing bioabsorbable and bioactive bioceramic particles and that the porosity thereof gradually changes to have an inclination so that it increases from an axial core part of the composite to a peripheral part thereof to be in contact with a bone in the range of 0-90%; and (2) an implant composite material which is to be attached as an anchor member to an end part of a ligamental member or tendinous member so as not to detach therefrom, and is characterized in that it comprises a porous composite of a biodegradable and bioabsorbable polymer containing bioabsorbable and bioactive bioceramic particles and that the porosity thereof gradually changes to have an inclination so that it increases from that end part of the composite to which a ligamental member or tendinous member is to be attached to an end part on the opposite side.

The implant composite materials as such modifications each have the following advantages when used for the reconstruction/fixing of a ligament, for example, in the following manner. The implant composite material is attached as an anchor member to each end of a ligamental member so as not to detach therefrom. These implant composite materials are inserted into holes respectively formed in the upper and lower living bones (thighbone and shinbone) of a knee joint. An interference screw is then screwed into the space between each implant composite material and the inner surface of the hole. As a result, the high-porosity peripheral part or end part of the porous composite constituting each implant composite material is rapidly hydrolyzed from the surface and inner parts thereof by a body fluid in contact with the surface of the composite and by a body fluid which has penetrated into interconnected pores of the peripheral part or end part. With this hydrolysis, bone tissues are inductively grown from the high-porosity peripheral part or end part to inner parts due to the bone inductivity of the bioactive bioceramic particles. This peripheral part or end part is thus replaced by a living bone in an early stage and the implant composite materials bond with the inner surfaces of the holes formed in the upper and lower living bones of the knee joint. As described above, the artificial ligament obtained by attaching the implant composite material as an anchor member to each end of a ligamental member has an advantage that the implant composite materials bond with living bones (inner surfaces of holes) in an early stage and, hence, both ends of the ligamental member come to have a greatly improved fixing strength as compared with the conventional physical fixing with interference screws only.

Furthermore, in those implant composite materials, the low-porosity axial core part of the porous composite and that end part of the porous composite to which a ligamental member is attached are strong, hydrolyze far more slowly than the high-porosity peripheral part and end part on the opposite side, and hence retain a sufficient strength until the hydrolysis proceeds to a certain degree. Finally, however, the low-porosity parts are wholly hydrolyzed and disappear while being replaced by a living bone conductively formed by the action of the bioactive bioceramic particles. As a result, the holes formed in the living bones are filled with the living bones. In addition, since the bioceramic particles contained in the compact composite and porous composite are bioabsorbable, they neither remain/accumulate in the living bones which have replaced and regenerated nor come into soft tissues or blood vessels.

In the implant composite material as the former modification (1), it is preferred that the content of the bioceramic particles should gradually change to have an inclination so that it increases from the axial core part of the porous composite to the peripheral part to be in contact with a bone in the range of 30-80% by mass. In the implant composite material as the latter modification (2), it is preferred that the content of the bioceramic particles should gradually change to have an inclination so that it increases from that end part of the porous composite to which a ligamental member or tendinous member is to be attached to the end part on the opposite side in the range of 30-80% by mass.

Those implant composite material modifications in which the content of the bioceramic particles inclines have the following advantage. Since the peripheral part or opposite-side end part has a high bioceramic-particle content and hence has higher bioactivity, the inductive growth of an osteoblast and bone tissues in the peripheral part or opposite-side end part is especially enhanced. Consequently, replacement by and bonding with a living bone are further accelerated.

Furthermore, in those implant composite material modifications also, it is desirable that the porous composite should have been impregnated with at least one of the biological bone growth factors described above and/or an osteoblast derived from a living organism. Such implant composite materials have an advantage that osteoblast multiplication/growth is greatly accelerated and, hence, bone tissues grow vigorously to enable bonding with and replacement by a living bone to proceed more rapidly. It is also preferred that many small holes or small projections for the attachment of a ligamental member or tendinous member be formed in or on an end part. This implant composite material has an advantage that a ligamental member or tendinous member can be attached thereto while preventing detachment without fail by passing organic fibers of the ligamental or tendinous member through the small holes and then hitching them or by hitching the organic fibers on the small projections.

Examples of such modifications of the implant composite material of the second type will be explained below by reference to drawings.

Fig. 27 is a vertical sectional view illustrating one example of modifications of implant composite materials of the second type, and Fig. 28 is a cross-sectional view of this implant composite material.

This anchor member 115 is a member in the form of an elliptic solid cylinder which comprises a porous composite 2 of a biodegradable and bioabsorbable polymer containing bioabsorbable and bioactive bioceramic particles. It is to be attached to each end of the artificial ligamental member 37 described above so as not to detach therefrom. The biodegradable and bioabsorbable polymer and bioceramic particles to be used as materials for the porous composite 2 may be the same as those for the porous composite 2 of the implant composite material 109 or the like described above.

The porous composite 2 constituting the anchor member 115 is one in which the porosity thereof gradually changes to have an inclination so that it increase from an axial core part 2h of the composite 2 to a peripheral part 2i thereof in the range of 0-90%, preferably in the range of 15-80%. In this porous composite 2, it is preferred that interconnected pores account for 50% or more, especially 70-90%, of all pores and that the pore diameter of the interconnected pores should have been regulated so as to be in the range of 50-600 µm, preferably in the range of 100-400 µm, and increase toward the high-porosity peripheral part 2i.

Such changes in porosity and pore diameter have the following advantages. That peripheral part 2i of the porous composite 2 which has a large pore diameter and a high porosity (hereinafter referred to as high-porosity peripheral part) is rapidly hydrolyzed because a body fluid easily penetrates thereinto. In addition, an osteoblast is apt to penetrate thereinto and this, coupled with the high content of the bioactive bioceramic particles as will be described later, enables bone tissues to inductively grow in an early stage. The peripheral part 2i thus bonds with a living bone and is replaced thereby in an early stage. Consequently, when the implant composite material 115 comprising this porous composite 2 is inserted into the hole 33 formed in each of the upper and lower living bones of a knee joint and is fixed with an interference screw, then the high-porosity peripheral part 2i of this implant composite material 115 bonds with the living bone in the inner surface of the hole 33 in an early stage. As a result, this implant composite material 115 comes to have a higher fixing strength than in the case of fixing with an interference screw only. Porosities exceeding 90% and pore diameters larger than 600 µm are undesirable for the high-porosity peripheral part 2i because this high-porosity peripheral part 2i has a reduced physical strength and is brittle. In case where the interconnected pores account for less than 50% of all pores and have a pore diameter smaller than 50 µm, this peripheral part 2i is undesirable because the penetration of a body fluid and an osteoblast thereinto is difficult and hydrolysis and the inductive growth of bone tissues are slow, resulting in a prolonged time period required for replacement by and bonding with a living bone.

On the other hand, that axial core part 2h of the porous composite 2 which has a low porosity (hereinafter referred to as low-porosity axial core part) is strong and the strength of this low-porosity axial core part 2h improves as the porosity decreases. Consequently, the low-porosity axial core part 2h need not always have a porosity of 0% when the anchor member for artificial ligaments is not required to have a high strength, although the porosity thereof should be 0% as shown above when a high strength is required. Because of this, it is desirable that the lower limit of the porosity of the low-porosity axial core part 2h should be preferably 15% to thereby impart a strength suitable for anchor members for ligaments and reduce the time period required for hydrolysis and complete replacement by a living bone. A projecting piece 2j has been formed so as to be united with and project from one edge face of this low-porosity axial core part 2h having a strength. The projecting piece 2j have small holes 2k. When organic fibers of an artificial ligamental member 37 are passed through these small holes 2k and hitched on, then this implant composite material 115 can be attached to the end part of the ligamental member 37 so as not to detach therefrom. It is a matter of course that the organic fibers in an end part of an artificial ligamental member 37 may be embedded in and fixed to the strong low-porosity axial core part 2h of the implant composite material 115 in such a manner than the organic fibers do not come out.

The content of the bioceramic particles in the porous composite 2 constituting the implant composite material 115 may be even throughout the whole composite 2. It is, however, preferred that the content thereof should gradually change to have an inclination so that it increases from the low-porosity axial core part 2h to the high-porosity peripheral part 2i in the range of 30-80% by mass. Namely, it is preferred that the bioceramic particle/biodegradable and bioabsorbable polymer proportion by mass should gradually change continuously so that it increases from the low-porosity axial core part 2h to the high-porosity peripheral part 2i in the range of from 30/70 to 80/20. Such an inclination of bioceramic-particle content has an advantage that the high-porosity peripheral part 2i has high bioactivity and the inductive growth of an osteoblast and bone tissues therein is especially enhanced, whereby replacement by and bonding with a living bone are further accelerated. In case where the content of the bioceramic particles in the high-porosity peripheral part 2i exceeds 80% by mass, this arouses a trouble that the high-porosity peripheral part 2i has a reduced physical strength. In case where the content thereof in the low-porosity axial core part 2h is lower than 30% by mass, this arouses a trouble that the inductive growth of bone tissues in the low-porosity axial core part 2h by the action of the bioceramic particles becomes slow and, hence, complete replacement by a living bone takes too much time. A more preferred upper limit of the content of the bioceramic particles is 70% by mass.

It is preferred that the porous composite 2 constituting the implant composite material 115 should be impregnated with any of the biological bone growth factors described above and/or an osteoblast derived from a living organism. By this impregnation, osteoblast multiplication and growth are greatly accelerated. As a result, bone tissues come to grow in the high-porosity peripheral part 2i of the porous composite 2 in an extremely short time period (about 1 week) to accelerate bonding with the inner surface of the hole 33. Furthermore, the time period required for the hole 33 to be mostly filled with a living bone through the complete replacement of the porous composite 2 can be reduced. The surface of this porous composite 2 may be subjected to an oxidation treatment such as corona discharge, plasma treatment, or hydrogen peroxide treatment to thereby improve the wettability of the surface of the porous composite 2 and further accelerate the penetration and growth of an osteoblast.

The implant composite material 115 comprising the porous composite 2 may be produced, for example, by the following process. First, a biodegradable and bioabsorbable polymer is dissolved in a volatile solvent and bioceramic particles are mixed therewith to prepare a suspension. This suspension is formed into fibers by spraying or another technique to produce a fibrous mass composed of fibers intertwined with one another. This fibrous mass is packed into an elliptic hollow cylinder so that the fiber amount decreases from the center to the periphery. The fibrous mass packed is further immersed in a volatile solvent to bring it into a swollen or semi-fused state. The fibrous mass in this state is pressed in the axial direction for the elliptic cylinder to obtain a porous fusion-bonded fibrous mass in the form of an elliptic solid cylinder. The fibers in this fusion-bonded fibrous mass are shrunk and fused, and are thereby deprived substantially of their fibrous shape to form a matrix. Thus, the fibrous mass is changed in form into a porous composite in which the spaces among the fibers have been changed into rounded interconnected pores. One end of this porous composite in the form of an elliptic solid cylinder is cut to form a projecting piece and small holes. Thus, the implant composite material 115 is produced. In the case where the porous composite 2 in which the content of bioceramic particles increases from the low-porosity axial core part 2h to the high-porosity peripheral part 2i is to be produced, the following method may be used. Several suspensions differing in the amount of bioceramic particles incorporated are prepared, and several fibrous masses differing in bioceramic-particle content are formed therefrom. These fibrous masses are packed into an elliptic hollow cylinder in such a manner that the fibrous mass having a lowest content is disposed in the center and the other fibrous masses are disposed toward the periphery in order of increasing content. The fibrous masses thus packed are brought into a swollen or semi-fused state with a volatile solvent and then pressed.

Fig. 29 is a vertical sectional view illustrating another example of the modifications of implant composite materials of the second type.

This implant composite material 116 also is a member in the form of an elliptic solid cylinder which comprise a porous composite of a biodegradable and bioabsorbable polymer containing bioceramic particles. However, in this porous composite 2, the porosity thereof gradually changes to have an inclination so that it increases from one end part of the composite 2, i.e., an end part 2m where a ligamental member 37 is to be attached, to an end part 2n on the opposite side in the range of 0-90%, preferably in the range of 15-80%. Furthermore, the content of the bioceramic particles also gradually changes to have an inclination so that it increases from the end part 2m to the end part 2n on the opposite side in the range of 30-80% by mass. A projecting piece 2j projects from the end part the porosity of which is low or 0% and which is strong, and many small holes 2k have been formed in this projecting piece 2j. Organic fibers of an artificial ligamental member 37 are inserted into these small holes 2k, whereby the implant composite material 116 is attached as an anchor member to the end part of the ligamental member 37 so as not to detach therefrom. The other constitutions of this implant composite material 116 are the same as those of the anchor member 115, and an explanation thereon is hence omitted.

This implant composite material 116 may be used in the following manner. The implant composite material 116 is attached as an anchor member to each end of a ligamental member 1. These composite materials 116 are inserted into the holes 33 respectively formed in the upper and lower living bones of a knee joint and are then fixed with interference screws. As a result, the upper side and peripheral surface of the opposite-side end part 2n, which has a high porosity, and the peripheral surface of the part which is located underneath the high-porosity opposite-side end part 2n and has a relatively high porosity are rapidly hydrolyzed and bond with the inner surface of each hole 33 while being replaced by a living bone in an early stage. Consequently, the fixing strength of each implant composite material 116 improves in an early stage. On the other hand, the end part 2m, the porosity of which is low or 0%, is slow in hydrolysis and retains its strength over a certain time period. However, this end part 2m is wholly replaced by a living bone and disappears shortly and each hole 33 is mostly filled with the living bone which has replaced.

Although the implant composite materials (anchor members) 115 and 116 described above each are in the form of an elliptic solid cylinder, the shapes thereof are not limited to elliptic solid cylinders. The implant composite materials may have any shape as long as they can be easily inserted into holes 33 respectively formed in the upper and lower living bones of a knee joint and can be stably fixed with interference screws.

The implant composite materials (anchor members) 109, 110, 111, 112, 113, 114, 115, and 116 described above each can be used after having been attached to an end part of a bio-derived ligamental member or tendinous member, an artificial tendinous member, or the like so as not to detach therefrom.

Next, the implant composite material of the third type, which is for use as an interference screw for tendon or ligament fixing, will be explained by reference to drawings.

Fig. 30 illustrates an implant composite material of the third type as still a further embodiment of the invention: (a), (b), and (c) are a front view, vertical sectional view, and plane view thereof, respectively.

This implant composite material 117 for tendon or ligament fixing comprises: an interference screw 10 (hereinafter referred to simply as screw) which comprises a compact composite of a biodegradable and bioabsorbable polymer containing bioabsorbable and bioactive bioceramic particles and has a through-hole 10c for Kirschner wire insertion formed along a center line CL therefor; and a packing 20 which comprises a porous composite of a biodegradable and bioabsorbable polymer containing bioabsorbable and bioactive bioceramic particles and with which the through-hole 10c is filled, the packing 20 having been impregnated with any of the biological bone growth factors described above.

The screw 10 of this implant composite material 117 has a screw head 10a in a nearly roughly hemispherical form. The diameter of the screw head 10a is almost the same as the outer diameter of the screw thread 10b. The top of the screw thread 10b has been cut so as to have a flat surface. Inparticular, the top of the screw thread 10b in an area near to the screw tip has been considerably cut so as to result in a reduced outer diameter. This screw shape enables the screw to be smoothly screwed deeply into a hole formed in a bone of a joint while the flat surface of the screw thread top is being pressed against both of the inner surface of the hole and that end-part transplant bone flap of a transplant tendon or the like which is inserted into the hole.

This screw 10 has a through-hole 10c for Kirschner wire insertion formed along the center line CL therefor. This through-hole 10c is made up of a complete-circle hole part 10e for inserting only a Kirschner wire therethrough and a large elongated-circle hole part 10d which is located on the hole part 10e and into which the tip of a rotating tool also can be fitted. The hole part 10d for fitting the tip of a rotating tool thereinto is not limited to an elongated-circle hole part, and may be a hole part of any shape, such as, e.g., an elliptic, quadrilateral, or hexagonal shape, in which the tip of a rotating tool does not idle and the rotating force can be transmitted to the screw 10.

Small holes (not shown) connected to the through-hole 10c (10d and 10e) may be formed in this screw 10 as long as the screw 10 can retain a strength required of screws for tendon or ligament fixing. Formation of such small holes has the following advantages. A body fluid and an osteoblast are apt to penetrate through the small holes into the packing 20 packed in the through-hole, and the biological bone growth factor contained is apt to be released with the degradation and assimilation of the packing 20. Consequently, the growth of a living bone and bone adhesion can be further accelerated.

The screw 10 of this implant composite material 117 comprises a compact composite of a biodegradable and bioabsorbable polymer containing bioabsorbable and bioactive bioceramic particles as described above, and is required to have a high strength which is equal to or higher than the upper and lower living bones (hard bones) of a joint. Because of this, a biodegradable and bioabsorbable polymer suitable for use as a raw material therefor is a crystalline polymer such as poly(L-lactic acid) or poly(glycolic acid). Especially preferred is poly(L-lactic acid) having a viscosity-average molecular weight of 150,000 or higher, desirably about 200,000-600,000.

As the bioceramic particles to be incorporated into the compact composite constituting the screw 10, it is preferred to use the same bioceramic particles as those contained in the compact composite 1 of the implant composite material 100 described above.

In the compact composite constituting the screw 10, the content of the bioceramic particles is preferably in the range of 30-60% by mass. In case where the content thereof exceeds 60% by mass, there is a possibility that the compact composite becomes brittle, leading to a strength deficiency in the screw 10. Contents thereof lower than 30% by mass result in a trouble that the conductive bone formation by the action of the bioceramic particles becomes insufficient and the complete replacement of this screw 10 by bone tissues requires much time. The content of the bioceramic particles may be even throughout the whole compact composite constituting the screw 10 in the range of 30-60% by mass, or may change to have an inclination so that it gradually increases from a central part toward the periphery of the compact composite in the range of 30-60% by mass. The screw 10 comprising the compact composite having such an inclination of bioceramic-particle content as in the latter case has an advantage that bone tissues conductively grow in an early stage in the peripheral parts having a high bioceramic-particle content (peripheral part of the screw head 10a and peripheral part of the screw shaft) and these peripheral parts bond in an early stage with the inner surface of each of holes respectively formed in the upper and lower living bones of a joint and with the transplant bone flap on each end of a transplant tendon inserted in the holes. The screw 10 hence does not suffer loosening, etc.

The screw 10 of the implant composite material 117 may be produced by molding a biodegradable and bioabsorbable polymer containing bioceramic particles to produce a compact-composite molded object in the form of a solid cylinder and cutting this molded object into a screw shape such as that shown in Fig. 30. In this case, when compression molding or forging is used to produce a compact-composite molded object and this molded obj ect is cut, the following advantage is brought about. This compact composite is highly compact due to the compression and polymer molecules and crystals therein have been three-dimensionally oriented. Consequently, a screw 10 having a higher strength can be obtained. Furthermore, a screw 10 may be produced by conducting stretch forming to obtain a molded object in which polymer molecules have been uniaxially oriented and cutting this molded object.

On the other hand, the packing 20 of the implant composite material 117 has been formed so as to have a columnar shape corresponding and conforming to the through-hole 10c of the screw 10. Namely, this packing 20 comprises a columnar part 20b having a complete-circle section and a length which correspond and conform to those of the complete-circle hole part 10e for inserting only a Kirschner wire therethrough and a columnar part 20a having an elongated-circle section and a length which correspond and conform to those of the large elongated-circle hole part 10d which is located on the hole part 10e and into which a rotating too also can be fitted, the columnar parts 20b and 20a being vertically united.

This packing 20, which comprises a porous composite of a biodegradable and bioabsorbable polymer containing bioabsorbable and bioactive bioceramic particles as stated above, has interconnected pores inside. Part of the bioceramic particles are exposed in inner surfaces of the interconnected pores and in the surfaces of the porous composite.

The porous composite constituting this packing 20 need not have a high strength such as that of the screw 10 comprising the compact composite, and a strength and flexibility which prevent the packing 20 from breaking upon insertion into the through-hole 10c of the screw 10 suffice for the porous composite. This porous composite is required to be rapidly degraded and be wholly replaced by a living bone in an early stage. Because of this, the biodegradable and bioabsorbable polymer to be used as a raw material for the porous composite preferably is the same as the biodegradable and bioabsorbable polymer in the porous composite 2 of the implant composite material 100 described above.

It is desirable that the porous composite constituting the packing 20 should be one in which the porosity thereof is 60-90%, preferably 65-85%, interconnected pores account for 50% or more, preferably 70-90%, of all pores, and the interconnected pores have a pore diameter of 50-600 µm, preferably 100-400 µm, when a necessary physical strength, suitability for impregnation with a biological bone growth factor, osteoblast penetration and stabilization, etc. are taken into account. In case where the porous composite has a porosity exceeding 90% and a pore diameter larger than 600 µm, this porous composite has a reduced physical strength to make the packing 20 brittle. On the other hand, when the porosity thereof is lower than 60%, the proportion of pores is lower than 50% based on all pores, and the pore diameter is smaller than 50 µm, then it is difficult to rapidly inject and infiltrate an appropriate amount of a biological bone growth factor and the penetration of a body fluid or osteoblast also becomes difficult. In this case, the hydrolysis of the porous composite and the inductive growth of bone tissues therein become slow and, hence, the time period required for the through-hole 1c of the screw 10 to be filled through complete replacement by a living bone is prolonged. However, it has been found that bone inductivity is exhibited when fine interconnected pores on submicron order of 1-0.1 µm coexist with interconnected pores having that preferred pore diameter.

The porosity of the porous composite constituting the packing 20 may be even throughout the whole composite in the range of 60-90%, or may continuously increase toward the peripheral surface and toward the upper and lower ends in the range of 60-90%. The pore diameter of the interconnected pores may be even throughout the whole composite in the range of 50-600 µm, or may gradually increase toward the peripheral surface and toward the upper and lower ends in the range of 50-600 µm. The packing 20 comprising the porous composite having such inclinations of porosity and pore diameter has the following advantages. An appropriate amount of a biological bone growth factor can be rapidly injected and infiltrated through the upper and lower ends and peripheral surface having a high porosity and a large pore diameter. Hydrolysis hence proceeds rapidly from the upper and lower ends and peripheral surface, and osteoblast penetration and the inductive growth of bone tissues are enhanced. Consequently, replacement by a living bone is further accelerated.

The bioceramic particles to be incorporated into the porous composite constituting the packing 20 may be the same as the bioceramic particles contained in the compact composite constituting the screw 10 described above. However, bioceramic particles having a particle diameter of about 0.1-5 µm are especially preferred because use of such bioceramic particles is free from the possibility of cutting the fibers to be formed, e.g., by spraying in producing the porous composite by the method which will be described later, and because such bioceramic particles have satisfactory bioabsorbability.

The content of the bioceramic particles in the porous composite constituting the packing 20 is preferably 60-80% by mass. The content thereof may be even throughout the whole porous composite in that range or may have an inclination so that it increases toward the peripheral surface and the upper and lower ends in that range. Contents thereof exceeding 80% by mass result in a trouble that such a high bioceramic-particle content coupled with the high porosity of the porous composite leads to a decrease in the physical strength of the packing 20 comprising the porous composite. Contents thereof lower than 60% by mass cause the following trouble. This porous composite has reduced bioactivity and, hence, the inductive growth of bone tissues becomes slow. As a result, the time period required for complete replacement by a living bone is prolonged. A more preferred range of the content of the bioceramic particles is 60-70% by mass. In the porous composite having such an inclination of content, bioactivity is high in the surface layer having a high content and the inductive growth of an osteoblast and bone tissues therein is especially enhanced. Consequently, replacement by a living bone is further accelerated.

The biological bone growth factor to be infiltrated into the packing 20 comprising the porous composite may be, for example, any of the BMP, TGF-β, EP4, b-FGF, and PRP described above. These factors may be infiltrated alone or as a mixture of two or more thereof. It is also preferred that any of those biological bone growth factors should be infiltrated into the packing 20 in combination with an osteoblast derived from a living organism. Any of those biological bone growth factors or a mixture thereof with an osteoblast derived from a living organism may be infiltrated into the core material 20 as an injection or dripping preparation in a solution or suspension state.

The infiltration of any of those biological bone growth factors and/or the osteoblast into the core material 20 has the following advantages. Prior to or simultaneously with the hydrolysis of the core material 20, the biological bone growth factor, etc. exude to considerably accelerate osteoblast multiplication/growth. Because of this, bone adhesion (e.g., adhesion between the transplant bone flap on one end of a transplant tendon and the inner surface of a hole formed in the upper or lower bone of a joint) is completed in about several weeks, and bone tissues come to grow in surface-layer parts of the porous composite constituting the packing 20. The porous composite is wholly replaced by a living bone rapidly thereafter and the through-hole 10c of the screw 10 is filled with the living bone. As stated above, BMPs and EP4, among the biological bone growth factors shown above, are especially effective in hard-bone growth. PRP is a plasma having a highly elevated platelet concentration, and addition thereof accelerates the growth of a newly regenerated bone. In some cases, another growth factor such as IL-1, TNF-α, TNF-β, or IFN-γ may be mixed.

The surface of the packing 20 comprising the porous composite may be subjected to an oxidation treatment such as corona discharge, plasma treatment, or hydrogen peroxide treatment. Such an oxidation treatment has the following advantage. The wettability of the surface of the packing 20 is improved and an osteoblast more effectively penetrates into interconnected pores through the minute gap between the packing 20 and the through-hole 10c and grows therein. Because of this, complete replacement by a living bone is further accelerated and the through-hole 10c of the screw 10 is filled with the living bone in an early stage. The surface of the screw 10 comprising the compact composite may, of course, be subjected to such an oxidation treatment.

The packing 20 comprising the porous composite can be produced, for example, by the following process. First, a biodegradable and bioabsorbable polymer is dissolved in a volatile solvent and bioceramic particles are mixed with the solution to prepare a suspension. This suspension is formed into fibers by spraying or another technique to produce a fibrous mass composed of fibers intertwined with one another. This fibrous mass is immersed in a volatile solvent such as methanol, ethanol, isopropanol, dichloroethane(methane), or chloroform to bring it into a swollen or semi-fused state. The fibrous mass in this state is pressed to obtain a porous fusion-bonded fibrous mass in a columnar form corresponding and conforming to the through-hole 10c of the screw 10. The fibers in this fusion-bonded fibrous mass are shrunk and fused, and are thereby deprived substantially of their fibrous shape to form a matrix. Thus, the fibrous mass is changed in form into a porous composite in which the spaces among the fibers have been changed into rounded interconnected pores to thereby produce the packing 20.

The implant composite material 117 for tendon or ligament fixing which is in the form of the interference screw described above may be used in the following manner. The interference screw is screwed into the space between the inner surface of each of holes respectively formed in the upper and lower bones of a joint and the transplant bone flap on each end of, e.g., a transplant tendon inserted into the holes to thereby press the transplant bone flap against the inner surface of the hole and fix it. In this application, the screw 10 itself; which comprises the compact composite comprising a biodegradable and bioabsorbable polymer containing bioceramic particles, has a sufficient mechanical strength, although it is a hollow object having a through-hole formed therein, and slowly undergoes hydrolysis by a body fluid. Because of this, the screw 10 retains its strength over a period of at least 3 months, which is necessary for the adhesion of the transplant bone flap on each end of the transplant tendon to the inner surface of the hole, and the transplant bone flap on each end of the transplant tendon can be pressed against and fixed to the inner surface of the hole without fail. On the other hand, the packing 20 which comprises the porous composite of a biodegradable and bioabsorbable polymer containing bioceramic particles and which has been inserted in the through-hole 10c of the screw 10 enables a body fluid and an osteoblast to penetrate into inner parts of the porous composite through interconnected pores, and is degraded and assimilated earlier than the screw comprising the compact composite while exhibiting bone conductivity or bone inductivity based on the bioactivity of the bioceramic particles. Prior to or simultaneously with this degradation/assimilation, the biological bone growth factor supported, such as a BMP, is gradually released. Because of this, bone adhesion is completed in about several weeks, which period is considerably shorter than three months necessary for ordinary bone adhesion, although that period differs depending on the part and the bone growth factor. Thus, the transplant bone flaps on both ends of the transplant tendon are fixed to the inner surfaces of the holes (i.e., to the living bones) in such an early stage and the inductive growth of a living bone in the packing 20 is efficiently accelerated. Thereafter, each screw 10 and the packing 20 further undergo degradation and assimilation and are finally replaced completely by a living bone formed by bone conduction or bone induction, whereby the joint is restored to the original state in which the through-hole 10c of the screw 10 does not remain vacant. Furthermore, since the biological bone growth factor contained in the packing 20 comprising the porous composite has not undergone the heat history attributable to screw production, it has no fear of having undergone thermal alteration. In addition, since the bioceramic particles contained in the screw 10 and in the packing 20 are bioabsorbable, they neither remain/accumulate in the living bones which have replaced nor come into/remain in soft tissues or blood vessels. Moreover, since the surface-layer part of each screw 10 bonds in an early stage with the transplant bone flap on an end of the transplant tendon and with the inner surface of the hole in an early stage due to bone tissues conductively grown with hydrolysis, the screw 10 can be prevented from becoming loose.

The implant composite material 117 for tendon or ligament fixing described above may be provided as a set of constituent members therefor.
One example of such sets for tendon or ligament fixing is characterized by comprising a combination of (1) an interference screw which comprises a compact composite of a biodegradable and bioabsorbable polymer containing bioabsorbable and bioactive bioceramic particles and has a through-hole for inserting a Kirschner wire thereinto, (2) a packing which comprises a porous composite of a biodegradable and bioabsorbable polymer containing bioabsorbable and bioactive bioceramic particles and which is to be packed into the through-hole, and (3) a biological bone growth factor to be infiltrated into the packing.
Another example is characterized by comprising a combination of (1) an interference screw which comprises a compact composite of a biodegradable and bioabsorbable polymer containing bioabsorbable and bioactive bioceramic particles and has a through-hole for inserting a Kirschner wire thereinto and (2) a packing which comprises a porous composite of a biodegradable and bioabsorbable polymer containing bioabsorbable and bioactive bioceramic particles and which contains a biological bone growth factor infiltrated therein and is to be packed into the through-hole.
Still another example is characterized by comprising a combination of (1) an interference screw which comprises a compact composite of a biodegradable and bioabsorbable polymer containing bioabsorbable and bioactive bioceramic particles and has a through-hole for inserting a Kirschner wire thereinto and (2) a packing which comprises a porous composite of a biodegradable and bioabsorbable polymer containing bioabsorbable and bioactive bioceramic particles and which is to be packed into the through-hole.

In each of those sets for tendon or ligament fixing, the content of the bioceramic particles in the compact composite constituting the interference screw is preferably 30-60% by mass, while the content of the bioceramic particles in the porous composite constituting the packing is preferably 60-80% by mass. In the porous composite constituting the packing, the porosity thereof is preferably 60-90%, interconnected pores account for preferably 50% or more of all pores, and the interconnected pores have a pore diameter of preferably 50-600 µm. It is also preferred to infiltrate at least one of the biological bone growth factors described above into the packing. The reasons for these are as explained above with regard to the implant composite material 117 for tendon or ligament fixing.

Fig. 31 illustrates one example of such sets for tendon or ligament fixing: (a) is a front view of an interference screw in the set; (b) is a front view of a packing in the set; and (c) is a front view of a container in the set, the container containing a biological bone growth factor. Fig. 32 is a view illustrating an example in which this set for tendon or ligament fixing is used.

This set for tendon or ligament fixing comprises a combination of a screw 10 having a through-hole 10c for Kirschner wire insertion formed along a center line CL therefor, a packing 20 to be packed into the through-hole 10c, and a biological bone growth factor enclosed in a container 41 such as an ampule. This combination may be packed into, e.g., a bag or case. This screw 10 is the same as the screw 10 in Fig. 30 described above, and the core material 20 also is the same as the core material 20 in Fig. 30 described above. Consequently, like parts are indicated by like numerals or signs in Fig. 31, and explanations thereon are omitted. Furthermore, the biological bone growth factor also is the same as the biological bone growth factor infiltrated into the core material 20 in Fig. 30 described above. It is enclosed in the container 41 as an injection or dripping preparation in a solution or dispersion state. An explanation thereon is hence omitted.

In the case where this set for tendon or ligament fixing is used for the transplantation/reconstruction of a tendon of, e.g., a knee joint, holes 33 and 33 are respectively formed in the thighbone 39 and the shinbone 40, and a transplant tendon 42 taken out so as to have transplant bone flaps 42a and 42a respectively on both ends is passed through the holes 33 and 33 as shown in Fig. 32. A Kirschner wire (not shown) and the tip of a rotating tool (not shown) are inserted into the through-hole 10c of a screw 10, and this screw 10 is screwed, while being rotated, in a proper direction into a proper position in the space between the inner surface of one hole 33 and the transplant bone flap 42a to thereby press this transplant bone flap 42a against the opposite-side inner surface of this hole 33 and fix it. Subsequently, the rotating tool and the Kirschner wire are drawn out and a packing 20 is packed into the through-hole 10c of the screw 10. Thereafter, a container 41 is opened and the biological bone growth factor is infiltrated into the packing 20. Alternatively, a container 41 is opened and the biological bone growth factor is infiltrated into the packing 20, before the packing 20 is packed. Likewise, a screw 10 is screwed into the space between the inner surface of the other hole 33 and the transplant bone flap 42a to press and fix this transplant bone flap 42a. Thereafter, a packing 20 is packed into the through-hole 10c and a biological bone growth factor is infiltrated into the packing 20. Thus, the operation of tendon transplantation/fixing is completed. Incidentally, the transplantation/fixing of a ligament is conducted by almost the same procedure as described above.

After the transplant tendon is thus fixed, each screw 10 retains a sufficient strength over a period of at least 3 months, which is usually necessary for the adhesion of the transplant bone flap 42a to the inner surface of the hole 33, to fix the osteosynthesis part without fail. On the other hand, the packing 20 releases the biological bone growth factor while being rapidly hydrolyzed. Consequently, the adhesion of the transplant bone flap 42a to the inner surface of the hole 33 is completed in about several weeks, although this period varies depending on the part and the bone growth factor. In addition, the packing 20 is replaced by a living bone due to the bioactivity of the bioceramic particles and the through-hole 10c is hence filled with the living bone in a relatively early stage. Finally, the screw 10 also is wholly replaced by a living bone and disappears, whereby the bone is restored to the original state in which the through-hole 10c does not remain vacant.

Fig. 33 illustrates another example of the sets for tendon or ligament fixing: (a) is a vertical front view of a screw in the set and (b) is a vertical sectional view of a packing in the set.

This set for tendon or ligament fixing differs from the set for tendon or ligament fixing shown in Fig. 31 in that the through-hole 10c of the screw 10 is a straight hole having an elongated-circle cross section throughout the whole screw length, that the packing 20 accordingly has a columnar shape having an elongated-circle sectional shape, and that a biological bone growth factor enclosed in a container or the like is not employed as a component of the combination and only the screw 10 and the packing 20 are employed in combination and packed in a bag, case, etc. This screw 10 having a straight hole with an elongated-circle cross section as the through-hole 10c has an advantage that cutting for forming the through-hole 10c is easy. However, there is a possibility that when a Kirschner wire is inserted and the screw 10 is screwed, the center of rotation might deflect slightly. For eliminating this possibility, it is preferred to form a tapered part 10f in a lower end part of the through-hole 10c as shown by the broken lines so that the lower end opening of the through-hole 10c is a complete circle having almost the same diameter as the Kirschner wire, and to form the packing 20 so as to have a shape corresponding and conforming to this through-hole 10c.

The compact composite of a biodegradable and bioabsorbable polymer which constitutes this screw 10, bioceramic particles contained therein, content thereof, and the like are the same as those in the screw in Fig. 30 described above, and explanations thereon are hence omitted. Furthermore, the porous composite of a biodegradable and bioabsorbable polymer which constitutes the packing 20, porosity thereof, proportion of interconnected pores, pore diameter of the interconnected pores, bioceramic particles contained therein, content thereof, and the like also are the same as those in the packing in Fig. 30 described above, and explanations thereon are hence omitted.

This set for tendon or ligament fixing may be used in the following manner as for the fixing set shown in Fig. 31 described above. The screw 10 is screwed into the space between the inner surface of each of holes respectively formed in the upper and lower bones of a joint and a transplant bone flap of a transplant tendon (or transplant ligament) inserted into the hole to fix the bone flap. The Kirschner wire is drawn out. Thereafter, the packing 20 is packed into the through-hole 10c of the screw 1 and a biological bone growth factor separately prepared is infiltrated into the packing 20. Alternatively, a biological bone growth factor is infiltrated into the packing 20 before this packing 20 is packed into the through-hole 10c of the screw 10. By thus fixing a transparent tendon (or transplant ligament), the same effects and advantages as in the case of the tendon- or ligament-fixing set in Fig. 31 described above are obtained.

Still another example of the sets for tendon or ligament fixing, i.e., a set for tendon or ligament fixing which comprises a combination of the interference screw and the packing impregnated with a biological bone growth factor, may be used in the same manner as for the fixing set in Fig. 31 described above although not shown in a drawing. The screw is screwed into the space between the inner surface of each of holes respectively formed in the upper and lower bones of a joint and a transplant bone flap of a transplant tendon (or transplant ligament) inserted into the hole to fix the bone flap. The Kirschner wire is drawn out. Thereafter, the packing impregnated with a biological bone growth factor is packed into the through-hole of the screw. By thus fixing a transparent tendon (or transplant ligament), the same effects and advantages as in the case of the tendon- or ligament-fixing set in Fig. 31 described above are obtained.

When the implant composite material 117 of the third type of the invention, which is for tendon or ligament fixing, or any of the sets for tendon or ligament fixing is used for the reconstruction/fixing of a tendon or ligament in the manner described above, the following remarkable advantages are obtained. The biological bone growth factor released from the packing 20 greatly reduces the time period required for bone adhesion between the inner surface of each of holes respectively formed in the upper and lower bones of a joint and that transplant bone flap of a transplant tendon or transplant ligament which has been inserted into this hole. Consequently, the patient, doctor, and hospital make a large profit. In addition, due to the bone conductivity or bone inductivity of the bioactive bioceramic particles contained in the screw 10 and packing 20, a living bone replaces and regenerates and the bone is restored so as not to have a residual hollow part.

If circumstances require, use may be made of a technique in which a packing is formed from a biodegradable and bioabsorbable polymer containing no bioceramic particles and a biological bone growth factor is infiltrated into this packing. It is not denied that such packing containing no bioceramic particles is slightly inferior to bioceramic-particle-containing packings in living-bone conductivity or inductivity after impregnation with a biological bone growth factor. However, the biological bone growth factor supported on the packing is released and, hence, the adhesion of the transplant bone flap on each end of a transplant tendon or transplant ligament is completed in about several weeks. Consequently, the time period required for the patient to leave his bed is significantly shortened, and all of the patient, doctor, and hospital make a large profit. Thus, one of the main objects of the invention can be sufficiently accomplished.

The implant composite material of the fourth type of the invention, which is for osteosynthesis, will be explained next by reference to drawings.

Fig. 34 illustrates an implant composite material for osteosynthesis as still a further embodiment of the invention: (a), (b), and (c) are a front view, vertical sectional view, and plan view thereof, respectively.

This implant composite material 118 for osteosynthesis comprises: a bone-uniting material main body which has been formed into a screw 11 and has a cylindrical deep hole 11b extending along the center line therefor from the upper end surface of the screw head 11a to a part near to the screw tip; and a filler 21 in a solid cylinder form which has a diameter and length conforming to the diameter and depth of the hole 11b and with which the hole 11b is filled, the filler 21 having been impregnated with any of the biological bone growth factors shown above.

The diameter of the hole 11b of the screw 11 is not particularly limited. However, it is preferred to regulate the diameter thereof so as to be about 1/3 to 2/3 the diameter of the shaft part (diameter as measured at valley parts between tops of the screw thread 11c) of the screw 11. In case where the diameter of this hole 11b is larger than 2/3 the diameter of the screw shaft part, this screw 11 has a reduced strength, resulting in the possibility that this screw 11 might break when screwed into a fractured part. In case where the diameter thereof is smaller than 1/3, the filler 21 to be packed is too thin and the proportion of this filler 21 is reduced, whereby the effects of inductively forming a living bone and accelerating bone adhesion become insufficient. Both cases are hence undesirable.

The head 11a of the screw 11 of this implant composite material 118 has a square plane shape in which the four corners have been rounded. This screw 11 can hence be screwed into a fractured part by applying a rotating tool having a tip in a hollow prism shape thereto so that the screw head 11a is fitted into the hollow-prism tip and rotating the tip. Consequently, this screw 11 is less apt to suffer breakage of the screw head 11a when screwed, as compared with ones in which the screw head has a plus groove for plus driver insertion, and hence can be tightly screwed until the lower surface of the screw head 11a is tightly pressed against the bone.

The shape of the screw head 1a is not limited to the square plane shape, and the screw can have a head of any of various shapes such as, e.g., a head having a hexagonal or octagonal plane shape, a head having a plus groove for plus driver insertion, a head having a minus groove for minus driver insertion, or a head having a square, hexagonal, or octagonal hole for square wrench, hexagon wrench, or octagon wrench insertion. This wrench insertion hole may be used as a hole 11b to be filled with a filler 21, and a filler 21 impregnated with a biological bone growth factor is packed thereinto.

Small holes (not shown) connected to the hole 11b to be filled with a filler 21 may be formed in the shaft part of the screw 11 of this implant composite material 118 as long as the screw 11 can retain a strength required of bone-uniting material main bodies. Formation of such small holes has the following advantages. A body fluid and an osteoblast are apt to penetrate through the small holes into the filler 21, and the biological bone growth factor becomes apt to exude with the degradation and assimilation of the filler 21. Consequently, the growth of a living bone and bone adhesion can be further accelerated.

In the screw 1 of this implant composite material 118, the screw shaft part has a screw thread 11c extending throughout the whole length thereof. However, the screw 11 may be one in which a screw thread 11c has been partly formed in an area ranging from a middle part of the screw shaft part to the tip thereof. Furthermore, although this screw 11 has a hole 11c extending from the upper end surface of the screw head 11a to a part near to the screw tip, the screw 11 may have a constitution in which a through-hole extending from the upper end surface of the screw head 11a to the screw tip is formed and a filler 21 is packed into this through-hole over the whole length thereof.

The bone-uniting material main body which has been formed into the screw 11 of this implant composite material 118 is one comprising a compact composite of a biodegradable and bioabsorbable polymer containing bioabsorbable and bioactive bioceramic particles, and is required to have a high strength which is equal to or higher than living bones (hard bones). Because of this, a biodegradable and bioabsorbable polymer suitable for use as a raw material therefor is a crystalline polymer such as poly(L-lactic acid) or poly(glycolic acid). Especially preferred is poly(L-lactic acid) having a viscosity-average molecular weight of 150,000 or higher, desirably about 200,000-600,000.

As the bioceramic particles to be incorporated into the compact composite constituting this screw 11 (bone-uniting material main body) , it is preferred to use the same bioceramic particles as those contained in the compact composite 2 of the implant composite material 100 described above.

In the compact composite constituting the screw 11 (bone-uniting material main body), the content of the bioceramic particles is preferably in the range of 30-60% by mass. In case where the content thereof exceeds 60% by mass, there is a possibility that the compact composite becomes brittle, leading to a strength deficiency in the screw 11. Contents thereof lower than 30% by mass result in a trouble that the conductive bone formation by the action of the bioceramic particles becomes insufficient and the complete replacement of the screw 11 by a living bone requires much time. The content of the bioceramic particles may be even throughout the whole compact composite constituting the screw 11 in the range of 30-60% by mass, or may change to have an inclination so that it gradually increases from the center line toward the periphery of the compact composite in the range of 30-60% by mass. The screw 11 comprising the compact composite having such an inclination of bioceramic-particle content as in the latter case has an advantage that bone tissues conductively grow in an early stage in the peripheral parts having a high bioceramic-particle content (peripheral part of the screw head la and peripheral part of the screw shaft) and these peripheral parts bond in a short time period with a living bone. The screw 1 can hence be prevented from suffering loosening, etc.

The screw 11 (bone-uniting material main body) may be produced by molding a biodegradable and bioabsorbable polymer containing bioceramic particles to produce a compact-composite molded object in the form of a solid cylinder and cutting this molded object into a screw shape such as that shown in Fig. 34. In this case, when compression molding or forging is used to produce a compact-composite molded object and this molded object is cut, the following advantage is brought about. This compact composite is highly compact due to the compression and polymer molecules and crystals therein have been obliquely oriented from the periphery toward the center line for the screw 11. Consequently, a screw 11 having a higher strength and higher hardness can be obtained. Incidentally, a screw 11 may be produced by conducting stretch forming to obtain a molded object in which polymer molecules have been uniaxially oriented and cutting this molded object.

On the other hand, the filler 21 packed in the hole 11b which has been formed in the screw 11 (bone-uniting material main body) and at least one end of which is open is one comprising a porous composite of a biodegradable and bioabsorbable polymer containing bioabsorbable and bioactive bioceramic particles. The filler 21 has interconnected pores inside. Part of the bioceramic particles are exposed in inner surfaces of the interconnected pores and in the surfaces of the porous composite. This filler 21 has been impregnated with an appropriate amount of a biological bone growth factor.

The porous composite constituting this filler 21 need not have a high strength such as that of the screw 11 (bone-uniting material main body) comprising the compact composite, and a strength, such as that of cancellous bones, which prevents the filler 21 from breaking or being damaged upon insertion into the hole 1b of the screw 1 suffices for the porous composite. This porous composite is required to be rapidly degraded and be wholly replaced by a living bone in an early stage. Because of this, the biodegradable and bioabsorbable polymer to be used as a raw material for the porous composite preferably is the same as the biodegradable and bioabsorbable polymer in the porous composite 2 of the implant composite material 100 described above.

It is desirable that the porous composite constituting the filler 21 should be one in which the porosity thereof is 60-90%, preferably 65-85%, interconnected pores account for 50% or more, preferably 70-90%, of all pores, and the interconnected pores have a pore diameter of 50-600 µm, preferably 100-400 µm, when the strength suitable for the filler 21, osteoblast penetration and stabilization, suitability for impregnation with a biological bone growth factor, etc. are taken into account. The reasons for these are as explained above with regard to the packing 20 comprising a porous composite in the implant composite material 117.

The porosity of the porous composite constituting the filler 21 may be even throughout the whole composite in the range of 60-90%, or may continuously increase toward the peripheral surface and toward the upper and lower ends in the range of 60-90%. The pore diameter of the interconnected pores may be even throughout the whole composite in the range of 50-600 µm, or may gradually increase toward the peripheral surface and toward the upper and lower ends in the range of 50-600 µm. The filler 21 comprising the porous composite having such inclinations of porosity and pore diameter has the following advantages. An appropriate amount of a biological bone growth factor can be rapidly infiltrated through the upper and lower ends and peripheral surface having a high porosity and a large pore diameter. Hydrolysis hence proceeds rapidly from the upper and lower ends and peripheral surface, and osteoblast penetration and the inductive growth of bone tissues are enhanced. Consequently, replacement by a living bone is further accelerated.

The bioceramic particles to be incorporated into the porous composite constituting the filler 21 may be the same as the bioceramic particles contained in the compact composite constituting the screw 11 described above. However, bioceramic particles having a particle diameter of about 0.1-5 µm are especially preferred because use of such bioceramic particles is free from the possibility of cutting the fibers to be formed, e.g., by spraying in producing the porous composite by the method which will be described later, and because such bioceramic particles have satisfactory bioabsorbability.

The content of the bioceramic particles in the porous composite constituting the filler 21 is preferably 60-80% by mass. The content thereof may be even throughout the whole porous composite in that range or may continuously increase toward the peripheral surface and the upper and lower ends in that range. In case where the content thereof exceeds 80% by mass, such a high bioceramic-particle content coupled with the high porosity of the porous composite results in a decrease in the physical strength of the filler 21 comprising the porous composite. Contents thereof lower than 60% by mass arouse the following trouble. This porous composite has reduced bioactivity and, hence, the inductive growth of bone tissues becomes slow. As a result, the time period required for the hole 11b of the screw 11 to be filled by complete replacement by a living bone is prolonged. A more preferred range of the content of the bioceramic particles is 60-70% by mass. In the filler 21 comprising the porous composite having such an inclination of content, bioactivity is high in the surface-layer parts having a high content and the inductive growth of an osteoblast and bone tissues therein is especially enhanced. Consequently, replacement by a living bone is further accelerated.

The surface of the filler 21 comprising the porous composite may be subjected to an oxidation treatment such as corona discharge, plasma treatment, or hydrogen peroxide treatment. Such an oxidation treatment has the following advantage. The wettability of the surface of the filler 21 is improved and an osteoblast more effectively penetrates into interconnected pores in the filler 21 through the minute gap between the filler 21 and the hole 1b of the screw 11 and grows therein. Because of this, complete replacement by a living bone is further accelerated and the filler-filled hole 11b of the screw 11 is filled with the living bone in an early stage. The surface of the screw 11 comprising the compact composite may, of course, be subjected to such an oxidation treatment.

The filler 21 comprising the porous composite can be produced, for example, by the following process. First, a biodegradable and bioabsorbable polymer is dissolved in a volatile solvent and bioceramic particles are mixed with the solution to prepare a suspension. This suspension is formed into fibers by spraying or another technique to produce a fibrous mass composed of fibers intertwined with one another. This fibrous mass is immersed in a volatile solvent such as methanol, ethanol, isopropanol, dichloroethane(methane), or chloroform to bring it into a swollen or semi-fused state. The fibrous mass in this state is pressed to obtain a porous fusion-bonded fibrous mass in a solid cylinder form corresponding and conforming to the hole 11b of the screw 11. The fibers in this fusion-bonded fibrous mass are shrunk and fused, and are thereby deprived substantially of their fibrous shape to form a matrix. Thus, the fibrous mass is changed in form into a porous composite in which the spaces among the fibers have been changed into rounded interconnected pores to thereby produce the filler 21.

The biological bone growth factor to be infiltrated into the porous composite constituting the filler 21 may be any of the BMP, TGF-β, EP4, b-FGF, and PRP described above. These biological bone growth factors may be enclosed alone or as a mixture of two or more thereof in a container 3. It is also preferred that an osteoblast derived from a living organism should be added to any of those biological bone growth factors. Any of those biological bone growth factors or a mixture thereof with an osteoblast derived from a living organism may be infiltrated into the filler 21 as an injection or dripping preparation in a solution or suspension state.

The infiltration of any of those biological bone growth factors and/or the osteoblast into the filler 21 has the following advantages. Prior to or simultaneously with the hydrolysis of the filler 21, the biological bone growth factor, etc. exude to considerably accelerate osteoblast multiplication/growth. Because of this, bone adhesion is completed in about several weeks although this period depends on the part and the growth factor, and bone tissues come to grow in surface-layer parts of the porous composite constituting the filler 21. The porous composite is wholly replaced by a living bone rapidly thereafter and the hole 11b of the screw 11 is filled with the living bone. Of the biological bone growth factors shown above, BMPs and EP4 are especially effective in hard-bone growth. PRP is a plasma having a highly elevated platelet concentration, and addition thereof accelerates the growth of a newly regenerated bone. In some cases, another growth factor such as IL-1, TNF-α, TNF-β, or IFN-γ may be mixed.

The implant composite material 118 having the constitution described above is intended to be used for uniting/fixing by screwing the screw 11 (bone-uniting material main body) into the bone of a fractured part. When a fractured part is united/fixed in this manner, the following advantages are brought about. The screw 11, which comprises the compact composite comprising a biodegradable and bioabsorbable polymer containing bioceramic particles, has a sufficient mechanical strength, although it is a hollow object having a hole 11b which is open at one end, and slowly undergoes hydrolysis by a body fluid. Because of this, the screw 11 retains its strength over a period of at least 3 months, which is necessary for ordinary bone adhesion, and can fix the osteosynthesis part without fail. On the other hand, the filler 21 which comprises the porous composite of a biodegradable and bioabsorbable polymer containing bioceramic particles and which has been inserted in the hole 11b of the screw 11 enables a body fluid and an osteoblast to penetrate into inner parts of the porous composite through interconnected pores threof, and is degraded and assimilated earlier than the screw 11 comprising the compact composite while exhibiting its bone conductivity and bone inductivity based on the bioactivity of the bioceramic particles. Prior to or simultaneously with this degradation/assimilation, the biological bone growth factor, e.g., a BMP, supported by the filler 21 is gradually released. Because of this, the conductive formation of a living bone is efficiently accelerated and bone adhesion is completed in about several weeks, which period is considerably shorter than three months necessary for ordinary bone adhesion, although that period varies depending on the part and the biological bone growth factor. Thereafter, the screw 11 and the filler 21 further undergo degradation and assimilation and are finally replaced completely by a living bone formed by bone conduction or bone induction, whereby the bone is restored to the original state in which the hole 11b of the screw 11 does not remain vacant. Furthermore, since the biological bone growth factor contained in the filler 21 comprising the porous composite has not undergone the heat history attributable to screw 11 production, it has not fear of having undergone thermal alteration and functions to accelerate bone growth. In addition, since the bioceramic particles contained in the filler 21 and in the screw 11 are bioabsorbable, they neither remain/accumulate in the living bones which have replaced nor come into/remain in soft tissues or blood vessels.

Fig. 35 illustrates an implant composite material for osteosynthesis as still a further embodiment of the invention: (a), (b), and (c) are a front view, vertical sectional view, and plan view thereof, respectively.

This implant composite material 119 for osteosynthesis comprises a bone-uniting material main body which comprises a compact composite and has been formed into a pin 12. This pin 12 has a hole 12a which is open at each end and extends along the center line for the pin 12 from a one-end surface (upper end surface) to the other-end surface (lower end surface) of the pin 12. A filler 22 in a solid cylinder form having a diameter and length conforming to the diameter and length of the hole 12a and impregnated with a biological bone growth factor has been packed in the hole 12a.

This pin 12 (bone-uniting material main body) comprises the same compact composite as the screw 11 described above, i.e., a compact composite of a biodegradable and bioabsorbable polymer containing bioabsorbable and bioactive bioceramic particles. The peripheral surface thereof has an alternation of a tapered surface 12b becoming gradually narrower toward an end (becoming gradually narrower downward) and a flange part 12c. This shape prevents the pin 12 which has been driven into a hole formed in the bone of a fractured part from coming out because the peripheral edges of the flange parts 12c bite into the living bone. The diameter of the through-hole 12a of this pin 12 is preferably regulated to about 1/3 to 2/3 the diameter of the pin 12 (diameter of the thinnest parts at the lower ends of the tapered surfaces). In case where the hole diameter is larger than that, this pin 12 has a reduced strength, resulting in a possibility that this pin 12 might break or be damaged when driven into a fractured part. In case where the hole diameter is smaller than that, the filler 22 is too thin and the proportion of the filler 22 is reduced, whereby the effect of inductively forming a living bone becomes insufficient.

The shape of the pin 12 is not limited to that in this embodiment, and can be any desired shape. For example, the pin 12 may be a pin of a mere hollow cylinder or hollow prism shape having a through-hole 12a extending along the center line or a pin of the hollow prism shape which has an alternation of an oblique surface and a flanged part on each of the four lateral sides for preventing the pin from coming out. Furthermore, the hole 12a is not limited to a through-hole which is open at each end. It may, of course, be a deep bottomed hole extending from a one-end surface (upper end surface) to a part close to the other-end surface (lower end surface) of the pin 12. Such a bottomed hole has an advantage that the lower end (tip) of the pin 12 has a high strength and is hence less apt to break when the pin 12 is driven.

On the other hand, the filler 22 is one comprising a porous composite of a biodegradable and bioabsorbable polymer containing a bioabsorbable and bioactive bioceramic particles. In this embodiment, the filler 22 is in the form of a solid cylinder having a diameter and length conforming to the diameter and length of the through-hole 12a of the pin 12, and has been impregnated with any of the bone growth factors described above. When this hole 12a is thin and long, then a filler 22 which is thin and long so as to conform to the hole is difficult to insert and pack into the hole. It is therefore preferred that two or more short fillers 22 having a solid cylinder form with a diameter conforming to the diameter of this hole 12a should be prepared and successively inserted into the hole 12a of the pin 12 to thereby pack the fillers 2 so as to fill the hole 12a over the whole length thereof.

In this implant composite material 119 for osteosynthesis also, small holes (not shown) connected to the hole 12a to be filled with the filler 22 may be formed in the pin 12 as long as a necessary strength can be maintained. Formation of such small holes has the following advantages. The small holes enable a body fluid and an osteoblast to easily come into contact with and penetrate into the filler 22 and facilitate the exudation of the biological bone growth factor.
Consequently, the growth of a living bone and bone adhesion are further accelerated.

The compact composite of a biodegradable and bioabsorbable polymer which constitutes the pin 12, bioceramic particles contained therein, content of the particles, and the like are the same as those in the screw 11 in Fig. 34 described above, and explanations thereon are hence omitted. Furthermore, the porous composite of a biodegradable and bioabsorbable polymer which constitutes the filler 22, porosity thereof, proportion of interconnected pores, pore diameter of the interconnected pores, bioceramic particles contained therein, content thereof, and the like also are the same as those in the filler 21 in Fig. 34 described above, and explanations thereon are hence omitted.

When this implant composite material 119 for osteosynthesis is used for bone uniting/fixing by driving the pin 12 (bone-uniting material main body) into a hole formed in the bone of a fractured part, the following advantages are brought about as in the case of the screw-form implant composite material 118 for osteosynthesis described above. The pin 12 retains a sufficient strength over a period of at least 3 months, which is necessary for bone adhesion, and fixes the osteosynthesis part without fail. On the other hand, the filler 22 releases the biological bone growth factor while being rapidly hydrolyzed. Because of this, bone adhesion in the united/fixed part is completed in about several weeks although this period varies depending on the part and the bone growth factor. In addition, due to the bioactivity of the bioceramic particles, the filler 22 is replaced by a living bone and the hole 12a of the pin 12 is hence filled with the living bone in a relatively early stage. Finally, the pin 12 also is wholly replaced by the living bone and disappears, whereby the fractured bone is restored to the original state in which the hole 12a does not remain vacant.

Other embodiments of the implant composite material for osteosynthesis of the invention include one comprising: a bone-uniting material main body which comprises a compact composite of a biodegradable and bioabsorbable polymer containing bioabsorbable and bioactive bioceramic particles and has been formed into the screw 11 or pin 12 described above; and a filler (filler not impregnating with a biological bone growth factor) comprising the porous composite described above, which comprises a biodegradable and bioabsorbable polymer containing bioabsorbable and bioactive bioceramic particles, the filler having been inserted into the hole 11b or 12a of the bone-uniting material main body, the hole 11b or 12a being open at at least one end.

This implant composite material for osteosynthesis may be used in the following manner. The bone-uniting material main body is screwed or driven into the bone of a fractured part to thereby unite/fix the bone. Prior to or after this uniting/fixing, any of the biological bone growth factors separately prepared is injected and infiltrated into the filler. As a result, the same advantages and effects as in the case of the implant composite materials 118 and 119 for osteosynthesis are obtained. In this case, when the bone-uniting material main body in the form of a screw 11 or pin 12 has the small holes connected to the hole 11b or 12a, this constitution has an advantage that the infiltration of the biological bone growth factor is facilitated.

The implant composite materials for osteosynthesis of the invention described above may be provided as a set of constituent members therefor.
A first osteosynthesis set among such sets is characterized by comprising a combination of (1) a filler-filled bone-uniting material main body obtained by forming a bone-uniting material main body comprising a compact composite of a biodegradable and bioabsorbable polymer containing bioabsorbable and bioactive bioceramic particles, forming in the main body a hole which is open at at least one end, and filling the hole with a filler comprising a porous composite of a biodegradable and bioabsorbable polymer containing bioabsorbable and bioactive bioceramic particles and (2) a biological bone growth factor to be infiltrated into the filler.
A second osteosynthesis set is characterized by comprising a combination of (1) bone-uniting material main body which comprises a compact composite of a biodegradable and bioabsorbable polymer containing bioabsorbable and bioactive bioceramic particles and has a hole formed therein which is open at at least one end, (2) a filler which is to be packed into the hole of the bone-uniting material main body and comprises a porous composite of a biodegradable and bioabsorbable polymer containing bioabsorbable and bioactive bioceramic particles, and (3) a biological bone growth factor to be infiltrated into the filler.
A third osteosynthesis set is characterized by comprising a combination of (1) bone-uniting material main body which comprises a compact composite of a biodegradable and bioabsorbable polymer containing bioabsorbable and bioactive bioceramic particles and has a hole formed therein which is open at at least one end and (2) a filler which is to be packed into the hole of the bone-uniting material main body, comprises a porous composite of a biodegradable and bioabsorbable polymer containing bioabsorbable and bioactive bioceramic particles, and contains a biological bone growth factor.
A fourth osteosynthesis set is characterized by comprising a combination of (1) bone-uniting material main body which comprises a compact composite of a biodegradable and bioabsorbable polymer containing bioabsorbable and bioactive bioceramic particles and has a hole formed therein which is open at at least one end and (2) a filler which is to be packed into the hole of the bone-uniting material main body and comprises a porous composite of a biodegradable and bioabsorbable polymer containing bioabsorbable and bioactive bioceramic particles.

Typical examples of the bone-uniting material main bodies in those osteosynthesis sets are: a screw having a hole formed therein which is to be filled with the filler and extends along the center line from the upper end surface of the screw head toward the screw tip; and a pin having a hole formed therein which is to be filled with the filler and extends along the center line from one-end surface toward the other-end surface.

The first osteosynthesis set is used in the following manner. When the filler-filled bone-uniting material main body is, e.g., a filler-filled screw, this main body is screwed into the bone of a fractured part to unite/fix the bone. When the filler-filled bone-uniting material main body is, e.g., a filler-filled pin, this main body is driven into the bone of a fractured part to unite/fix the bone. Thereafter, the biological bone growth factor, e.g., a BMP, is injected/infiltrated into the filler. By using the set in this manner, the same effects and advantages as in the case of the implant composite materials 118 and 119 are obtained.

The second osteosynthesis set is used in the following manner. The bone of a fractured part is united/fixed with the bone-uniting material main body. Thereafter, the filler is packed into the hole of the bone-uniting material main body, the hole being open at at least one end, and the biological bone growth factor, e.g., a BMP, is then injected/infiltrated into this filler. Alternatively, after the bone of a fractured part has been united/fixed with the bone-uniting material main body, the biological bone growth factor is infiltrated into the filler and this filler is packed into the hole of the bone-uniting material main body. By using the set in this manner, the same effects and advantages as in the case of the implant composite materials 118 and 119 are obtained.

The third osteosynthesis set is used in the following manner. The bone of a fractured part is united/fixed with the bone-uniting material main body. Thereafter, the filler impregnated with a biological bone growth factor, e.g., a BMP, is packed into the hole of the bone-uniting material main body, the hole being open at at least one end. By using the set in this manner, the same effects and advantages as in the case of the implant composite materials 118 and 119 are obtained.

The fourth osteosynthesis set is used in the following manner. The bone of a fractured part is united/fixed with the bone-uniting material main body. Thereafter, the filler is packed into the hole of the bone-uniting material main body, the hole being open at at least one end, and a biological bone growth factor separately prepared, e.g., a BMP, is then injected/infiltrated into this filler. Alternatively, after the bone of a fractured part has been united/fixed with the bone-uniting material main body, a biological bone growth factor separately prepared is infiltrated into the filler and this filler is packed into the hole of the bone-uniting material main body. By using the set in this manner, the same effects and advantages as in the case of the implant composite materials 118 and 119 are obtained.

In each of those osteosynthesis sets, the content of the bioceramic particles in the compact composite constituting the bone-uniting material main body is preferably 30-60% by mass, and the content of the bioceramic particles in the porous composite constituting the filler is preferably 60-80% by mass. The osteosynthesis set having such contents exhibits satisfactory bone conductivity while retaining the intact strength required of the bone-uniting material main body, and can be replaced by a living bone. The filler also exhibits satisfactory bone inductivity and can be replaced by a living bone in an early stage. It is also preferred that the porous composite constituting the filler should be one in which the porosity thereof is 60-90%, at least 50% of all pores are accounted for by interconnected pores, and the interconnected pores have a pore diameter of 50-600 µm. The osteosynthesis set having such porosity and pore diameter has the following advantages. An appropriate amount of a biological bone growth factor can be easily injected/infiltrated into the filler to facilitate the penetration of a body fluid and an osteoblast. Consequently, hydrolysis of the filler proceeds and bone tissues inductively grow in an early stage, whereby the filler is wholly replaced by a living bone and disappears in a short time period. As the biological bone growth factor, use may be made of any one of or a mixture of two or more of the BMP, TGF-β, EP4, b-FGF, and PRP shown above.

Fig. 36 illustrates one example of those bone-uniting material sets: (a) is a vertical sectional view of a filler-filled bone-uniting material main body in this set and (b) is a front view of a container in this set, the container containing a biological bone growth factor.

This bone-uniting material set comprises a combination of: a filler-filled bone-uniting material main body comprising a screw 11 having a hole 11b formed therein which extends along the center line for the screw 11 and is open at at least one end and a filler 21 packed in the hole 11b; and a biological bone growth factor enclosed in a container 41 such as an ampule. This combination may be packed into, e.g., a bag or case. The screw 11 (bone-uniting material main body) is the same as the screw 11 in the implant composite material 118 in Fig. 34 described above, and the filler 21 also is the same as the filler 21 in the implant composite material 118 in Fig. 34 described above. Explanations thereon are hence omitted. Furthermore, the biological bone growth factor also is the same as the biological bone growth factor infiltrated into the filler 21 in the implant composite material 118 in Fig. 34 described above, and is enclosed in the container 41 as an injection or dripping preparation in a solution or dispersion state. An explanation thereon is hence omitted.

This bone-uniting material set may be used in the following manner. The screw 11 filled with the filler 21 (filler-filled bone-uniting material main body) is screwed into the bone of a fractured part to unite/fix the bone. Prior to or before this uniting/fixing, the container 41 is opened and the biological bone growth factor is injected and infiltrated into the filler 21. This set, when used in this manner, brings about the following advantages. The screw 11 retains a sufficient strength over a period of 3 months, which is necessary for bone adhesion, to fix the osteosynthesis part without fail. On the other hand, the filler 21 releases the biological bone growth factor while being rapidly hydrolyzed. Consequently, bone adhesion in the osteosynthesis part is completed in about several weeks, although this period varies depending on the part and the bone growth factor. In addition, the filler 21 is replaced by a living bone due to the bioactivity of the bioceramic particles and the hole 11b of the screw is hence filled with the living bone in a relatively early stage. Finally, the screw 11 also is wholly replaced by a living bone and disappears, whereby the fractured bone is restored to the original state in which the hole 11b does not remain vacant.

Fig. 37 illustrates another example of the bone-uniting material sets: (a) is a front view of a bone-uniting material main body in this set, (b) is a front view of a filler in the set, and (c) is a front view of a container in the set, the container containing a biological bone growth factor. Fig. 38 is a vertical sectional view of the bone-uniting material main body in this bone-uniting material set.

This bone-uniting material set comprises: a bone-uniting material main body which has been formed into a screw 11 and has a hole 11b extending along the center line for the screw 11; a filler 21 to be packed into the hole 11b; and a biological bone growth factor enclosed in a container 41 such as an ampule. This combination may be packed into, e.g., a bag or case. This screw 11 (bone-uniting material main body) comprises a compact composite of a biodegradable and bioabsorbable polymer containing bioabsorbable and bioactive bioceramic particles. As shown in Fig. 38, the screw 11 has a hole 11d for Kirschner wire 43 insertion which penetrates the screw 11 so as to extend along the center line for the screw 11 from the upper end surface of the screw head 11a to the screw tip. This Kirschner wire insertion hole 11d is used also as the hole 11b to be filled with the filler 21. Like the screw 11 in Fig. 34 described above, this screw 11 has been formed so that the screw head 11a has a square plane shape in which the four corners have been rounded. However, a screw thread 11c has not been formed throughout the whole length of the screw shaft as in the screw 11 described above but formed partly in an area ranging from a middle part of the screw shaft to the tip thereof.

On the other hand, the filler 21 comprises a porous composite of a biodegradable and bioabsorbable polymer containing bioabsorbable and bioactive bioceramic particles, and is in a solid cylinder form having a diameter and length corresponding and conforming to the through-hole 11b (Kirschner wire insertion hole 11d) formed along the center line for the screw 11. However, when the hole 11b to be filled with this filler is thin and long, it is preferred that short cylindrical fillers having a length of about 1/2, 1/3, or 1/4 the length of this hole 1b should be formed and two, three, or four such short fillers be included in a set together with the screw 11. Such short fillers have an advantage that even when the hole 11b of the screw 11 is long, the operation of filler insertion is easy because such fillers can be successively inserted into the hole 11b.

The compact composite of a biodegradable and bioabsorbable polymer which constitutes the screw 11 (bone-uniting material main body), bioceramic particles contained therein, content thereof, and the like are the same as those in the screw 11 of the implant composite material 118 in Fig. 34 described above. Explanations thereon are hence omitted. The porous composite of a biodegradable and bioabsorbable polymer which constitutes the filler 21, porosity thereof, proportion of interconnected pores, pore diameter of the interconnected pores, bioceramic particles contained, content thereof, and the like also are the same as those in the filler 21 in the implant composite material 118 in Fig. 34 described above, and explanations thereon are hence omitted. Furthermore, the biological bone growth factor enclosed in the container 41 also is the same as the biological bone growth factor enclosed in the container 41 in the bone-uniting set in Fig. 36 described above, and an explanation thereon is hence omitted.

This bone-uniting material set may be used for uniting/fixing the bone of a fractured part in the following manner. First, a Kirschner wire 43 is inserted into the hole 11b (Kirschner wire insertion hole 11d) extending along the center line for the screw 11 (bone-uniting material main body). This Kirschner wire 43 is used as a guide to precisely screw the screw 11 in a given direction into the target position in the fractured part to unite/fix the bone. The Kirschner wire is then drawn out. Thereafter, the filler 21 is packed into the hole 11b of the screw 11. The container 41 is opened and the biological bone growth factor is injected and infiltrated into the filler 21. Alternatively, the container 41 is opened and the biological bone growth factor is infiltrated into the filler 21, before this filler 21 impregnated with the bone growth factor is packed into the hole 11b of the screw 11. As a result, the same effects and advantages as in the case of the bone-uniting material set in Fig. 36 described above are obtained.

Although the bone-uniting material sets described above each include a biological bone growth factor enclosed in a container 41 as a constituent material combined, bone-uniting material sets need not always include a biological bone growth factor as a constituent material to be combined. Fig. 39 illustrates still another example of such bone-uniting material sets: (a) is a front view of a bone-uniting material main body in this set and (b) is a front view of a filler in the set. Fig. 40 (a) is a vertical sectional view of the bone-uniting material main body in this bone-uniting material set and (b) is a plan view thereof.

This bone-uniting material set comprises a combination of: a bone-uniting material main body formed into a large screw 13 which is for greater-trochanter fracture osteosynthesis and has a hole 13b extending along the center line for this large screw 13; and a filler 23 to be packed into the hole 13b. This combination may be packed into, e.g., a bag or case. This large screw 13 (bone-uniting material main body) comprises a compact composite of a biodegradable and bioabsorbable polymer containing bioabsorbable and bioactive bioceramic particles. As shown in Fig. 40, a large hole 13e having a female thread in the inner surface thereof and a hole13d for Kirschner wire insertion thereinto have been successively formed so as to penetrate the large screw 13 along the center line therefor from the upper end surface of the screw head 13a to the screw tip. The large hole 13e and the Kirschner wire insertion hole 13d are used also as the hole 13b to be filled with the filler 23. The head 13a of this large screw 13 is in the form of a solid hexagonal prism as shown in Fig. 40 (a) and (b). In that part in the screw shaft which is near to the tip, a male thread 13c has been formed in which the male thread top is flat and the valley is a rounded groove. As shown in Fig. 39 (a), the peripheral surface of the screw shaft has a small external groove 13f extending in parallel with the center line.

On the other hand, the filler 23 comprises a porous composite of a biodegradable and bioabsorbable polymer containing bioabsorbable and bioactive bioceramic particles. As shown in Fig. 39 (b), the filler 23 is one obtained by integrally and coaxially forming a large-diameter solid-cylinder part 23a having a diameter and length corresponding and conforming to the large hole 13e of the large screw and a small-diameter solid-cylinder part 23b having a diameter and length corresponding and conforming to the Kirschner wire insertion hole 13d.

The compact composite of a biodegradable and bioabsorbable polymer which constitutes the large screw 13 (bone-uniting material main body) for greater-trochanter fracture osteosynthesis, bioceramic particles contained therein, content thereof, and the like are the same as those in the screw 11 in Fig. 34 described above. Explanations thereon are hence omitted. Furthermore, the porous composite of a biodegradable and bioabsorbable polymer which constitutes the filler 23, porosity thereof, proportion of interconnected pores, pore diameter of the interconnected pores, bioceramic particles contained, content thereof, and the like also are the same as those in the filler 21 in the bone-uniting material in Fig. 34 described above, and explanations thereon are hence omitted.

This bone-uniting material set may be used for uniting/fixing a fractured part of a femur head or the like in the following manner. First, a Kirschner wire is inserted into the Kirschner wire insertion hole 13d of the large screw 13 (bone-uniting material main body) through the large hole 13e. This Kirschner wire is used as a guide to precisely screw the large screw 13 in a given direction into the target position in the fractured part to unite/fix the bone. The Kirschner wire is then drawn out. Thereafter, the filler 23 is packed into the large hole 13e and Kirschner wire insertion hole 13d of the large screw 13 which are used as a hole 13b to be filled, and a biological bone growth factor separately prepared is injected and infiltrated into this filler 23. Alternatively, a biological bone growth factor separately prepared is infiltrated into the filler 23, before this filler 23 impregnated with the biological bone growth factor is inserted into the large hole 13e and the Kirschner wire insertion hole 13d. As a result, the same effects and advantages as in the case of the bone-uniting material sets in Fig. 36 and Fig. 37 described above are obtained.

Other examples of the bone-uniting material sets which do not include a biological bone growth factor as a constituent material to be combined include a bone-uniting material set comprising a combination of: a screw (bone-uniting material main body) which comprises a compact composite of a biodegradable and bioabsorbable polymer containing bioabsorbable and bioactive bioceramic particles and has a bored hole for filler insertion which extends along the center line for the screw from the upper end surface of the screw head toward the screw tip; and a filler which is to be inserted into the hole and which comprises a porous composite of a biodegradable and bioabsorbable polymer containing bioabsorbable and bioactive bioceramic particles and contains a biological bone growth factor infiltrated therein. This combination may be packed into, e.g., a bag or case.

This bone-uniting material set may be used in the following manner. The screw (bone-uniting material main body) is used to unite/fix the bone of a fractured part. Thereafter, the filler impregnated with a biological bone growth factor is packed into the hole of the screw. As a result, the same effects and advantages as in the case of the bone-uniting material sets in Fig. 36, Fig. 37, and Fig. 39 described above are obtained.

The bone-uniting material sets described above each are one in which the bone-uniting material main body is a screw and this screw has a bored hole to be filled with a filler, the hole extending along the center line for the screw from the upper end surface of the screw head toward the screw tip. However, it is a matter of course that the bone-uniting material main body of such a bone-uniting material set may be a pin for osteosynthesis in which a hole to be filled with a filler has been formed so as to extend along the center line from a one-end surface toward the other-end surface of the pin.

Furthermore, those bone-uniting material sets and the implant composite materials for osteosynthesis described hereinabove each are one in which the filler comprises a biodegradable and bioabsorbable polymer containing bioabsorbable and bioactive bioceramic particles. However, use may be made of a constitution in which a filler is formed from a biodegradable and bioabsorbable polymer containing no bioceramic particles and a biological bone growth factor is infiltrated into this filler. It is not denied that such filler containing no bioceramic particles is slightly inferior to bioceramic-particle-containing fillers in living-bone conductivity or inductivity after impregnation with a biological bone growth factor. However, the biological bone growth factor supported on the filler exudes and, hence, bone adhesion in the osteosynthesis part is completed in about several weeks. Consequently, the time period required for the patient to leave his bed is significantly shortened, and all of the patient, doctor, and hospital make a large profit. Thus, one of the main objects of the invention can be sufficiently accomplished.

### INDUSTRIAL APPLICABILITY

The implant composite material of the invention can be advantageously used as a temporary prosthetic/scaffold material in the treatment or reconstruction of a necrotized part of an articular bone head or in the reinforcement of a ligament part adherent to a joint, or as an anchor member to be attached to an end part of a ligamental member or tendinous member, or as an interference screw for tendon or ligament fixing, or as a bone-uniting material for fractured parts. The porous composite is replaced by bone tissues in an early stage to attain bonding with and fixing to a living bone, while the compact composite retains a necessary strength over a necessary time period. Finally, the compact composite is wholly replaced by the living bone and disappears. This implant composite material can sufficiently meet desires in this medical field.

## Claims

1. A bioabsorbable and bioactive implant composite material, which comprises a compact composite of a biodegradable and bioabsorbable polymer containing bioabsorbable and bioactive bioceramic particles and a porous composite of a biodegradable and bioabsorbable polymer containing bioabsorbable and bioactive bioceramic particles, wherein the porous composite is united with the compact composite.

2. The implant composite material according to claim 1, wherein the porous composite has been superposed on and united with one side or all surfaces of the compact composite.

3. The implant composite material according to claim 1 for use as an end anchor of a ligamental member or tendinous member, wherein it is an implant composite material to be attached as an anchor member to an end part of a ligamental member or tendinous member so as not to detach therefrom, and wherein the porous composite has been superposed on and united with part or all of the surfaces of the compact composite.

4. The implant composite material according to claim 1 for osteosynthesis, which comprises a bone-uniting material main body comprising the compact composite and having a hole bored to have at least one open end; and a filler packed in the hole, the filler comprising the porous composite.

5. The implant composite material according to claim 4, wherein the uniting material main body is a screw having a bored hole to be filled with the filler, wherein the hole extends along the center line of this screw from the upper end surface of the screw head toward the screw tip.

6. The implant composite material according to claim 4, wherein the bone-uniting material main body is a pin having a bored hole to be filled with the filler, wherein the hole extends along the center line of this pin from one end toward the other end of the pin.

7. The implant composite material according to claim 1 for tendon or ligament fixing, which comprises an interference screw comprising the compact composite and having a through-hole for inserting a Kirschner wire thereinto; and a packing comprising the porous composite wherein the packing is filled in the through-hole, wherein the packing contains a biological bone growth factor.

8. The implant composite material according to claim 2 or 3, wherein the porous composite contains a biological bone growth factor and/or an osteoblast derived from a living organism.

9. The implant composite material according to claim 4, wherein the filler comprising the porous composite contains a biological bone growth factor.

10. The implant composite material according to claim 2, wherein the porosity of the porous composite gradually changes to have an inclination so that the porosity increases from an inner-layer part to a surface-layer part of the porous composite in the range of 50-90%.

11. The implant composite material according to claim 3, wherein the porous composite has a porosity of 50-90%, at least 50% of all pores are accounted for by interconnected pores, and the porosity of the porous composite gradually changes to have an inclination so that the porosity increases from an inner-layer part to a surface-layer part of the porous composite.

12. The implant composite material according to claim 4 or 7, wherein the porous composite has a porosity of 60-90%, at least 50% of all pores are accounted for by interconnected pores, and the interconnected pores have a pore diameter of 50-600 µm.

13. The implant composite material according to claim 2 or 3, wherein the content of the bioceramic particles in the porous composite gradually changes to have an inclination so that it increases from an inner-layer part to a surface-layer part of the porous composite in the range of 30-80% by mass.

14. The implant composite material according to claim 4 or 7, wherein the content of the bioceramic particles in the compact composite is 30-60% by mass and the content of the bioceramic particles in the porous composite is 60-80% by mass.

15. The implant composite material according to any one of claim 7 to 9, wherein the biological bone growth factor is at least one member selected from a BMP (Bone Morphogenic Protein), TGF-β (Transforming Growth Factor β), EP4 (Prostanoid Receptor), b-FGF (basic Fibroblast Growth Factor), and PRP (platelet-rich plasma).
